# EUROPEAN PATENT APPLICATION

(11) **EP 3 492 481 A1**
(43) Date of publication of application: **05.06.2019**
(21) Application number: 17204806.8
(22) Date of filing: 30.11.2017
(51) Int. Cl.: C07H 15/04, C07H 15/08, C07H 15/18, C07H 15/26, A61K 39/108, A61K 47/64

(54) **VACCINE AGAINST KLEBSIELLA PNEUMONIAE**

(71) Applicant: Vaxxilon AG, 4153 Reinach (CH)
(72) Inventor: PEREIRA, Claney Lebev, 12203 Berlin (DE)
(74) Representative: Arth, Hans-Lothar

(57) **Abstract**

The present invention relates to a synthetic saccharide of general formula **(I)** that is related to *Klebsiella pneumoniae* serotype O3, O3b and/or O5 lipopolysaccharide and conjugate thereof. Said synthetic saccharide, said conjugate and pharmaceutical composition containing said synthetic saccharide or said conjugate are useful for prevention and/or treatment of diseases associated with *Klebsiella pneumoniae.* Furthermore, the synthetic saccharide of general formula **(I)** is useful as marker in immunological assays for detection of antibodies against *Klebsiella pneumoniae* serotype O3, O3b and/or O5 bacteria.

## Description

### Field of the invention

The present invention relates to a synthetic saccharide of general formula (**I**) that is related to *Klebsiella pneumoniae* serotype 03, O3b and/or 05 lipopolysaccharide and conjugate thereof. Said synthetic saccharide, said conjugate and pharmaceutical composition containing said synthetic saccharide or said conjugate are useful for prevention and/or treatment of diseases associated with *Klebsiella pneumoniae,* more specifically of diseases associated with *Klebsiella pneumoniae* serotype 03, O3b and/or 05. Furthermore, the synthetic saccharide of general formula (**I**) is useful as marker in immunological assays for detection of antibodies against *Klebsiella pneumoniae* bacteria.

### Background of the invention

*Klebsiella pneumoniae* is a gram-negative, facultative anaerobic, rod-shaped bacterium colonizing mostly of the respiratory and urinary tracts and causing *K*. *pneumoniae* infections (KPIs). KPI is the main cause of nosocomial infections, primarily affecting immunocompromised patients. In the last ten years, infections caused by *K. pneumoniae* are becoming an important challenge in health-care settings due to the emergence of strains resistant to almost all available antimicrobial agents and their worldwide dissemination. Infections caused by *Klebsiella pneumoniae* are responsible of high rates of morbidity and mortality. Thus, prevention of infections caused by *K. pneumoniae* is highly desirable, and vaccination of risk groups is the most cost-efficient and the most powerful means.

*K. pneumoniae* bacteria typically express two types of antigens on their cell surfaces. The first, O-antigen, is a component of the lipopolysaccharide (LPS), of which 9 varieties exist. The second is K antigen, a capsular polysaccharide with more than 80 varieties. The O-antigen is the most variable portion of the LPS and provides serological specificity, which is together with the K antigen used for serotyping. Both antigens are composed of complex polysaccharides on the bacterial surface, which are highly immunogenic and nontoxic. In comparison with proteins, carbohydrates are evolutionarily more stable. When covalently connected to a carrier protein, oligosaccharide antigens can elicit long lasting, T-cell-dependent protection.

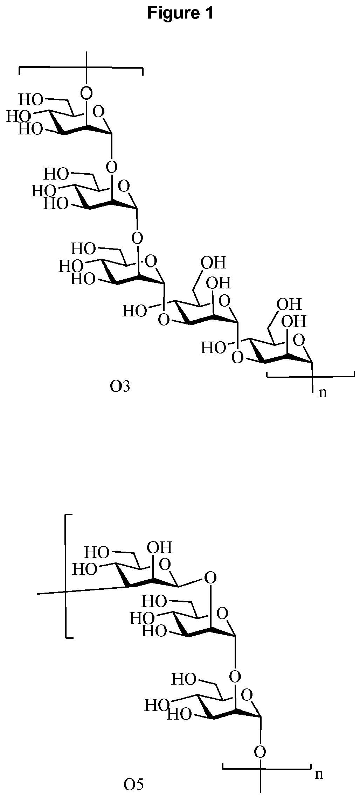

The repeating unit of the O-antigens, i.e. O-polysaccharides of *K. pneumoniae* was elucidated (The Journal of Biological Chemistry, 2002, 277 (28), pp.25070-25081) (see **Figure 1**).
The repeating unit of the O-polysaccharide of *K. pneumoniae serotype* 03 consists of:

→ 2)-α-D-Man-(1,2)-α-D-Man-(1,2)-α-D-Man-(1,3)-α-D-Man-(1,3)-α-D-Man-(1→]ₘ.

The repeating unit of the O-polysaccharide of *K. pneumoniae serotype* O3b consists of:

→ 2)-α-D-Man-(1,3)-α-D-Man-(1,3)-α-D-Man-(1→]ₘ.

The repeating unit of the O-polysaccharide of *K. pneumoniae serotype* 05 consists of:

→ 3)-β-D-Man-(1,2)-α-D-Man-(1,2)-α-D-Man-(1→.

It is the objective of the present invention to provide a well-defined synthetic saccharide of general formula (I) that is related to *Klebsiella pneumoniae* serotype O3, O3b and 05 lipopolysaccharide and contains a protective immunogenic O-antigen epitope i.e. a O-antigen epitope that elicits antibodies which protect against diseases caused by *Klebsiella pneumoniae* serotype O3, O3b and serotype 05. Said saccharide can be conjugated to an immunogenic carrier to provide a conjugate and pharmaceutical composition thereof that are useful for prevention and/or treatment of diseases associated with *Klebsiella pneumoniae* serotype O3, O3b and serotype 05. Furthermore, the synthetic saccharide of general formula (I) is useful as marker in immunological assays for detection of antibodies against *Klebsiella pneumoniae* bacteria.

The objective of the present invention is solved by the teaching of the independent claims. Further advantageous features, aspects and details of the invention are evident from the dependent claims, the description, the figures, and the examples of the present application.

### Description of the invention

### Definitions

The term "linker" as used herein encompasses molecular fragments capable of connecting the reducing-end monosaccharide of a saccharide with an immunogenic carrier or a solid support, optionally by binding to at least one interconnecting molecule. Thus, the function of the linker *per se* or together with the interconnecting molecule is to establish, keep and/or bridge a special distance between the reducing-end monosaccharide and an immunogenic carrier or a solid support. More specifically, one extremity of the linker is connected to the exocyclic oxygen atom at the anomeric center of the reducing-end monosaccharide and the other extremity is connected *via* the nitrogen atom with the interconnecting molecule, or directly with the immunogenic carrier or the solid support.

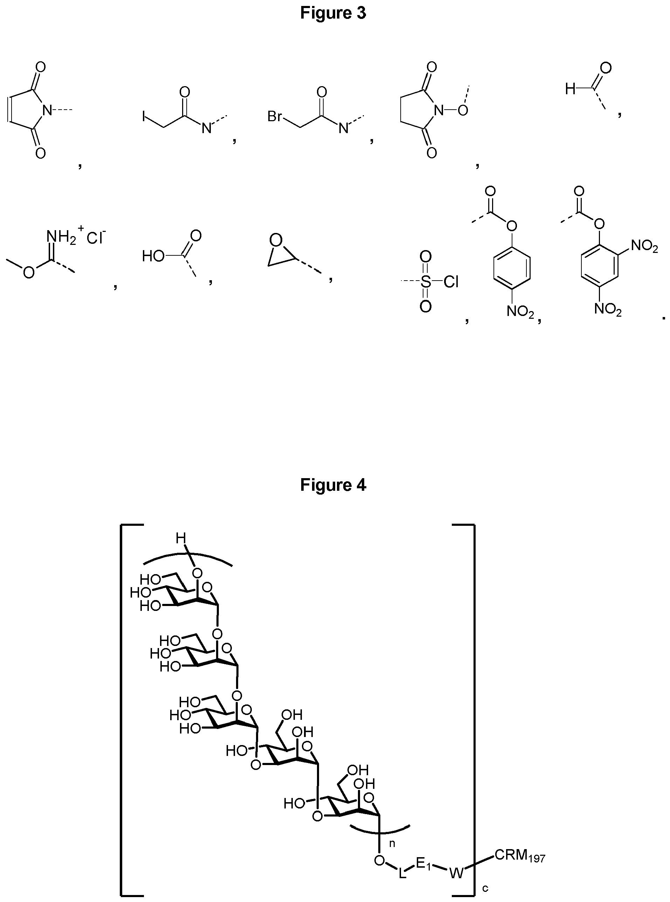

As used herein, the term "interconnecting molecule" refers to a bifunctional molecule containing functional group X and functional group Y, wherein functional group X is capable of reacting with the terminal amino group on the linker L and the functional group Y is capable of reacting with a functionality present on an immunogenic carrier or on a solid support. **Figure 3** displays examples of commercially available interconnecting molecules, but does not restrict the interconnecting molecules that can be used according to the present invention to the examples displayed herein.

The term "adjuvant" as used herein refers to an immunological adjuvant i.e. a material used in a vaccine composition that modifies or augments the effects of said vaccine by enhancing the immune response to a given antigen contained in the vaccine without being antigenically related to it. For the person skilled in the art, classically recognized examples of adjuvants include:
- mineral-containing compositions, including calcium salts and aluminium salts (or mixtures thereof). Calcium salts include calcium phosphate. Aluminium salts include hydroxides, phosphates, sulfates, etc., with the salts taking any suitable form (e.g. gel, crystalline, amorphous, etc.). Adsorption to these salts is preferred. The mineral containing compositions may also be formulated as a particle of metal salt. The adjuvants known as aluminium hydroxide and aluminium phosphate may be also used. The invention can use any of the "hydroxide" or "phosphate" adjuvants that are in general used as adjuvants. The adjuvants known as "aluminium hydroxide" are typically aluminium oxyhydroxide salts, which are usually at least partially crystalline. The adjuvants known as "aluminium phosphate" are typically aluminium hydroxyphosphates, often also containing a small amount of sulfate (i. e. aluminium hydroxyphosphate sulfate). They may be obtained by precipitation, and the reaction conditions and concentrations during precipitation influence the degree of substitution of phosphate for hydroxyl in the salt. Mixtures of both an aluminium hydroxide and an aluminium phosphate can be employed in the formulation according to the present invention;
- saponins, which are a heterologous group of sterol glycosides and triterpenoid glycosides that are found in the bark, leaves, stems, roots and even flowers of a wide range of plant species. Saponins from the bark of the *Quillaia saponaria,* Molina tree have been widely studied as adjuvants. Saponins can also be commercially obtained from Smilax ornata (sarsaprilla), Gypsophilla paniculata (brides veil), and Saponaria oficianalis (soap root). Saponin adjuvant formulations include purified formulations, such as QS21, as well as lipid formulations, such as ISCOMs. Saponin compositions have been purified using HPLC and RP-HPLC. Specific purified fractions using these techniques have been identified, including QS 7, QS 17, QS 18, QS2 1, QH-A, QH-B and QH-C. Saponin formulations may also comprise a sterol, such as cholesterol. Combinations of saponins and cholesterols can be used to form unique particles called immunostimulating complexes (ISCOMs). ISCOMs generally include a phospholipid such as phosphatidylethanolamine or phosphatidylcholine. Any known saponin can be used in ISCOMs. Preferably, the ISCOM includes one or more of QuilA, QHA & QHC;

- microparticles (i.e. a particle of 100 nm to 150 pm in diameter, more preferably 200 nm to 30 pm in diameter, or 500 nm to 10 pm in diameter) formed from materials that are biodegradable and non-toxic. Such non-toxic and biodegradable materials include, but are not restricted to poly(α-hydroxy acid), polyhydroxybutyric acid, polyorthoester, polyanhydride, polycaprolactone;
- CD1d ligands, such as an α-glycosylceramide, phytosphingosine-containing α-glycosylceramides, OCH, KRN7000 [(2S,3*S*,4*R*)-1-*O*-(α-D-galactopyranosyl)-2-(*N-*hexacosanoylamino)-1,3,4-octadecanetriol], CRONY- 101, 3"-sulfo-galactosyl-ceramide;
- immunostimulatory oligonucleotides, such CpG motif containing ones (a dinucleotide sequence containing an unmethylated cytosine residue linked by a phosphate bond to a guanosine residue), or Cpl motif containing ones (a dinucleotide sequence containing cytosine linked to inosine), or a double-stranded RNA, or an oligonucleotide containing a palindromic sequence, or an oligonucleotide containing a poly(dG) sequence. Immunostimulatory oligonucleotides can include nucleotide modifications/analogs such as phosphorothioate modifications and can be double-stranded or (except for RNA) single-stranded;
- compounds containing lipids linked to a phosphate-containing acyclic backbone, such as the TLR4 antagonist E5564;
- oil emulsions (e.g. Freund's adjuvant).

Theoretically, each molecule or substance that is able to favor or amplify a particular situation in the cascade of immunological events, ultimately leading to a more pronounced immunological response, can be defined as an adjuvant.
In principle, through the use of adjuvants in vaccine formulations, one can:
- direct and optimize immune responses that are appropriate or desirable for the vaccine;
- enable mucosal delivery of vaccines, i.e. administration that results in contact of the vaccine with a mucosal surface such as buccal or gastric or lung epithelium and the associated lymphoid tissue;
- promote cell-mediated immune responses;
- enhance the immunogenicity of weaker immunogens, such as highly purified or recombinant antigens;
- reduce the amount of antigen or the frequency of immunization required to provide protective immunity; and
- improve the efficacy of vaccines in individuals with reduced or weakened immune responses, such as newborns, the aged, and immunocompromised vaccine recipients.
Although little is known about their mode of action, it is currently believed that adjuvants augment immune responses by one of the following mechanisms:
- increasing the biological or immunologic half-life of antigens;
- improving antigen delivery to antigen-presenting cells (APCs), as well as antigen processing and presentation by the APCs e.g., by enabling antigen to cross endosomal membranes into the cytosol after ingestion of antigen-adjuvant complexes by APC;
- mimicking danger inducing signals from stressed or damaged cells, which serve to initiate an immune response;
- inducing the production of immunomodulatory cytokines;
- biasing the immune response towards a specific subset of the immune system; and - blocking the rapid dispersal of the antigen challenge.

Saccharides are known by the person skilled in the art as TI-2 (T cell independent-2) antigens and poor immunogens. Therefore, to produce a saccharide-based vaccine, said saccharides are conjugated to an immunogenic carrier to provide a conjugate, which presents an increased immunogenicity in comparison with the saccharide. In this context the term "immunogenic carrier" is defined as a structure, which is conjugated to the saccharide to form a conjugate that presents an increased immunity in comparison with the saccharide *per se.* Thus, the conjugation of the saccharides to the immunogenic carrier, preferably protein carrier, has as effect the stimulation of the immune response against said saccharide, without inducing an immune response against the said immunogenic carrier.

Hence, the present invention is directed to a saccharide of general formula (**I**)

**T*-[(-Uₓ₊₄-Uₓ₊₃-Uₓ₊₂-Uₓ₊₁-Uₓ)ₘ-(Vₓ₊₂-Vₓ₊₁-Vₓ)₁₋ₘ]ₙ-T-O-L-E** **(I)**

wherein
m is an integer selected from 0 and 1;
x is an integer selected from 1 to 2 × m + 3;
n is an integer selected from 1, 2, 3, 4, 5, 6 ,7 ,8, 9 and 10; 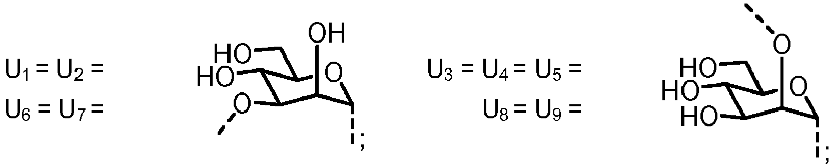 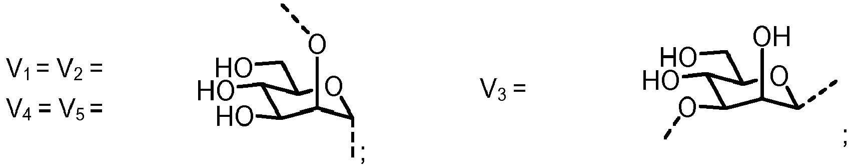 or 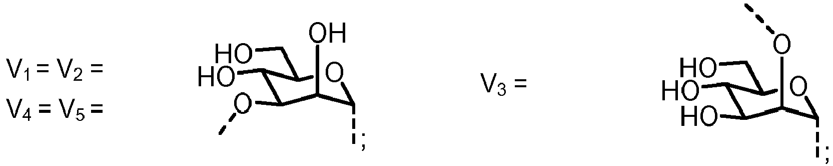
**-T-** represents a bond, -(Uₓ₊₄)ₘ-(Vₓ₊₂)₁₋ₘ-, -(Uₓ₊₄-Uₓ₊₃)ₘ-(Vₓ₊₂-Vₓ₊₁)₁₋ₘ-, -(Uₓ₊₄-Uₓ₊₃-Uₓ₊₂)ₘ- or -Uₓ₊₄-Uₓ₊₃-Uₓ₊₂-Uₓ₊₁)ₘ-;
**T*-** represents H-, H-(Uₓ)ₘ-(Vₓ)₁₋ₘ-, H-(Uₓ₊₁-Uₓ)ₘ-(Vₓ₊₁-Vₓ)₁₋ₘ-, H-(Uₓ₊₂-Uₓ₊₁-Uₓ)ₘ- or H-(Uₓ₊₃-Uₓ₊₂-Uₓ₊₁-Uₓ)ₘ-;
**L** represents a linker;
**E** represents -NH₂, -N₃, -CN, -O-NH₂, -CH=CH₂, -C=CH, -Br, -CI, **-I,** -CO₂R', -CONH-NH₂, -SH, or -SAc;
**R'** represents -H, -Me, -Et, 4-nitrophenyl, pentafluorophenyl, or *N*-hydroxysuccinimidyl;
or a diastereoisomer or a pharmaceutically acceptable salt thereof.

The linker L preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms.

The shortest atom chain between the oxygen atom (i.e. the oxygen of -O-L-NH₂) and the NH₂-group consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the NH₂-group) consists of 2 to 6 atoms, these are preferably carbon atoms. In case the shortest chain consists of 4 to 8 atoms, the chain may contain 1 or 2 heteroatoms selected from O, N and S. In case the shortest chain consists of 9 to 14 atoms, the chain may contain 1, 2, 3, or 4 heteroatoms selected from O, N and S.

It is also preferred that the linker -L-, or the shortest chain is fully or partially fluorinated. The linker -L- may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker -L- may also contain amide (-NH-CO-, -CO-NH-) and/or urea (-NH-CO-NH-) residues and preferably only one amide or urea residue. The linker may also contain substituents and preferably two substituents such as R¹⁰ and R¹¹ or four substituents such as R¹⁰, R¹¹, R¹⁵ and R¹⁴, which have the meanings as defined herein and which are preferably selected from: -F, -CI, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂, and -N(C₂H₅)₂.

In case the linker -L- is fluorinated, more than two substituents -F are preferred.

Preferably the linker -L- is selected from: -CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -CF₂-, -(CF₂)₂-, -(CF₂)₃-, -(CF₂)₄-, -(CF₂)₅-, -(CF₂)₆-, -(CF₂)₇-, -(CF₂)₈-, -(CF₂)₉-, -(CF₂)₁₀-, -(CH₂)₂-O-(CH₂)₂-, -CH₂-O-(CH₂)₃-, -(CH₂)₃-O-CH₂--CH₂-O-(CH₂)₂-, -(CH₂)₂-O-CH₂-, -(CH₂)₃-O-(CH₂)₂-, -(CH₂)₂-O-(CH₂)₃-, -(CH₂)₄-O-CH₂-, -CH₂-O-(CH₂)₄-, -L^{a}-, -L^{a}-L^{e}-, -L^{a}-L^{b}-L^{e}-, -L^{a}-L^{b}-L^{d}-L^{c}-L^{e}-, -L^{a-}L^{d-}L^{e}-;
wherein
-L^{a}- is selected from: -(CH₂)ₒ-, -(CF₂)ₒ-, -(CH₂-CH₂-O)ₒ-C₂H₄-, -(CH₂-CH₂-O)ₒ-CH₂-, -(CR¹⁰R¹¹)ₒ-, 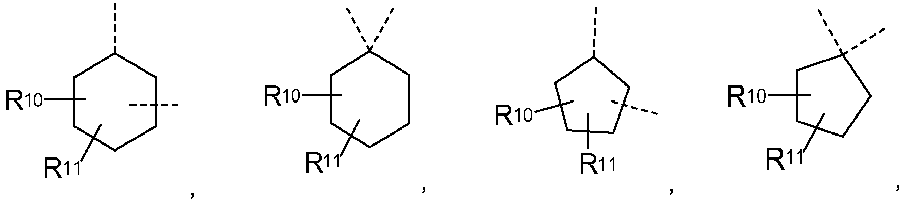 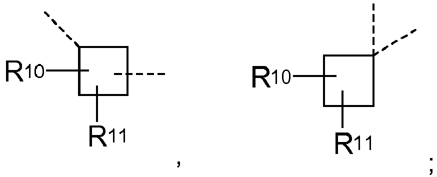 -L^{b}- and -L^{c}- are independently of each other selected from: -O-, -NH-C(O)-NH-, -NH-C(S)-NH-, -NH-C(O)-, -C(O)-NH-, -NH-C(O)-O-, -NR⁹-, -NR¹⁸-, -SO₂-, 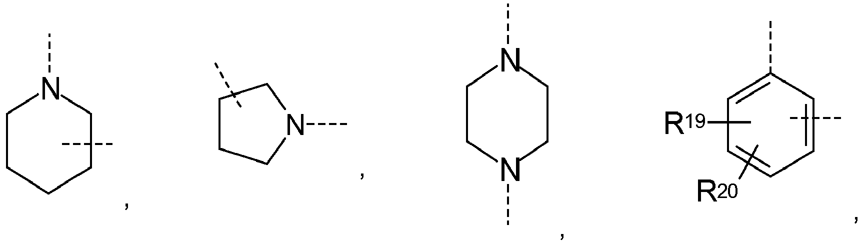 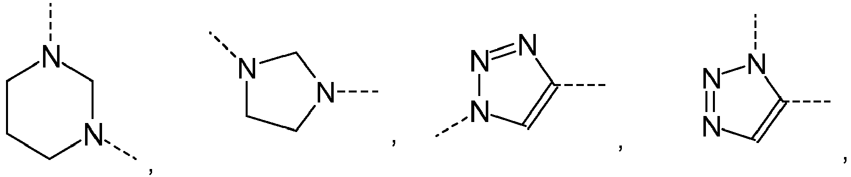 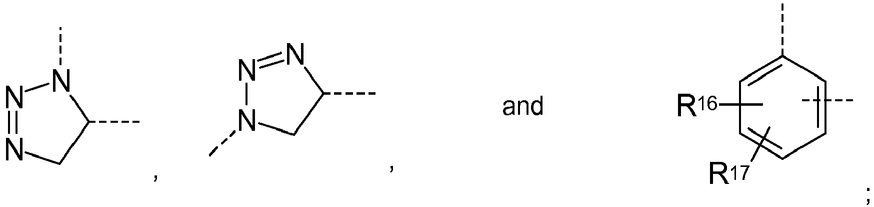 -L^{d}- represents -(CH₂)_{q}-, -(CF₂)_{q}-, -(CR¹²R¹³)_{q}-, -(CH₂-CH₂-O)_{q}-C₂H₄-, -(CH₂-CH₂-O)_{q}-CH₂-, 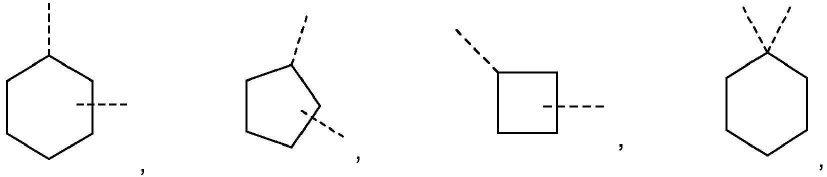 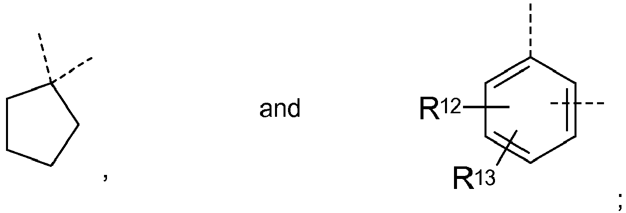 -L^{e}- is selected from: -(CH₂)ₚ₁-, -(CF₂)ₚ₁-, -C₂H₄-(O-CH₂-CH₂)ₚ₁-, -CH₂-(O-CH₂-CH₂)ₚ₁-, -(CH₂)ₚ₁-O-(CH₂)ₚ₂-, -(CR¹⁴R¹⁵)ₚ₁-, -(CR¹⁴R¹⁵)ₚ₁-O-(CR²¹R²²)ₚ₂-, 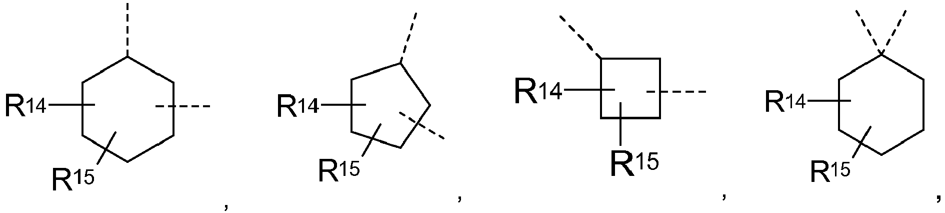 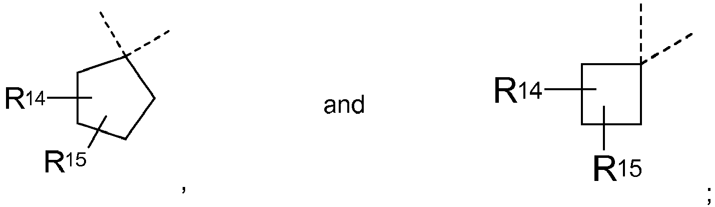 R⁹ and R¹⁸ are independently of each other selected from: -CH₃, -C₂H₅, -C₃H₇ and -C(O)CH₃;
R¹⁰, R¹¹, R¹², R¹³, R¹⁴, R¹⁵, R¹⁶, R¹⁷, R¹⁹, R²⁰, R²¹ and R²² are independently of each other selected from: -H, -F, -CI, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂ and -N(C₂H₅)₂;
o, q, p1 and p2 are independently of each other an integer selected from 1, 2, 3, 4, 5, 6, 7, 8, 9, and 10.

More preferred, **-L-** represents **-L^{a}-**, **-L^{a}-L^{e}-, -L^{a}-L^{b}-L^{e}-,** or **-L^{a}-L^{d}-L^{e}-; -L^{a}-** represents -(CH₂)ₒ-, -(CH₂-CH₂-O)ₒ-C₂H₄-, or -(CH₂-CH₂-O)ₒ-CH₂; **-L^{b}-** represents -O-;
**-L^{d}-** represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂-;
**-L^{e}-** represents -(CH₂)ₚ₁-, -(CF₂)ₚ₁-, -C₂H₄-(O-CH₂-CH₂)ₚ₁-,
-CH₂-(O-CH₂-CH₂)ₚ₁- or -(CH₂)ₚ₁-O-(CH₂)ₚ₂-; and
**o, q, p1** and **p2** are independently of each other an integer selected from 1, 2, 3, 4, 5, and 6.

Most preferred, the saccharide of the formula (I) has the group -O-**L-E** selected from the group consisting of: 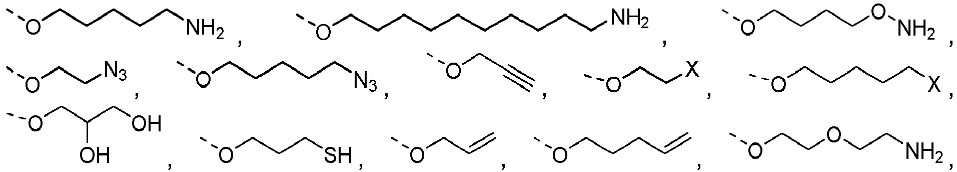 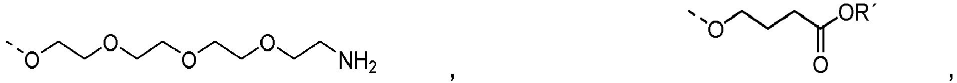 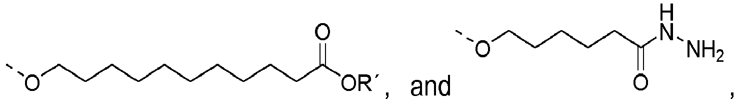
wherein R' represents -H, -Me, -Et, 4-nitrophenyl, pentafluorophenyl, or *N*-hydroxysuccinimidyl;
X represents -Br, -CI, -I, -CO₂H, or -SAc.

The anomers of saccharides of the present invention mean the α/β-anomers at C-1-postion to which the group -O-**L-E** is bounded. It is clear for the skilled person in the art of carbohydrate chemistry that the stereochemistry of the glycosidic bond is defined by the stereochemistry indicated for the anomeric center of the sugar fragments U₁, U₂, U₃, U₄, U₅, U₆, U₇, U₈, U₉, V₁, V₂, V₃, V₄, and V₅, in the general formula (**I**).

The saccharides of the present invention can be hygroscopic and thus can build various hydrates thereof. Preferred, molar ratio of water molecule to the saccharide is in the range of 1 to 20, more preferred, 1 to 10, most preferred, 5-10.
The saccharides of the present invention bear basic and/or acidic substituents and they may form salts with organic or inorganic acids or bases.

Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (*o*, *m*, *p*)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Examples of suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of a base, selected out of the group mentioned above.

It is clear for the skilled person in the art of carbohydrate chemistry that the saccharides of general (**I**) are not containing -O-O- bonds and or sugar fragments (Uₓ, Uₓ₊₁, Uₓ₊₂, Uₓ₊₃, Uₓ₊₄, Vₓ, Vₓ₊₁, Vₓ₊₂) connected or bound to each other *via* their anomeric or C-1 carbons.

Surprisingly, it was found that a saccharide of general formula (**I**) contains an immunogenic protective epitope and is able to induce a protective immune response against *K. pneumoniae* serotype O3, O3b and/or 05 bacteria in a human and/or animal host. The saccharide of general formula (**I**) elicits antibodies that are cross-reacting with the natural *K. pneumoniae* serotype O3, O3b and/or O5 O-antigen of the lipopolysaccharide, recognize specifically *K. pneumoniae* serotype 03, O3b and/or 05 bacteria and opsonize them for killing by phagocytes, thus conferring protection against *K. pneumoniae* serotype O3, O3b and/or 05 bacteria.

The saccharides of the present invention overcome all the problems associated with the saccharides produced from bacterial sources and conjugates thereof in terms of purity and easiness of production. It is well known that the isolation and purification of pure saccharides of defined length and structure from the O-antigen of lipopolysaccharides of pathogenic bacteria is a tedious and sometimes not feasible process. Firstly, the production of the O-antigens of lipopolysaccharides requires optimization of the growth conditions. Secondly, depolymerization conditions under which the structural integrity of the constituting monosaccharides is maintained need to be found. Finally, purification conditions enabling the isolation of the pure saccharide of defined length and structure need to be determined. Besides usual contaminants, such as cellular polysaccharides, nucleic acids, lipids and proteins, also the undesired saccharides obtained through the depolymerization process, must be excluded. Thus, the production of pure saccharides of defined structure and length from bacterial sources is a tedious, almost impossible process.

Preferred are synthetic saccharides of general formula (**II**)

**T*-[(-Uₓ₊₄-Uₓ₊₃-Uₓ₊₂-Uₓ₊₁-Uₓ)ₘ-(Vₓ₊₂-Vₓ₊₁-Vₓ)₁₋ₘ]ₙ-O-L-E** (**II**)

wherein m, n, x, L, E, Uₓ₊₁, Uₓ₊₂, Uₓ₊₃, Uₓ₊₄, Vₓ, Vₓ₊₁, Vₓ₊₂ and T* have the meanings as defined herein.
Thus, a saccharide of general formula (**II-a**), (**II-b**), (**II-c**), (**II-d**), (**II-e**), (**II-f**), (**II-g**), (**II-h**), (**II-i**), (**II-j**) or (**II-k**) wherein n, L, E and T* have the meanings defined herein is especially preferred.

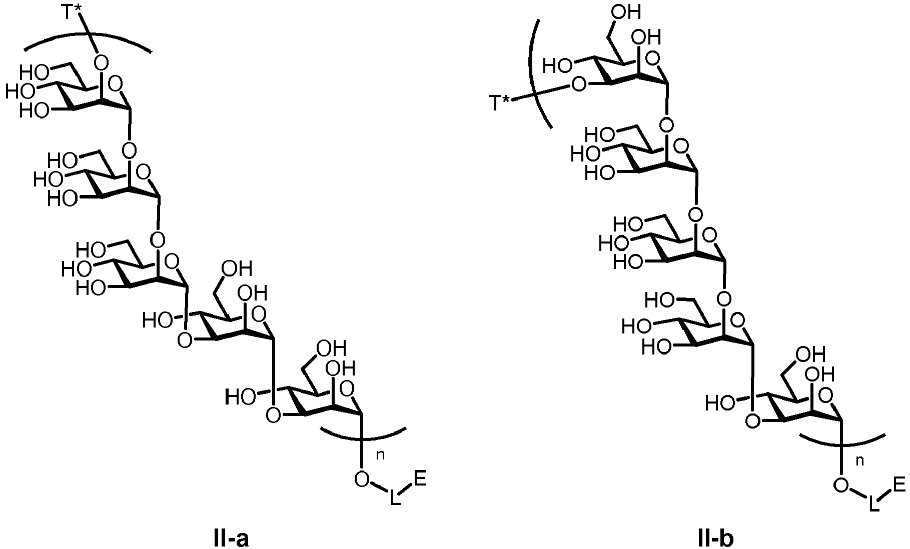

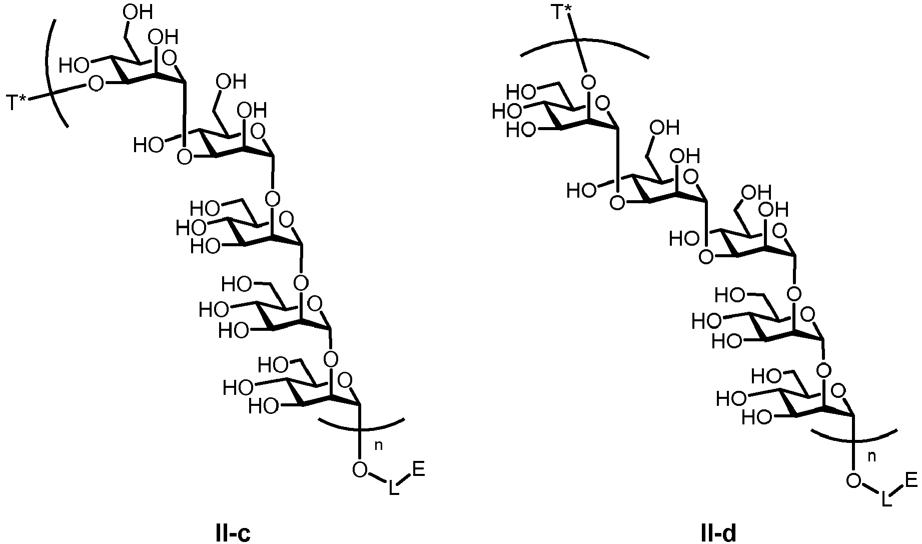

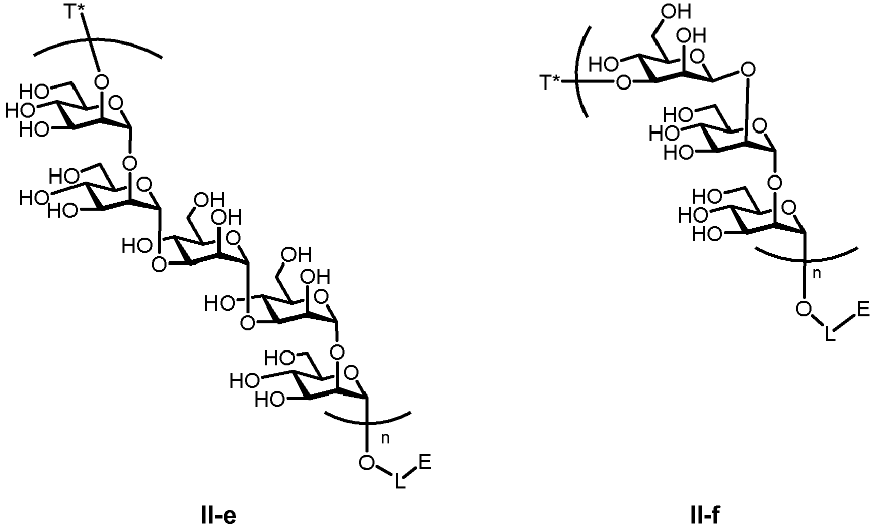

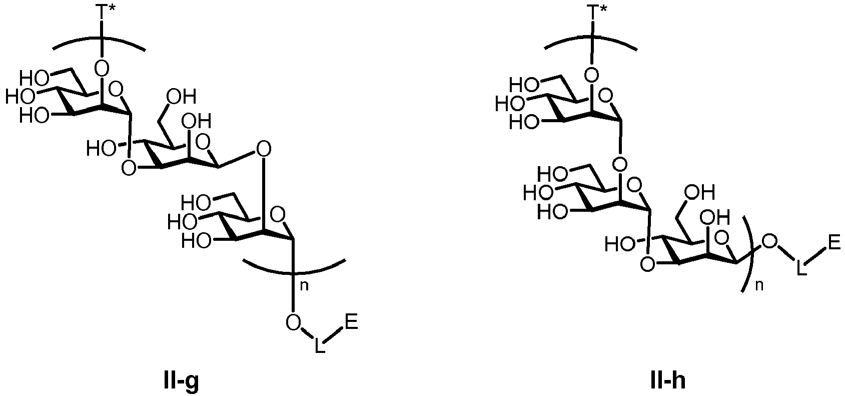

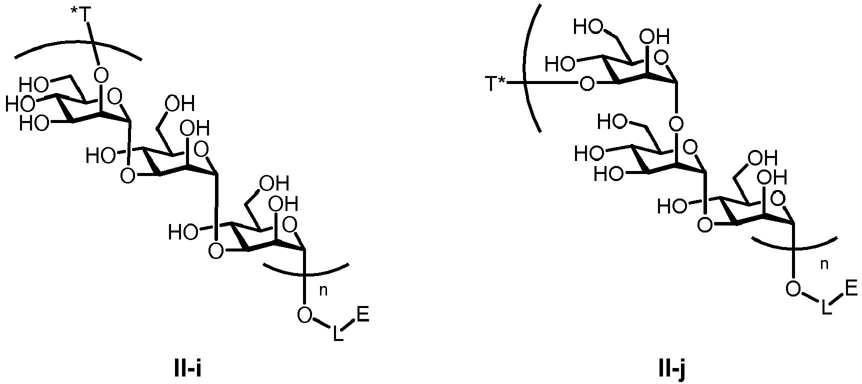

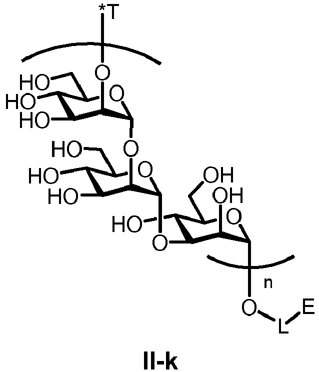

Also preferred are synthetic saccharides of general formula (**III**)

**T*-[(-Uₓ₊₄-Uₓ₊₃-Uₓ₊₂-Uₓ₊₁-Uₓ)ₘ-(Vₓ₊₂-Vₓ₊₁-Vₓ)₁₋ₘ]ₙ-(Uₓ₊₄)ₘ-(Vₓ₊₂)₁₋ₘ-O-L-E** (**III**)

wherein m, n, x, L, E, Uₓ₊₁, Uₓ₊₂, Uₓ₊₃, Uₓ₊₄, Vₓ, Vₓ₊₁, Vₓ₊₂ and T* have the meanings as defined herein.

Thus, a saccharide of general formula (**III-a**), (**III-b**), (**III-c**), (**III-d**), (**III-e**), (**III-f**), (**III-g**), (**III-h**), (**III-i**), (**III-j**) and (**III-k**) wherein n, L, E and T* have the meanings defined herein is especially preferred. 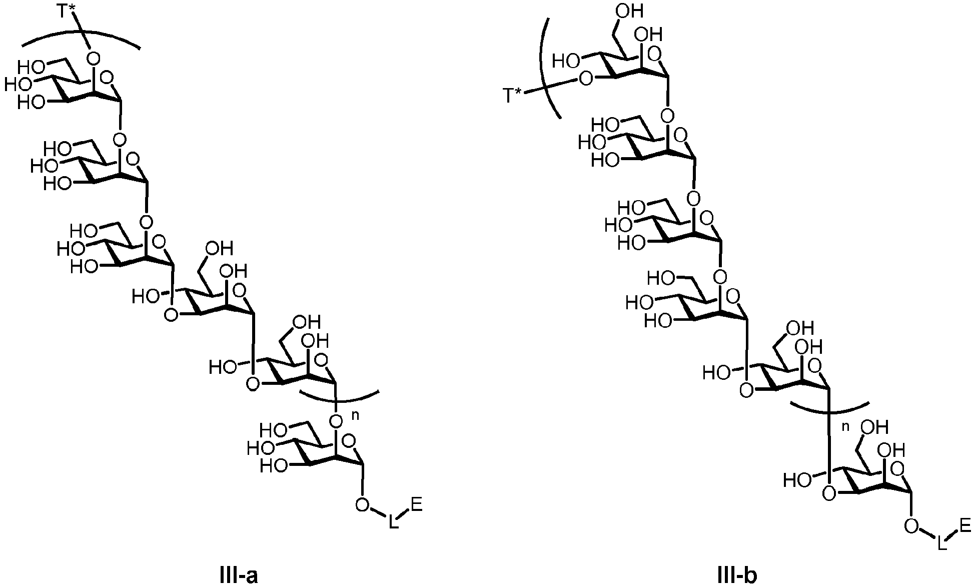 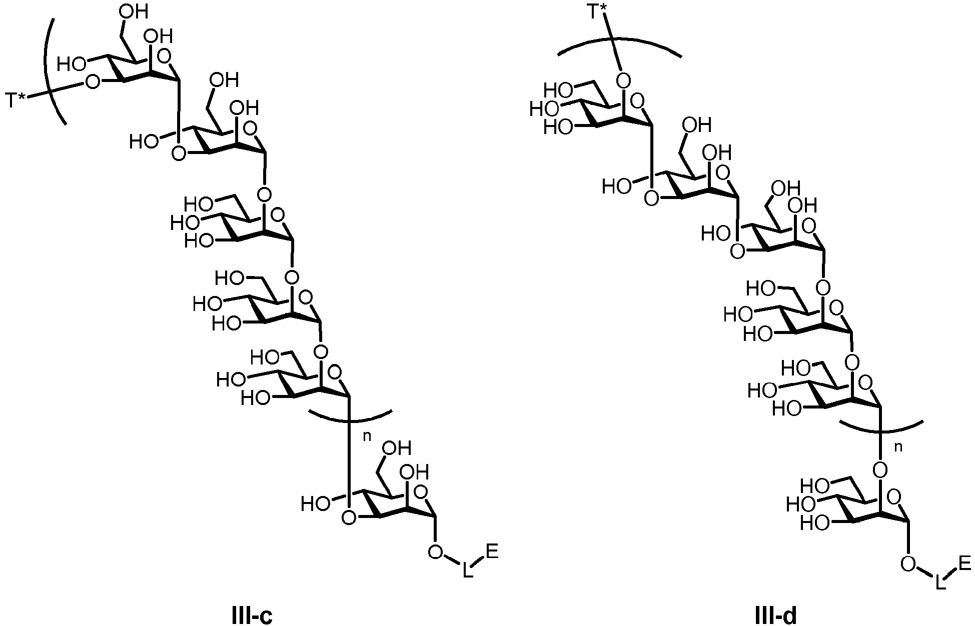 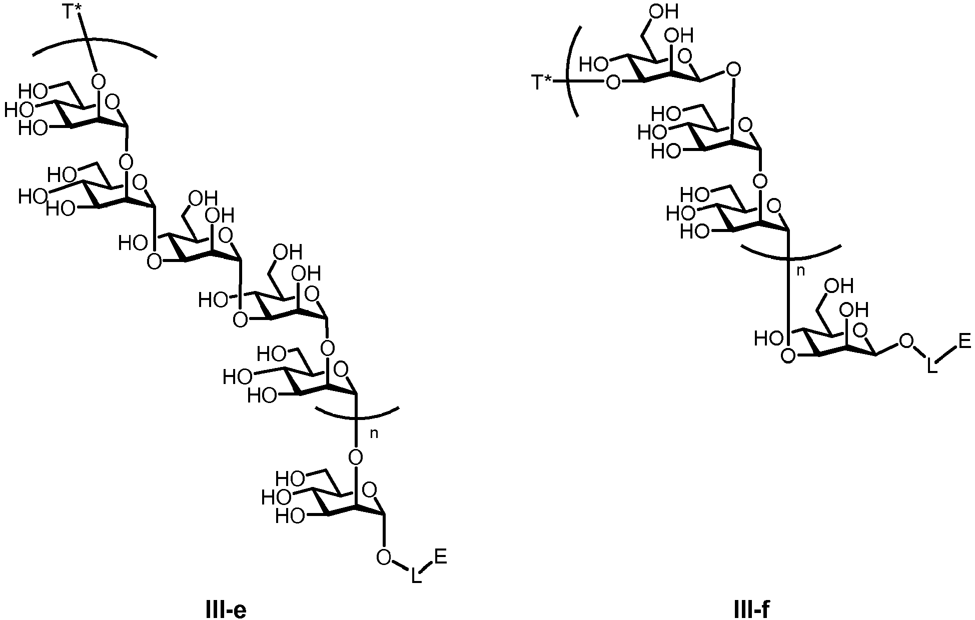 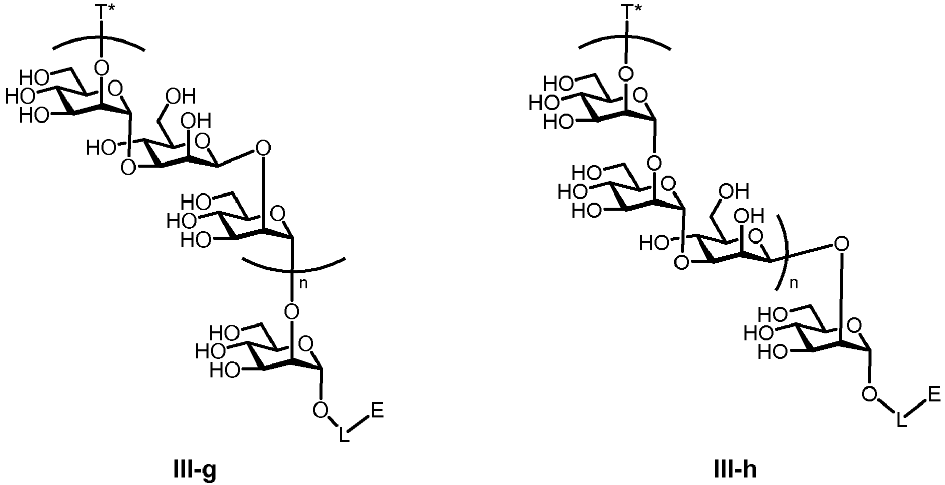 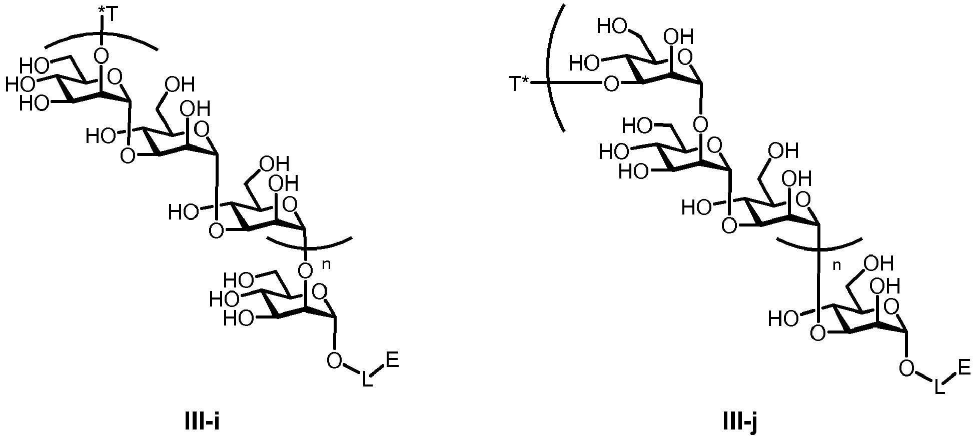 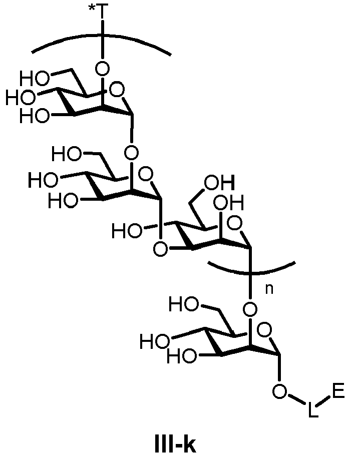 wherein n, L and E have the meanings as defined herein.

Preferably, the integer x represents 1, Therefore, a compound of general formula (**I**), (**II**) or (**III**), wherein x represents 1 is especially preferred. Even more preferred is a compound of general formula (**I**), (**II**) or (**III**), wherein x represents 1 and **T*** represents H-. A compound of general formula (**I**), (**II**) or (**III**), wherein **T*** represents H- is also preferred.

Preferably, n represents an integer selected from 2 to 10. Hence, a saccharide of general formula (**I**), (**II**), (**II-a**), (**II-b**), (**II-c**), (**II-d**), (**II-e**), (**II-f**), (**II-g**), (**II-h**), (**II-i**), (**II-j**), (**II-k**), (**III**), (**III-a**), (**III-b**), (**III-c**), (**III-d**), (**III-e**), (**III-f**), (**III-g**), (**III-h**), (**III-i**), (**III-j**) or (**III-k**), wherein n represents an integer selected from 2 to 10 is especially preferred. In an alternative embodiment, the integer is preferably 1. Hence, a saccharide of general formula (**I**), (**II**), (**II-a**), (**II-b**), (**II-c**), (**II-d**), (**II-e**), (**II-f**), (**II-g**), (**II-h**), (**II-i**), (**II-j**), (**II-k**), (**III**), (**III-a**), (**III-b**), (**III-c**), (**III-d**), (**III-e**), (**III-f**), (**III-g**), (**III-h**), (**III-i**), (**III-j**) or (**III-k**), wherein n represents 1 is also preferred.

Preferably the linker **-L-** represents **-L^{a}-, -L^{a}-L^{e}-, -L^{a}-L^{b}-L^{e}-,** or **-L^{a}-L^{d}-L^{e}-; -L^{a}-** represents -(CH₂)ₒ-, -(CH₂-CH₂-O)ₒ-C₂H₄-, or -(CH₂-CH₂-O)ₒ-CH₂;
**-L^{b}-** represents -O-;
**-L^{d}-** represents -(CH₂)_{q}-, -(CH(OH))_{q}-, -(CF₂)_{q}-, -(CH₂-CH₂-O)_{q}-C₂H₄-, or
-(CH₂-CH₂-O)_{q}-CH₂-;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂-; and
**o, q, p1** and **p2** are independently of each other an integer selected from 1, 2, 3, 4, 5, and 6.

Therefore, a saccharide of any one of general formulae (**I**), (**II**), (**II-a**), (**II-b**), (**II-c**), (**II-d**), (**II-e**), (**II-f**), (**II-g**), (**II-h**), (**II-i**), (**II-j**), (**II-k**), (**III**), (**III-a**), (**III-b**), (**III-c**), (**III-d**), (**III-e**), (**III-f**), (**III-g**), (**III-h**), (**III-i**), (**III-j**) or (**III-k**), wherein
**-L-** represents **-L^{a}- -L^{a}-L^{e}-, -L^{a}-L^{b}-L^{e}-,** or **-L^{a}-L^{d}-L^{e}-** ;
**-L^{a}-** represents -(CH₂)ₒ-, -(CH₂-CH₂-O)ₒ-C₂H₄-, or -(CH₂-CH₂-O)ₒ-CH₂;
**-L^{b}-** represents -O-;
**-L^{d}-** represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂-;
**-L^{e}-** represents -(CH₂)ₚ₁-, -(CF₂)ₚ₁-, -C₂H₄-(O-CH₂-CH₂)ₚ₁-,
-CH₂-(O-CH₂-CH₂)ₚ₁- or -(CH₂)ₚ₁-O-(CH₂)ₚ₂-; and
**o, q, p1** and **p2** are independently of each other an integer selected from 1, 2, 3, 4, 5, and 6 is especially preferred.

A saccharide of any one of general formulae (**I**), (**II**), (**II-a**), (**II-b**), (**II-c**), (**II-d**), (**II-e**), (**II-f**), (**II-g**), (**II-h**), (**II-i**), (**II-j**), (**II-k**), (**III**), (**III-a**), (**III-b**), (**III-c**), (**III-d**), (**III-e**), (**III-f**), (**III-g**), (**III-h**), (**III-i**), (**III-j**) or (**III-k**), wherein
-L- is selected from: -L^{a}-, -L^{a}-L^{e}-, -L^{a}-L^{b}-L^{e}-, and -L^{a}-L^{d}-L^{e}-;
-L^{a}- is selected from: -(CH₂)ₒ-, -(CH₂-CH₂-O)ₒ-C₂H₄-, -(CH₂-CH₂-O)ₒ-CH₂; -L^{b}- represents -O-;
-L^{d}- is selected from: -(CH₂)_{q}-, -(CF₂)_{q}-, -(CH₂-CH₂-O)_{q}-C₂H₄-, and -(CH₂-CH₂-O)_{q}-CH₂-;
-L^{e}- is selected from: -(CH₂)ₚ₁-, -(CF₂)ₚ₁-, -C₂H₄-(O-CH₂-CH₂)ₚ₁-, -CH₂-(O-CH₂-CH₂)ₚ₁- and -(CH₂)ₚ₁-O-(CH₂)ₚ₂-;
o, q, p1 and p2 are independently of each other an integer selected from 1, 2, 3, 4, 5, and 6; and n represents 1 is also preferred.

Even more preferred is a saccharide of general formula (**I**), (**II**), (**II-a**), (**II-b**), (**II-c**), (**II-d**), (**II-e**), (**II-f**), (**II-g**), (**II-h**), (**II-i**), (**II-j**), (**II-k**), (**III**), (**III-a**), (**III-b**), (**III-c**), (**III-d**), (**III-e**), (**III-f**), (**III-g**), (**III-h**), (**III-i**), (**III-j**) or (**III-k**), wherein -L- represents -(CH₂)ₒ- and o is an integer selected from 2, 3, 4, 5 and 6.

Also preferred is a saccharide of general (**I**), (**II**), (**II-a**), (**II-b**), (**II-c**), (**II-d**), (**II-e**), (**II-f**), (**II-g**), (**II-h**), (**II-i**), (**II-j**), (**II-k**), (**III**), (**III-a**), (**III-b**), (**III-c**), (**III-d**), (**III-e**), (**III-f**), (**III-g**), (**III-h**), (**III-i**), (**III-j**) or (**III-k**), wherein **-L-** represents -(CH₂)ₒ-, o is an integer selected from 2, 3, 4, 5 and 6, and n represents 1.

In the most preferred embodiment, -O-**L-E** is selected from the group consisting of: 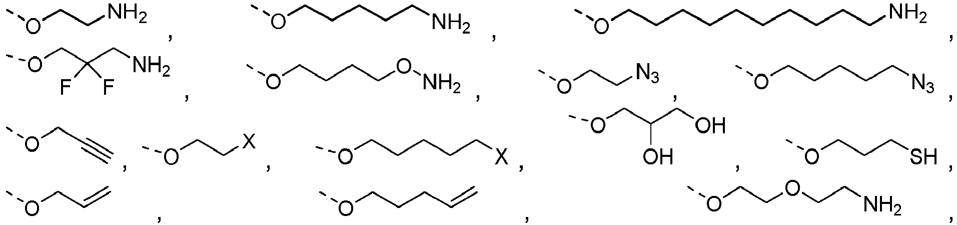 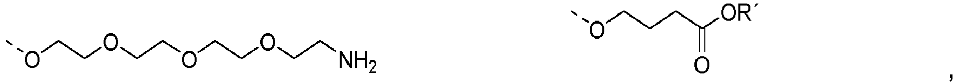 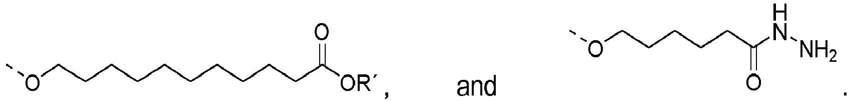
wherein R' represents -H, -Me, -Et, 4-nitrophenyl, pentafluorophenyl, or *N*-hydroxysuccinimidyl;
X represents -Br, -CI, -I, -CO₂H, or -SAc.

Also preferred is a saccharide of general formula (**I**), (**II**), (**II-a**), (**II-b**), (**II-c**), (**II-d**), (**II-e**), (**II-f**), (**II-g**), (**II-h**), (**II-i**), (**II-j**), (**II-k**), (**III**), (**III-a**), (**III-b**), (**III-c**), (**III-d**), (**III-e**), (**III-f**), (**III-g**), (**III-h**), (**III-i**), (**III-j**) or (**III-k**), wherein -L- represents -(CH₂)ₒ-, o is an integer selected from 2, 3, 4, 5 and 6 E represents an amino group.

In yet another preferred embodiment, the saccharide according to the present invention is selected from the group consisting of:

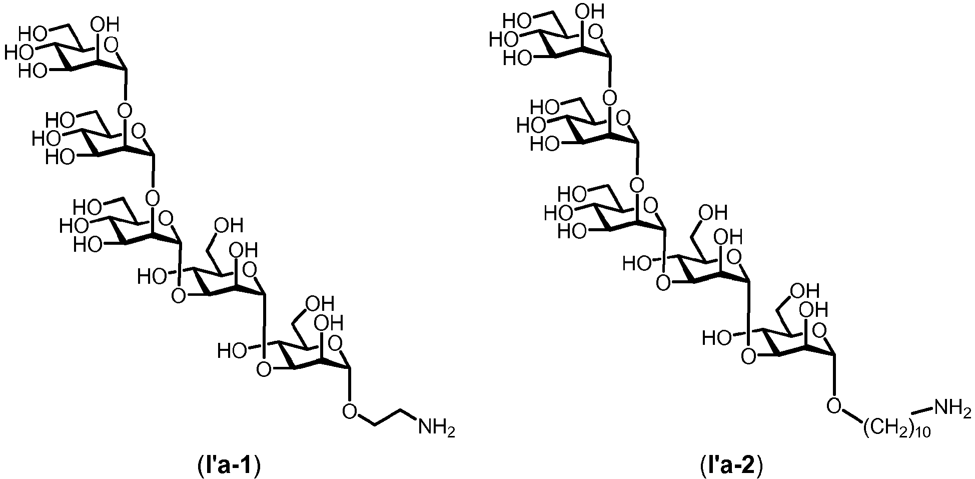

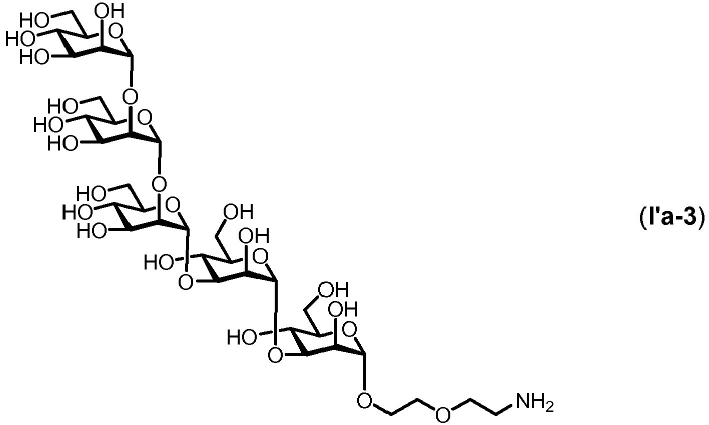

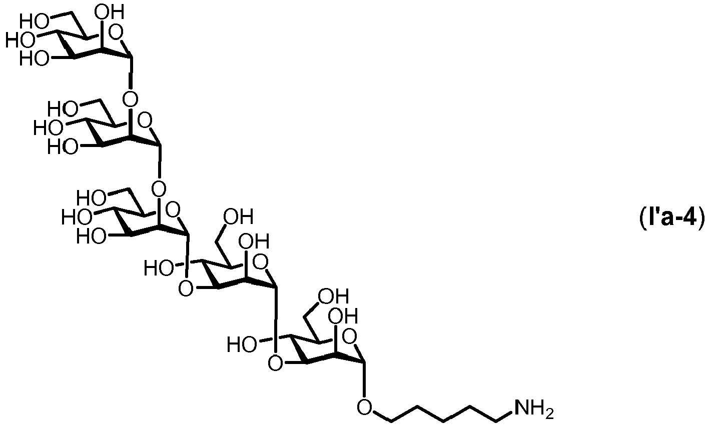

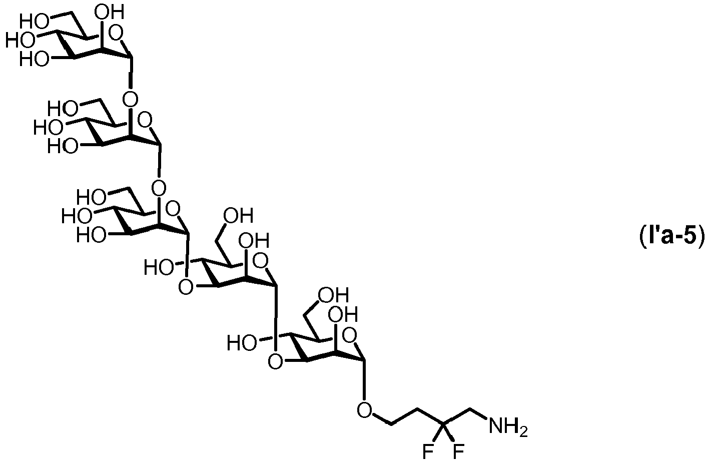

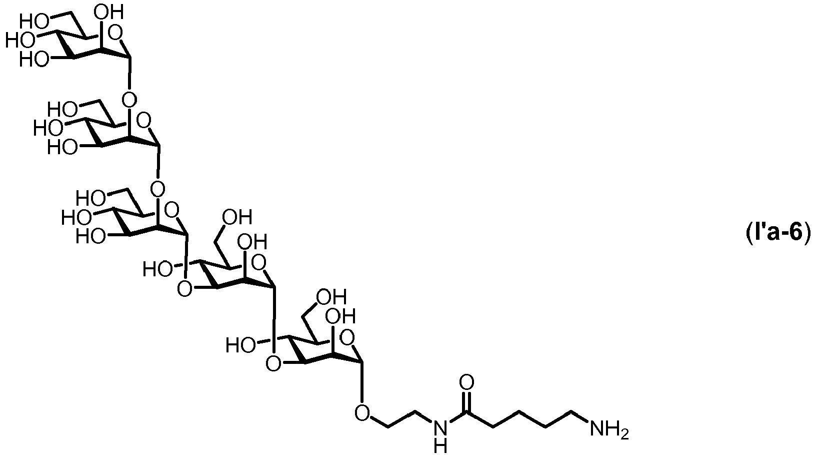

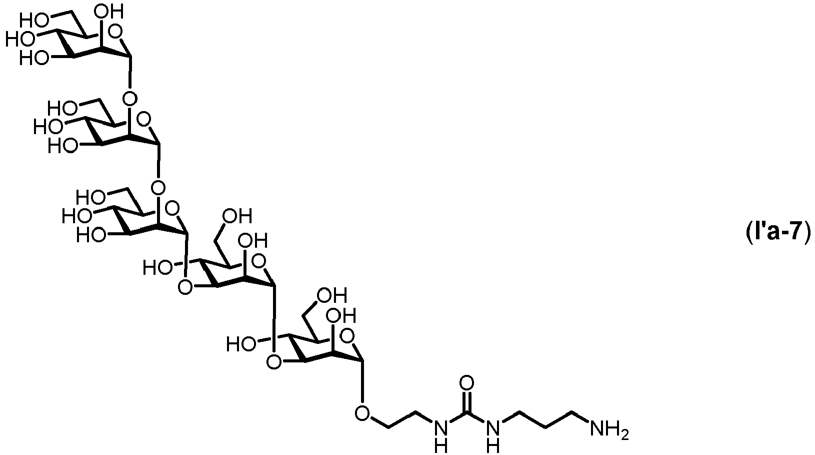

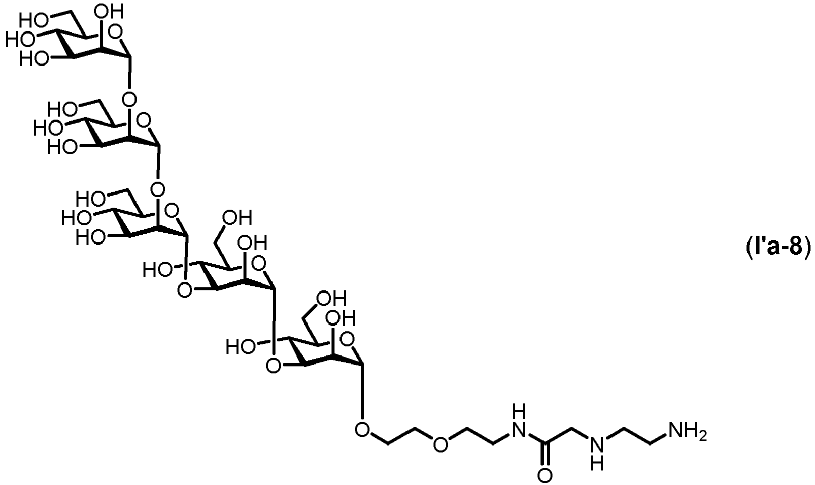

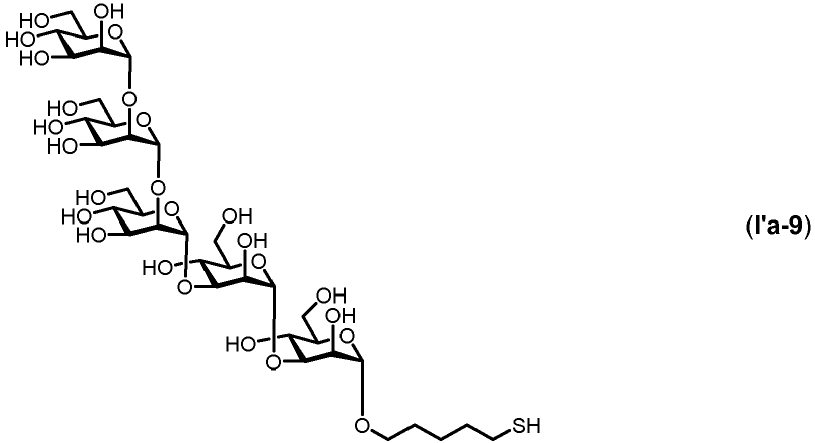

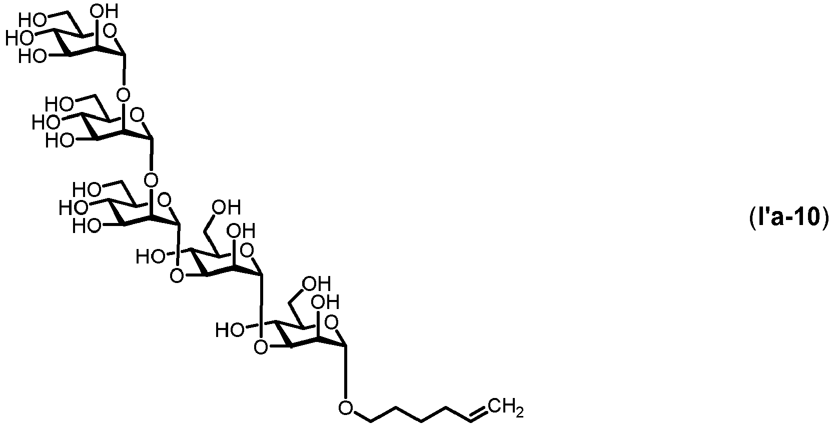

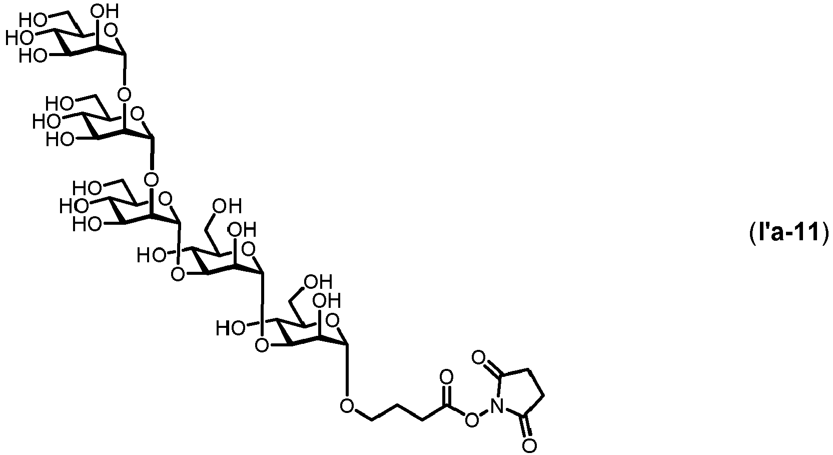

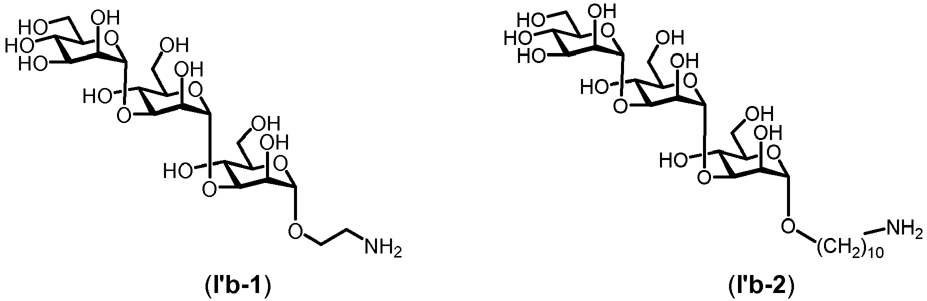

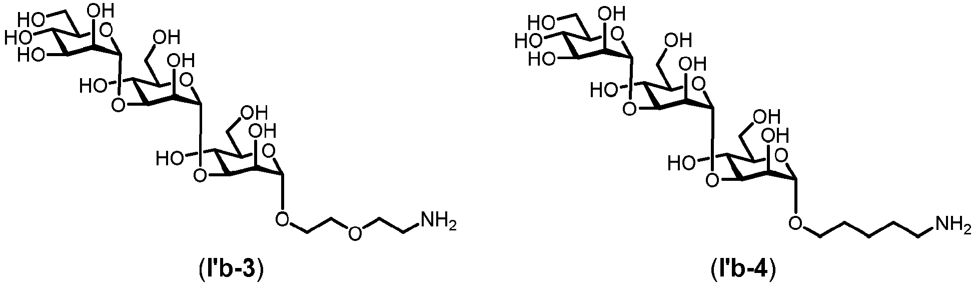

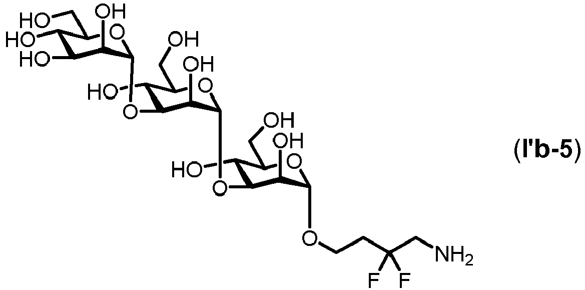

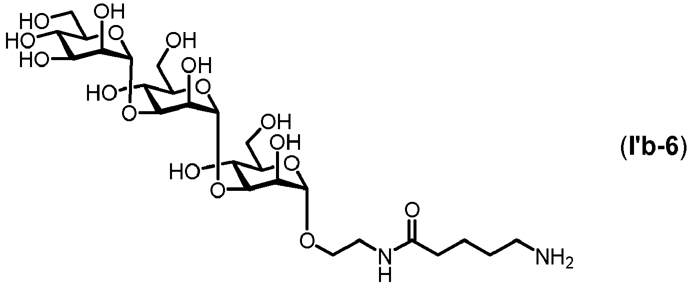

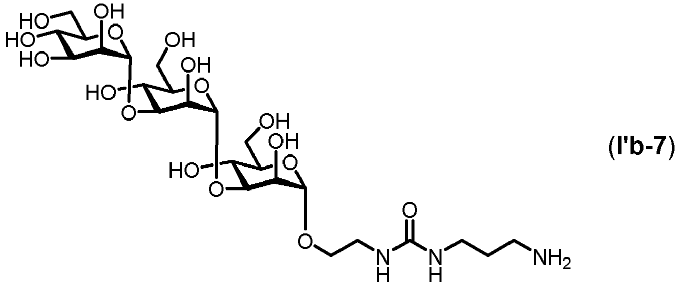

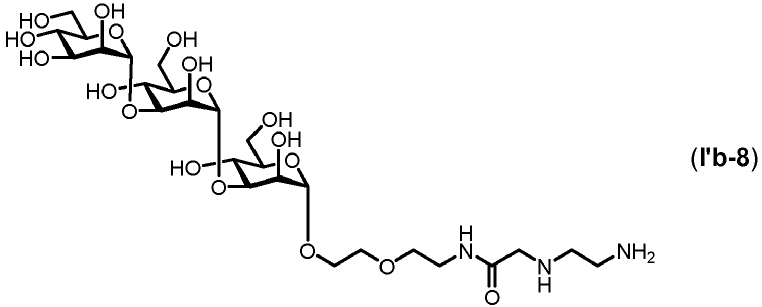

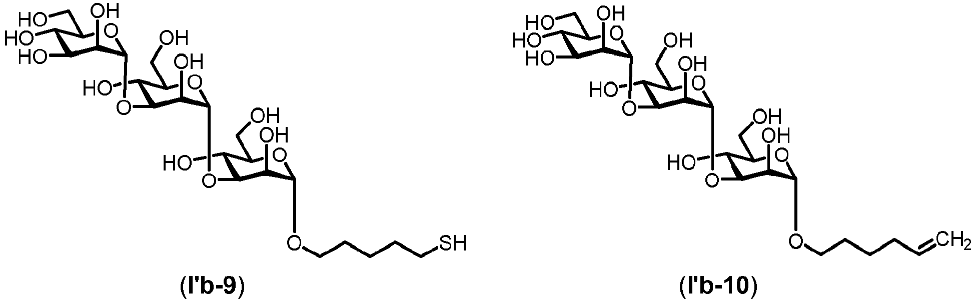

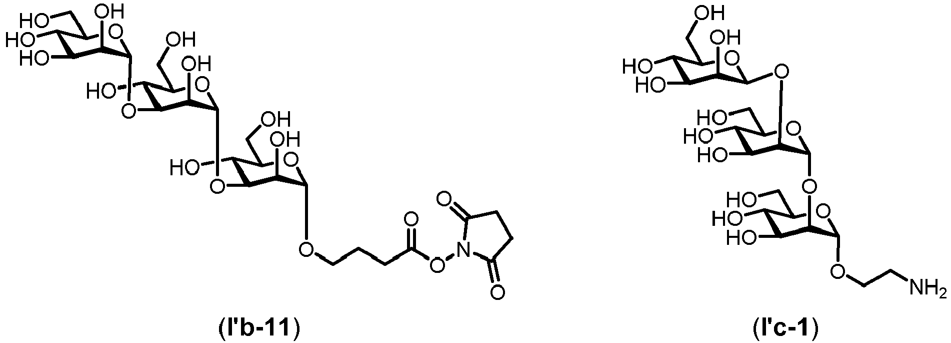

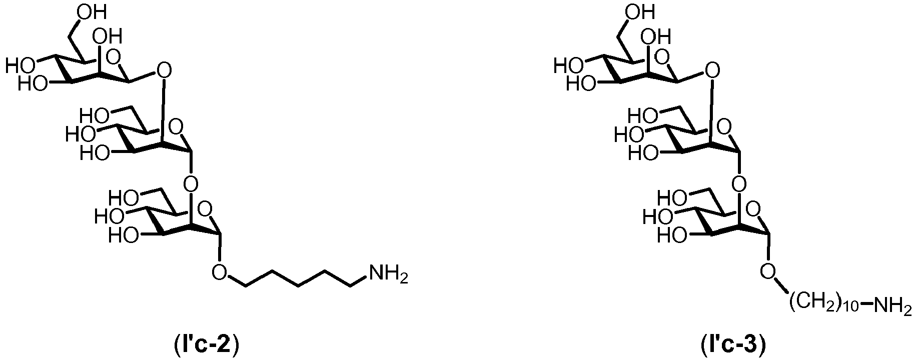

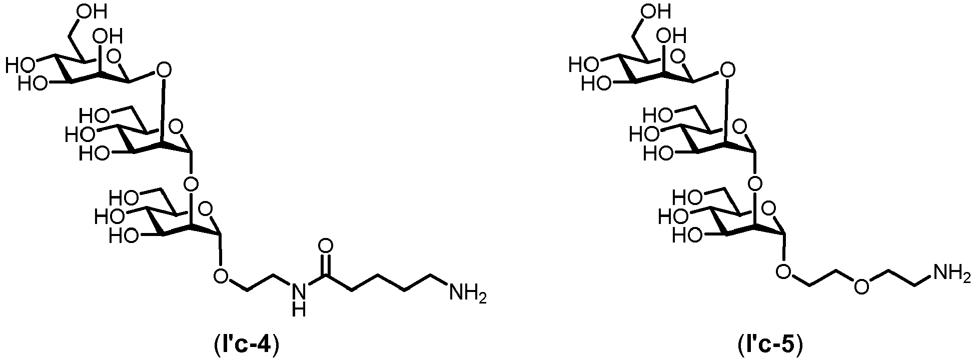

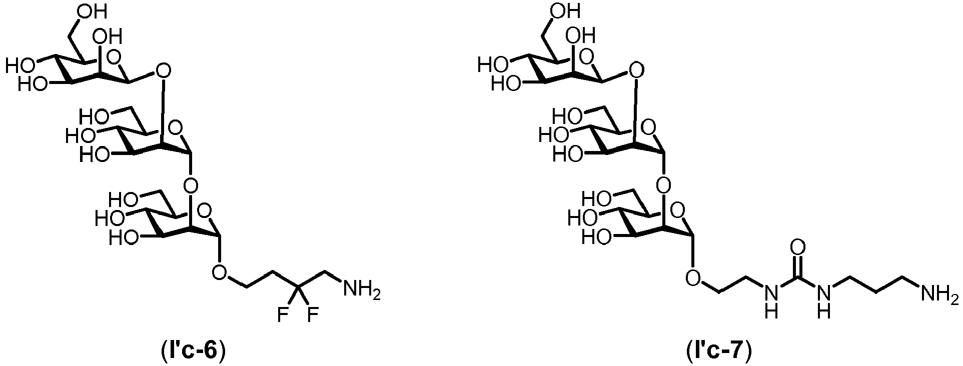

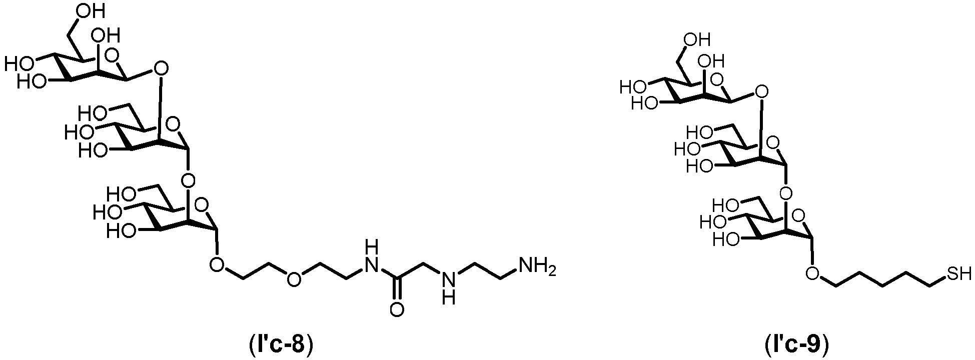

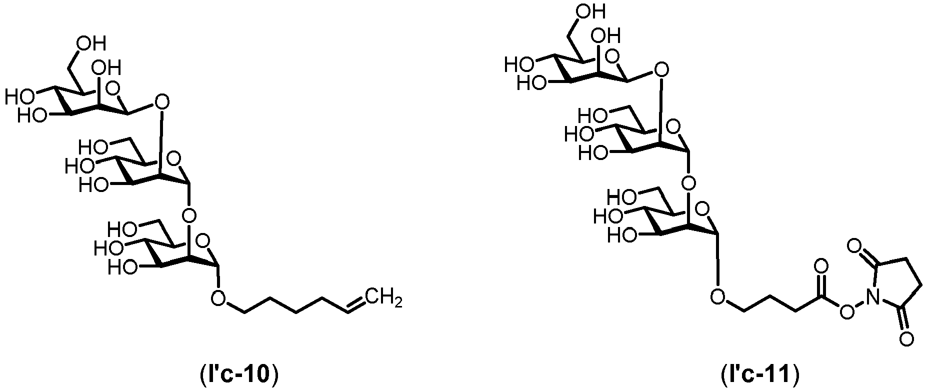

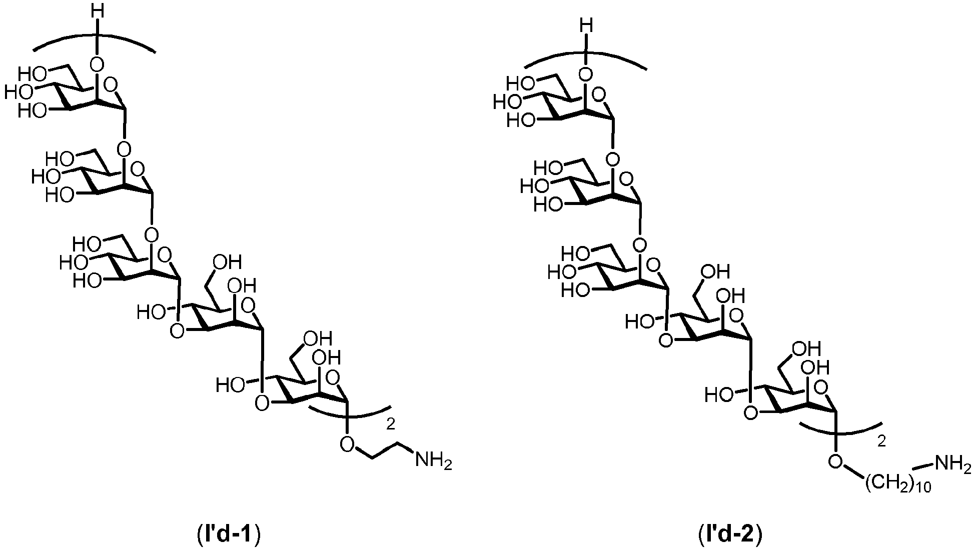

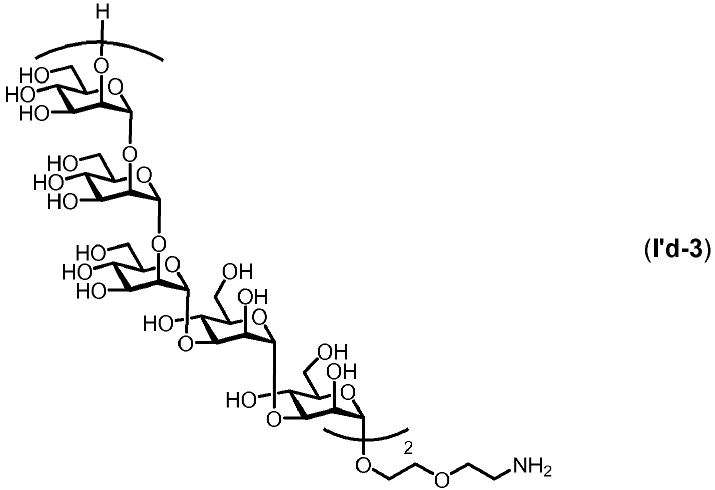

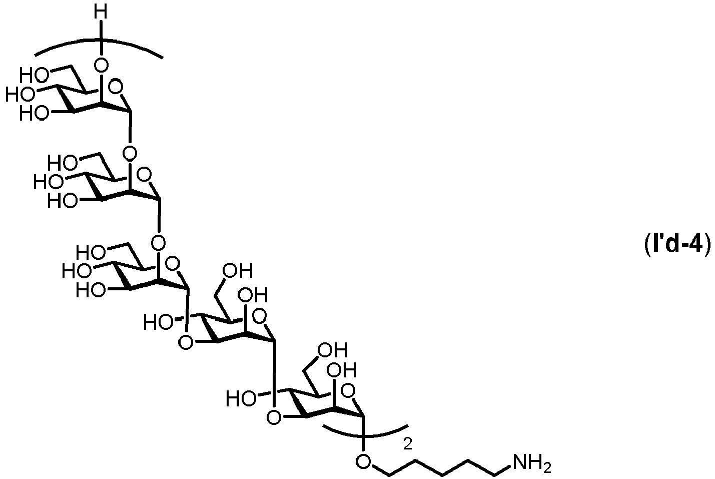

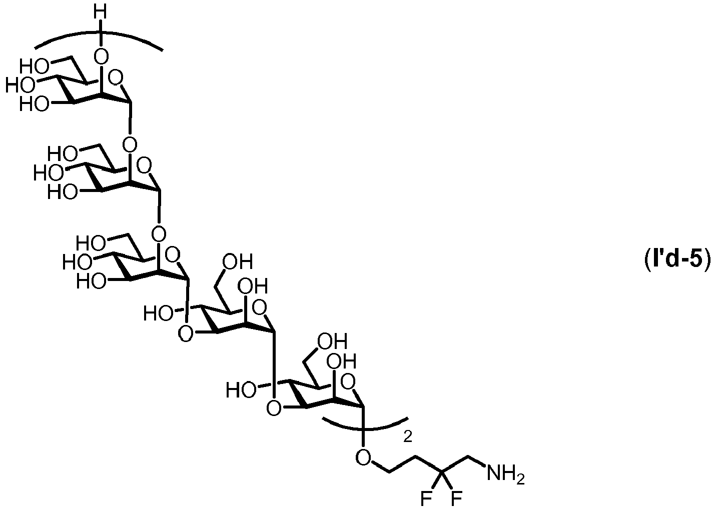

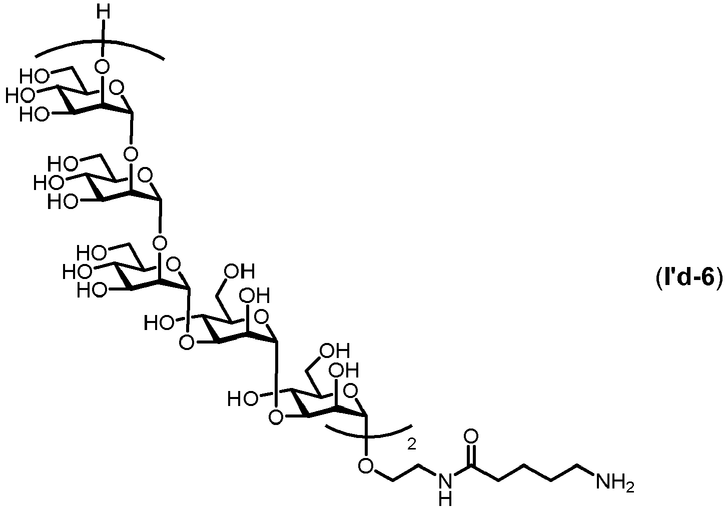

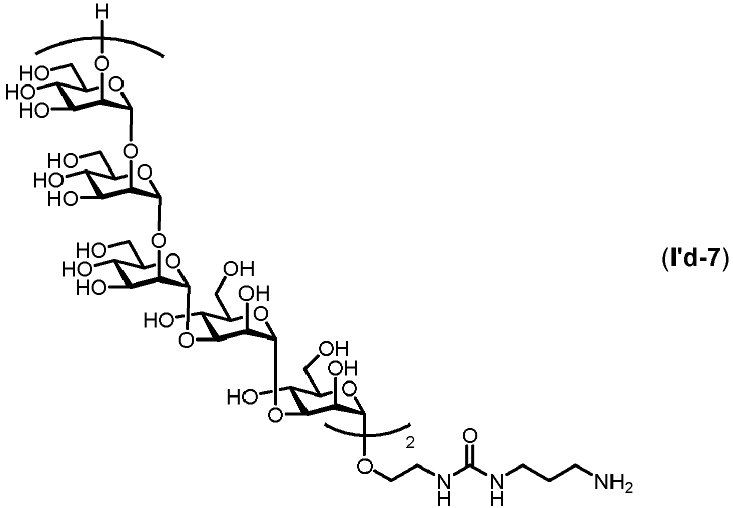

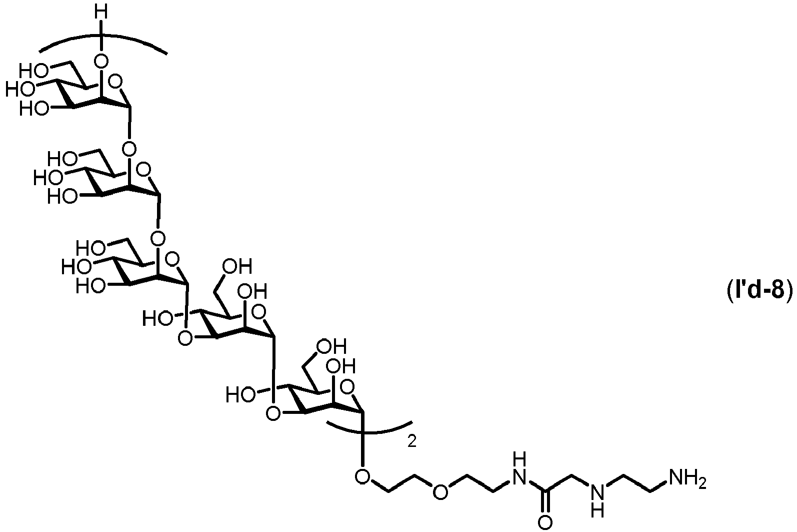

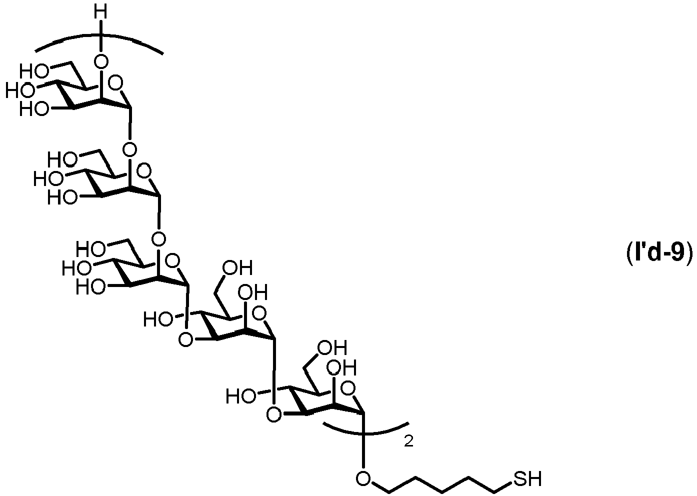

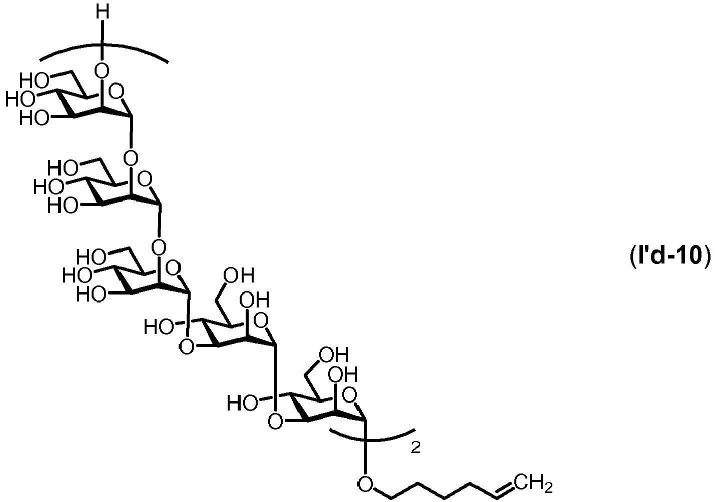

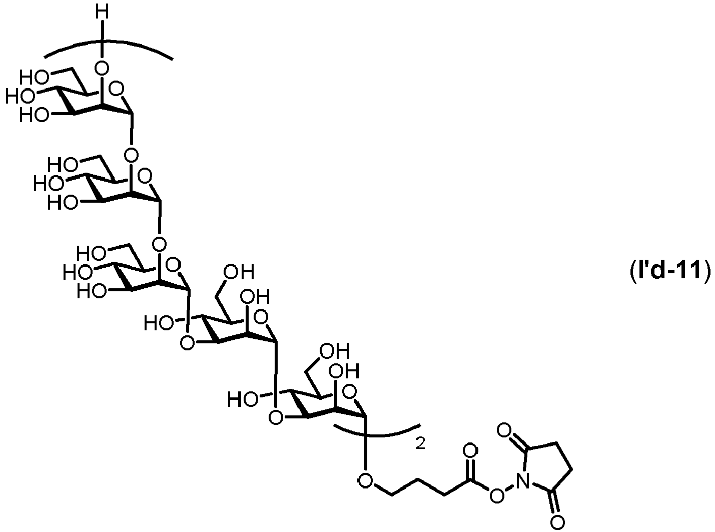

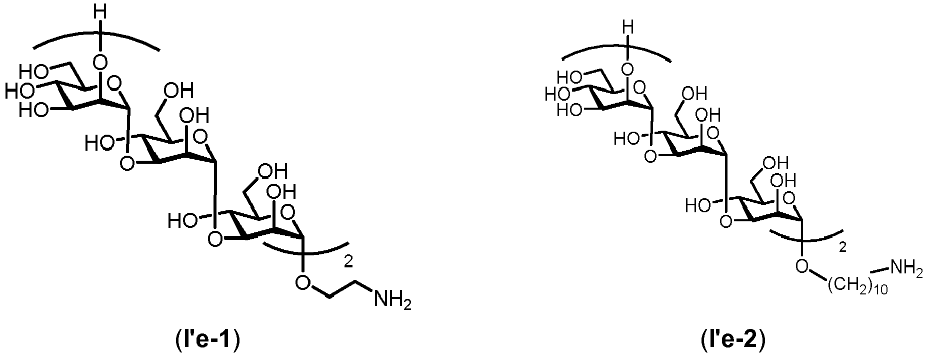

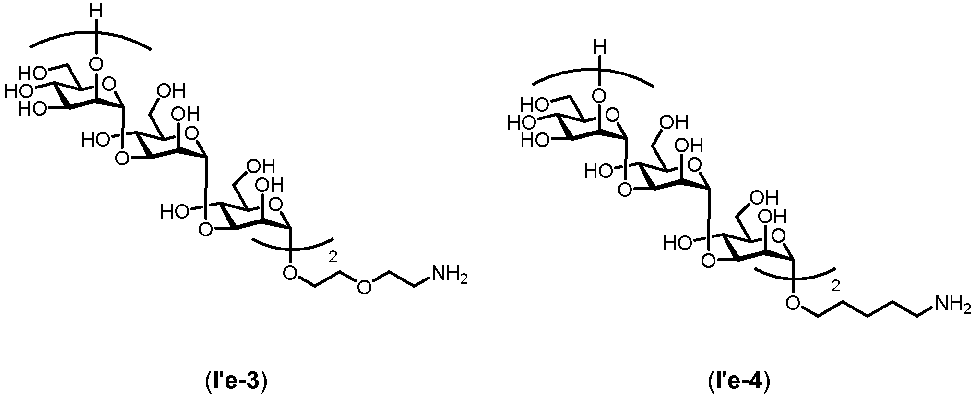

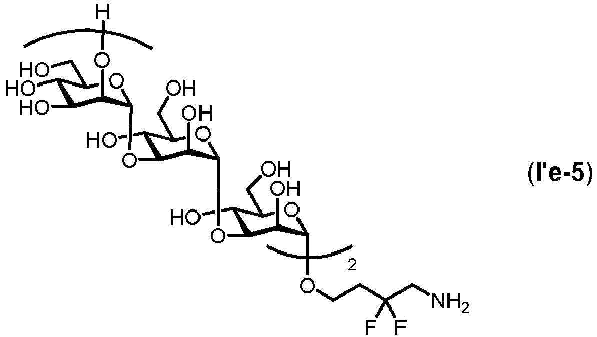

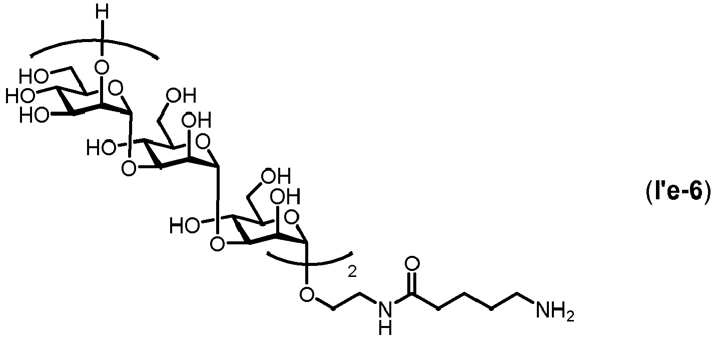

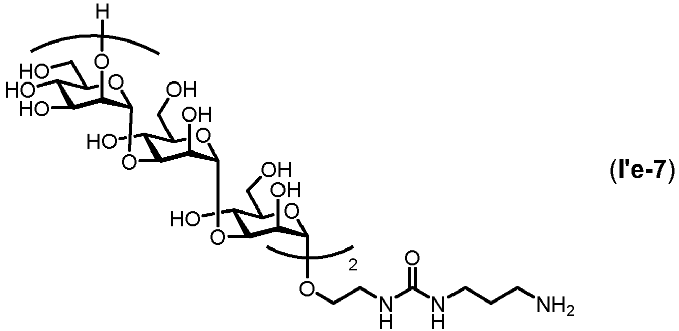

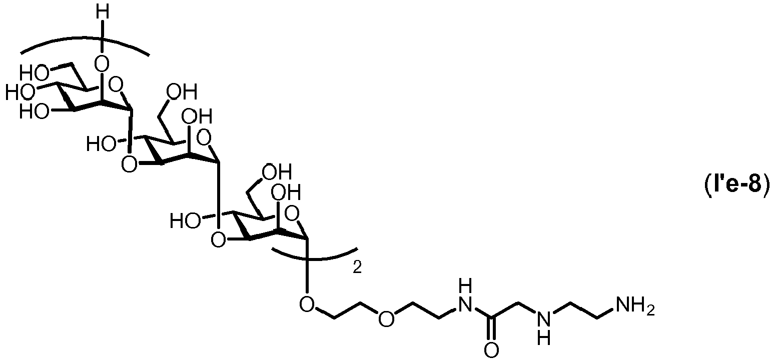

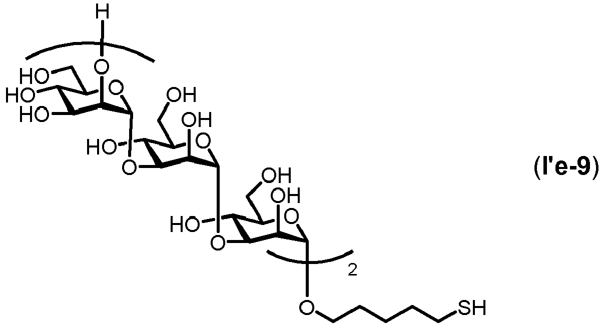

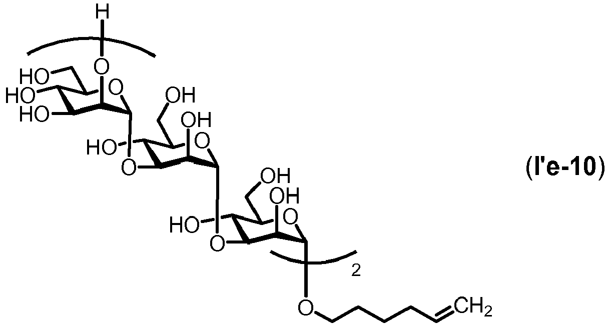

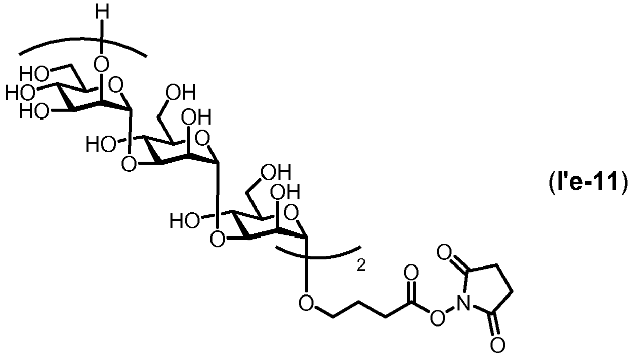

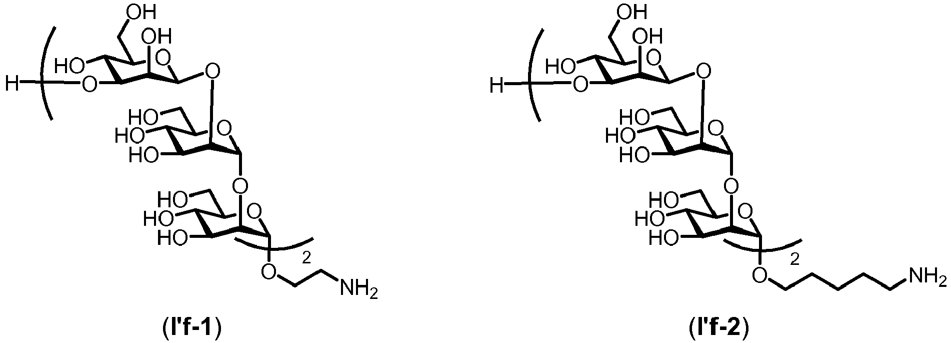

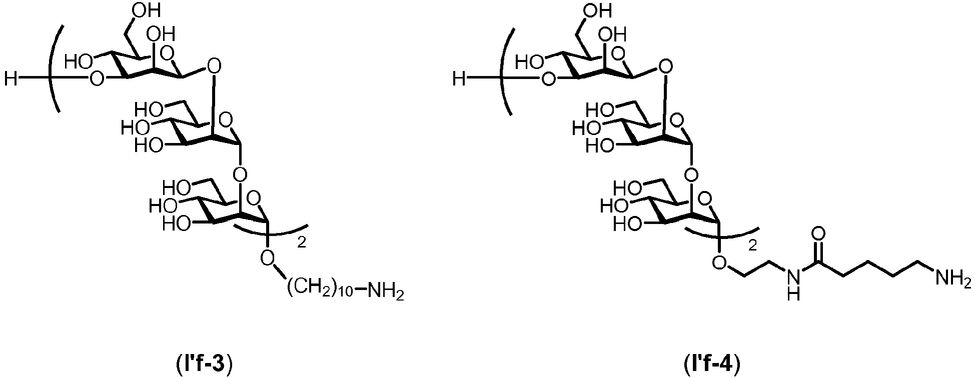

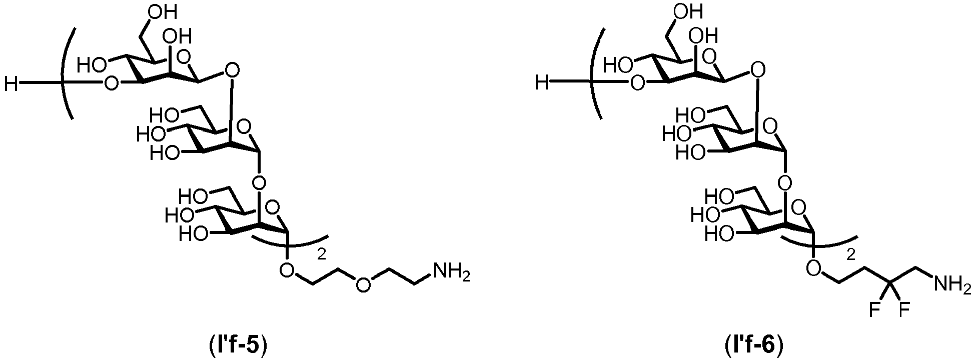

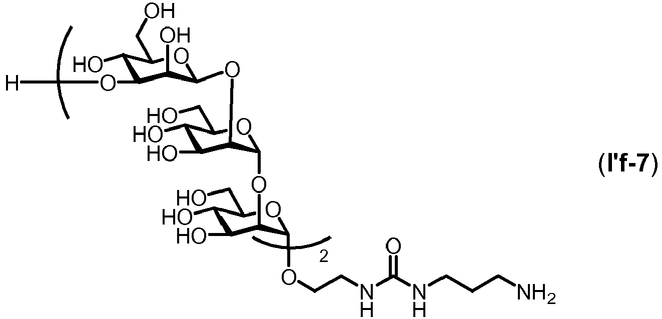

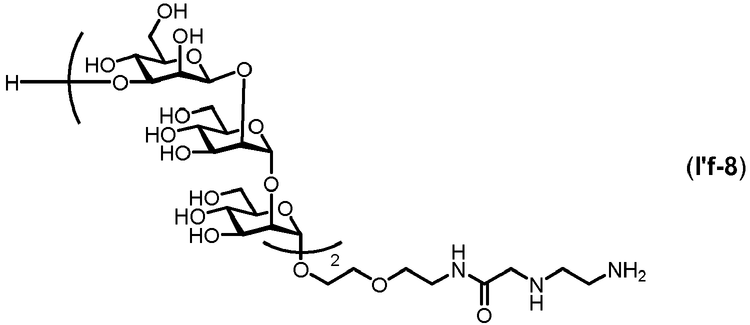

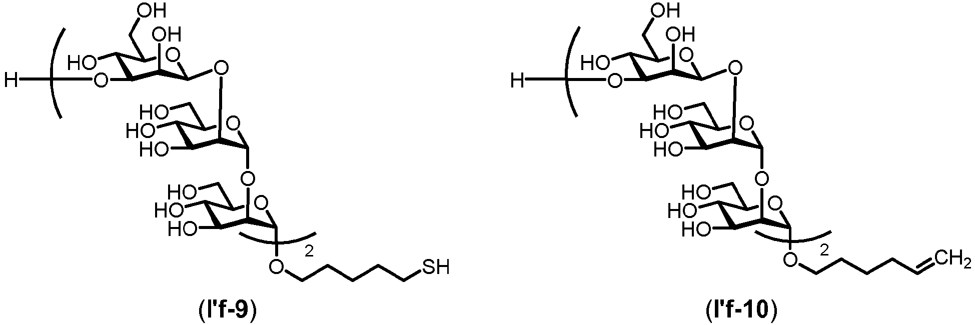

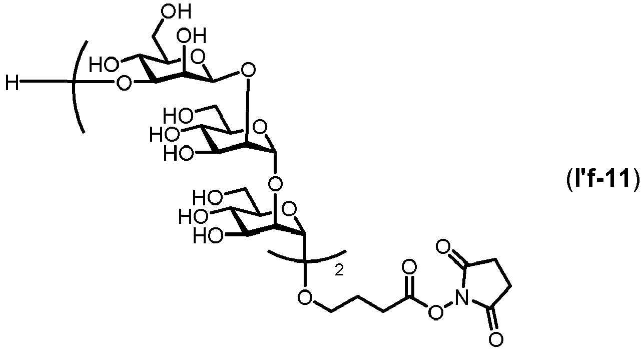

### Chemical synthesis

Another aspect of the present invention is directed to a method of synthesis of a saccharide of general formula **(I)**

**T*-[(-Uₓ₊₄-Uₓ₊₃-Uₓ₊₂-Uₓ₊₁-Uₓ)ₘ-(Vₓ₊₂-Vₓ₊₁-Vₓ)₁₋ₘ]ₙ-T-O-L-E** (**I**)

wherein
m is 1;
x is an integer selected from 1 to 2 × m + 3;
n is an integer selected from 1, 2, 3, 4, 5, 6 ,7 ,8, 9 and 10; 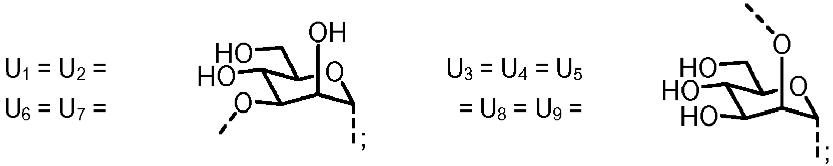 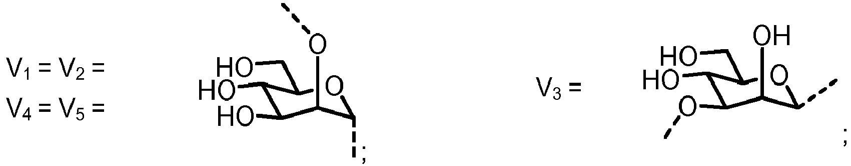 or 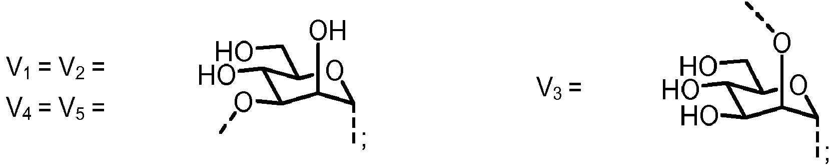
**-T-** represents a bond;
**T*-** represents H;
**L** represents a linker;
**E** represents -NH₂, -N₃, -CN, -O-NH₂, -CH=CH₂, -C=CH, -Br, -CI, -I, -CO₂R', -CONH-NH₂, -SH, or -SAc;
**R'** represents -H, -Me, -Et, 4-nitrophenyl, pentafluorophenyl, or *N*-hydroxysuccinimidyl;
comprising the following steps:
**A1)** providing a monosaccharide 1 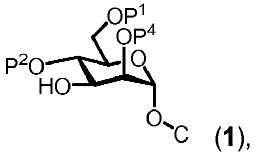 wherein P¹, P² and P⁴ represent protecting groups and C represents -L-Eₚ with Eₚ being a solid support or a protected end group E, wherein E and L have the meanings as defined herein;
**A2)** treating monosaccharide **1** with a building block **2** in presence of an activating agent 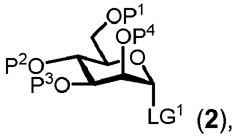 wherein P¹ - P⁴ represent protecting groups and LG¹ represents a leaving group;
**A3)** performing removal of protecting group P³;
**A4)** treating the product of step A3) with a building block **3** in presence of an activating agent 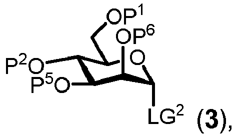 wherein P¹, P², P⁵ and P⁶ represent protecting groups and LG² represents a leaving group;
**A5)** performing removal of protecting group P⁶;
**A6)** repeating steps A4) and A5) two times to obtain intermediate compound **4a**; 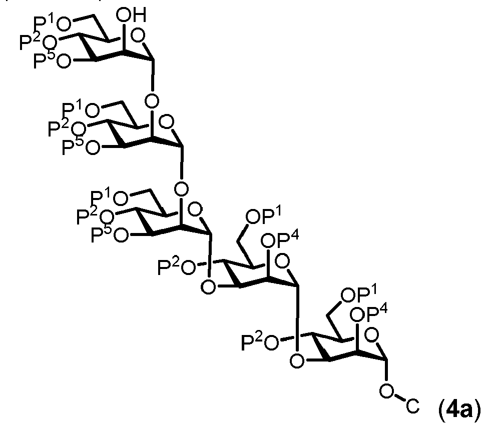 wherein P¹, P², P⁴ and P⁵ represent protecting groups and C represents -L-Eₚ with Eₚ being a solid support or a protected end group E, wherein E and L have the meanings as defined herein;
**A7)** optionally repeating steps A2) - A6) in the following order A2)→A3)→A2)→A3)→ A4)→A5)→A6) n-1 times to obtain intermediate compound of formula **5a,** 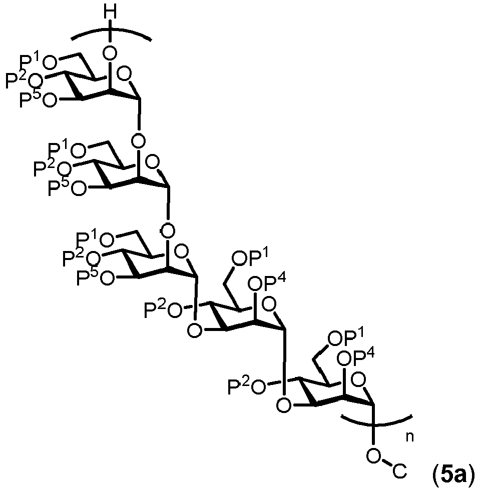 wherein P¹, P², P⁴ and P⁵ represent protecting groups and C represents -L-Eₚ with Eₚ being a solid support or a protected end group E, wherein n, E and L have the meanings as defined herein;
**A8)** performing removal of all protecting groups to obtain a saccharide of general formula (**I**).

A further method of the synthesis of a saccharide of general formula (**I**) comprises the following steps:
**B1)** providing a monosaccharide **1** 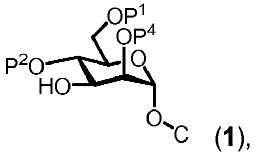 wherein P¹, P² and P⁴ represent protecting groups and C represents -L-Eₚ with Ep being a solid support or a protected end group E, wherein E and L have the meanings as defined herein;
**B2)** treating monosaccharide **1** with a building block **3** in presence of an activating agent 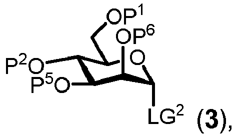 wherein P¹, P², P⁵ and P⁶ represent protecting groups and LG² represents a leaving group;
**B3)** performing removal of protecting group P⁶;
**B4)** repeating steps B2) and B3) two times;
**B5)** treating the product of step B4) with building block **2** in presence of an activating agent 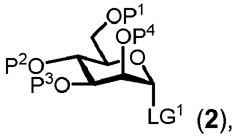 wherein P¹ - P⁴ represent protecting groups and LG¹ represents a leaving group;
**B6)** performing removal of protecting group P³ to obtain intermediate compound **4b**; 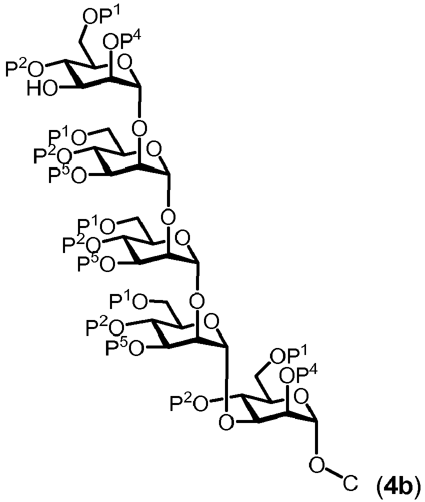 wherein P¹, P², P⁴ and P⁵ represent protecting groups and C represents -L-Eₚ with Eₚ being a solid support or a protected end group E, wherein E and L have the meanings as defined herein;
**B7)** optionally repeating steps B2) - B6) in the following order B5)→B6)→B2)→B3)→ B4)→B5)→B6) n-1 times to obtain intermediate compound of formula **5b** 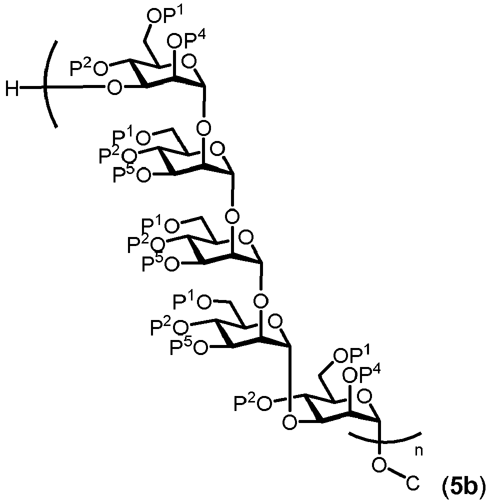 wherein P¹, P², P⁴ and P⁵ represent protecting groups and C represents -L-Eₚ with Ep being a solid support or a protected end group E, wherein n, E and L have the meanings as defined herein;
**B8)** performing removal of all protecting groups to obtain a saccharide of general formula (**I**).

A further method of the synthesis of a saccharide of general formula (**I**) comprises the following steps:
**C1)** providing a monosaccharide **6** 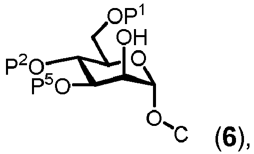 wherein P¹, P² and P⁵ represent protecting groups and C represents -L-Eₚ with Eₚ being a solid support or a protected end group E, wherein E and L have the meanings as defined herein;
**C2)** treating monosaccharide **6** with a building block **3** in presence of an activating agent 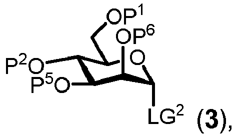 wherein P¹, P², P⁵ and P⁶ represent protecting groups and LG² represents a leaving group;
**C3)** performing removal of protecting group P⁶;
**C4)** treating the product of step C3) building block **2** in presence of an activating agent 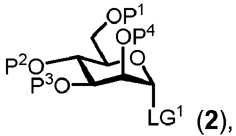 wherein P¹ - P⁴ represent protecting groups and LG¹ represents a leaving group;
**C5)** performing removal of protecting group P³;
**C6)** repeating steps C4) and C5);
**C7)** repeating steps C2) and C3) to obtain intermediate compound **4c**; 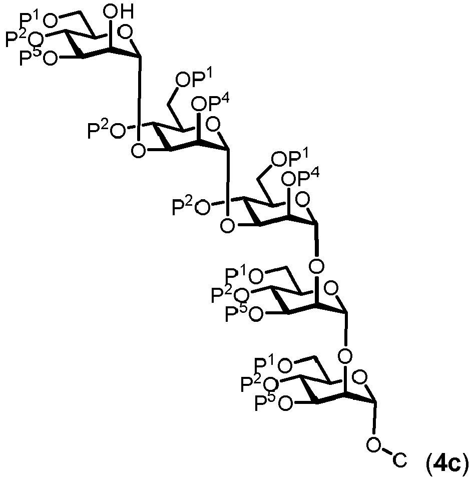 wherein P¹, P², P⁴ and P⁵ represent protecting groups and C represents -L-Eₚ with Eₚ being a solid support or a protected end group E, wherein E and L have the meanings as defined herein;
**C8)** optionally repeating steps C2) - C7) in the following order C2)→C3)→C2)→C3)→ C4)→C5)→C6)→C7) n-1 times to obtain intermediate compound of formula **5c** 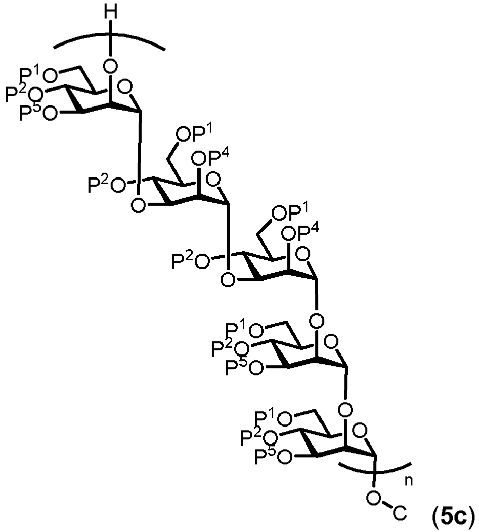 wherein P¹, P², P⁴ and P⁵ represent protecting groups and C represents -L-Eₚ with Ep being a solid support or a protected end group E, wherein n, E and L have the meanings as defined herein;
**C9)** performing removal of all protecting groups to obtain a saccharide of general formula (**I**).

A further method of the synthesis of a saccharide of general formula (**I**) comprises the following steps:
**D1)** providing a monosaccharide **6** 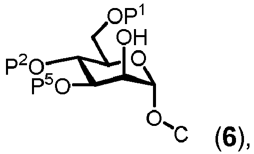 wherein P¹, P² and P⁵ represent protecting groups and C represents -L-Eₚ with Ep being a solid support or a protected end group E, wherein E and L have the meanings as defined herein;
**D2)** treating monosaccharide **6** with a building block **3** in presence of an activating agent 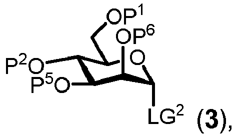 wherein P¹, P², P⁵ and P⁶ represent protecting groups and LG² represents a leaving group;
**D3)** performing removal of protecting group P⁶;
**D4)** repeating steps D2) and D3);
**D5)** treating the product of step D4) building block **2** in presence of an activating agent 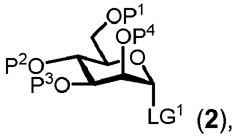 wherein P¹ - P⁴ represent protecting groups and LG¹ represents a leaving group;
**D6)** performing removal of protecting group P³;
**D7)** repeating steps D5) and D6) to obtain intermediate compound **4d**; 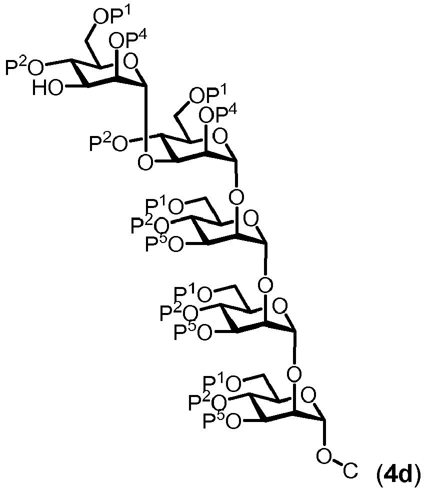 wherein P¹, P², P⁴ and P⁵ represent protecting groups and C represents -L-Eₚ with Ep being a solid support or a protected end group E, wherein E and L have the meanings as defined herein;
**D8)** optionally repeating steps D2)-D7) in the following order D2)→D3)→D4)→D4)→ D5)→D6)→D7) n-1 times to obtain intermediate compound of formula **5d,** 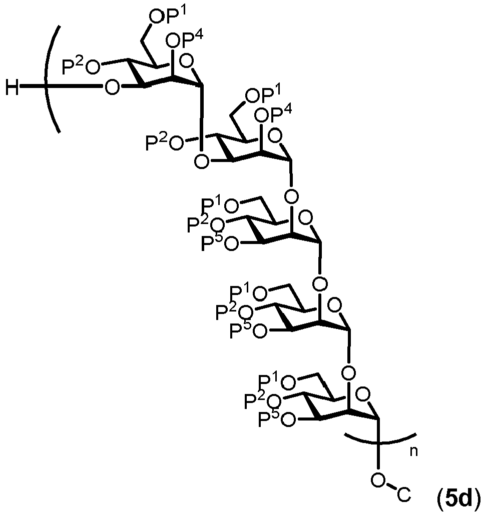 wherein P¹, P², P⁴ and P⁵ represent protecting groups and C represents -L-Eₚ with Ep being a solid support or a protected end group E, wherein n, E and L have the meanings as defined herein;
**D9)** performing removal of all protecting groups to obtain a saccharide of general formula (**I**).

Another method of the synthesis of a saccharide of general formula (**I**) comprises the following steps:
**E1)** providing a monosaccharide **1** 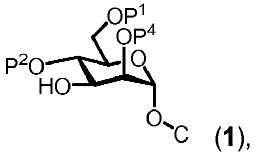 wherein P¹, P² and P⁴ represent protecting groups and C represents -L-Eₚ with Ep being a solid support or a protected end group E, wherein E and L have the meanings as defined herein;
**E2)** treating monosaccharide **1** with a building block **3** in presence of an activating agent 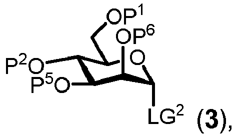 wherein P¹, P², P⁵ and P⁶ represent protecting groups and LG² represents a leaving group;
**E3)** performing removal of protecting group P⁶;
**E4)** repeating steps E2) and E3) two times;
**E5)** treating the product of step E4) building block **2** in presence of an activating agent 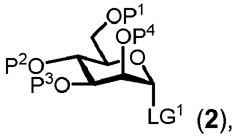 wherein P¹ - P⁴ represent protecting groups and LG¹ represents a leaving group;
**E6)** performing removal of protecting group P³ to obtain intermediate compound **4e**; 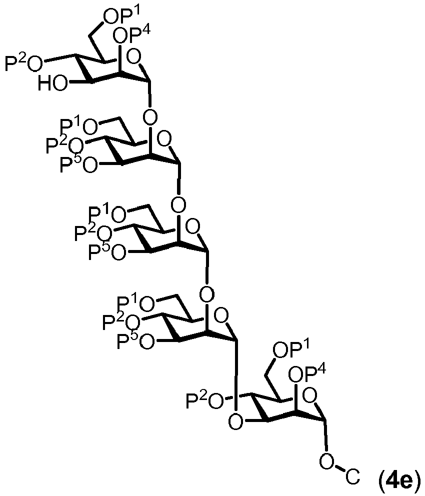 wherein P¹, P², P⁴ and P⁵ represent protecting groups and C represents -L-Eₚ with Ep being a solid support or a protected end group E, wherein E and L have the meanings as defined herein;
**E7)** optionally repeating steps E2) - E6) in the following order E5)→E6)→E2)→E3)→ E4)→E5)→E6) n-1 times to obtain intermediate compound of formula **5e,** 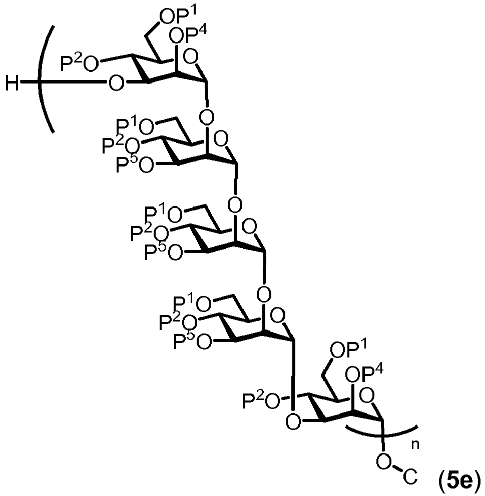 wherein P¹, P², P⁴ and P⁵ represent protecting groups and C represents -L-Eₚ with Ep being a solid support or a protected end group E, wherein n, E and L have the meanings as defined herein;
**E8)** performing removal of all protecting groups to obtain a saccharide of general formula (**I**).

Another method of the synthesis of a saccharide of general formula (**I**) comprises the following steps:
**F1)** providing a monosaccharide **7** 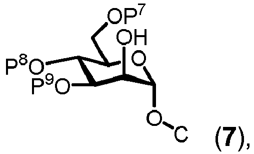 wherein P⁷, P⁸ and P⁹ represent protecting groups and C represents -L-Eₚ with Eₚ being a solid support or a protected end group E, wherein E and L have the meanings as defined herein;
**F2)** treating monosaccharide **7** with a building block **8** in presence of an activating agent 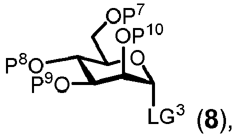 wherein P⁷- P¹⁰ represent protecting groups and LG³ represents a leaving group;
**F3)** performing removal of protecting group P¹⁰;
**F4)** treating the product of step F3) with building block **9** in presence of an activating agent 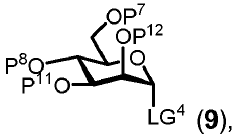 wherein P⁷, P⁸, P¹¹ and P¹² represent protecting groups and LG⁴ represents a leaving group;
**F5)** performing removal of protecting group P¹¹ to obtain intermediate compound **4f;** 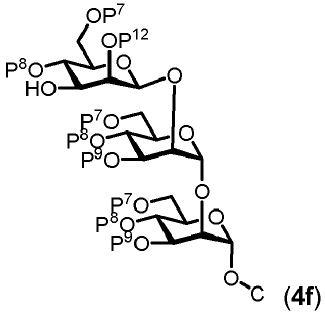 wherein P⁷- P⁹ and P¹² represent protecting groups and C represents -L-Eₚ with Ep being a solid support or a protected end group E, wherein E and L have the meanings as defined herein;
**F6)** optionally repeating steps F2) - F5) in the following order F2)→F3)→F2)→F4)→ F5) n-1 times to obtain intermediate compound of formula **5f,** 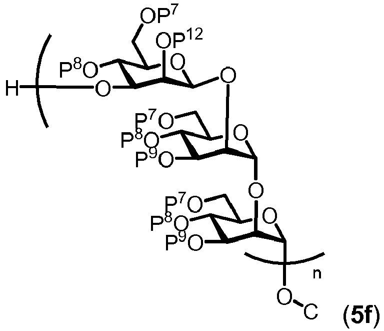 wherein P⁷- P⁹ and P¹² represent protecting groups and C represents -L-Eₚ with Ep being a solid support or a protected end group E, wherein n, E and L have the meanings as defined herein;
**F7)** performing removal of all protecting groups to obtain a saccharide of general formula (**I**).

Another method of the synthesis of a saccharide of general formula (**I**) comprises the following steps:
**G1)** providing a monosaccharide **7** 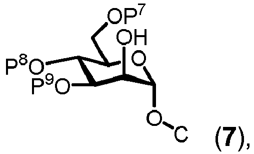 wherein P⁷, P⁸ and P⁹ represent protecting groups and C represents -L-Eₚ with Ep being a solid support or a protected end group E, wherein E and L have the meanings as defined herein;
**G2)** treating monosaccharide **7** with building block **9** in presence of an activating agent 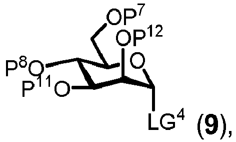 wherein P⁷, P⁸, P¹¹ and P¹² represent protecting groups and LG⁴ represents a leaving group;
**G3)** performing removal of protecting group P¹¹;
**G4)** treating the product of step G3) with building block **8** in presence of an activating agent 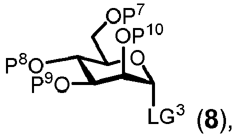 wherein P⁷- P¹⁰ represent protecting groups and LG³ represents a leaving group;
**G5)** performing removal of protecting group P¹⁰ to obtain intermediate compound **4g;** 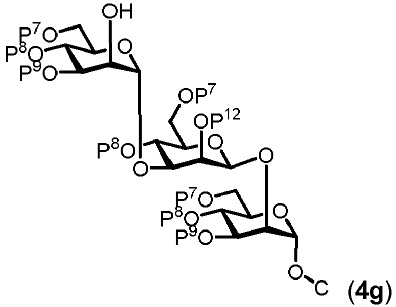 wherein P⁷- P⁹ and P¹² represent protecting groups and C represents -L-Eₚ with Ep being a solid support or a protected end group E, wherein E and L have the meanings as defined herein;
**G6)** optionally repeating steps G2) -G5) in the following order G4)→G5)→G2)→G3)→ G4)→G5) n-1 times to obtain intermediate compound of formula **5g,** 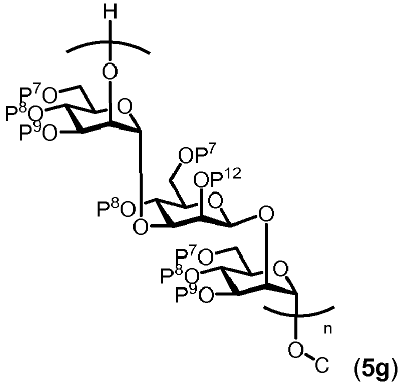 wherein P⁷- P⁹ and P¹² represent protecting groups and C represents -L-Eₚ with Ep being a solid support or a protected end group E, wherein n, E and L have the meanings as defined herein;
**G7)** performing removal of all protecting groups to obtain a saccharide of general formula (**I**).

Another method of the synthesis of a saccharide of general formula (**I**) comprises the following steps:
**H1)** providing a monosaccharide **10** 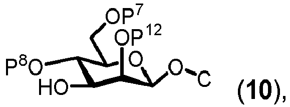 wherein P⁷, P⁸ and P¹² represent protecting groups and C represents -L-Eₚ with Ep being a solid support or a protected end group E, wherein E and L have the meanings as defined herein;
**H2)** treating monosaccharide **10** with building block **8** in presence of an activating agent 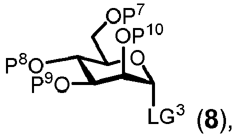 wherein P⁷- P¹⁰ represent protecting groups and LG³ represents a leaving group;
**H3)** performing removal of protecting group P¹⁰;
**H4)** repeating steps H2) and H3) to obtain intermediate compound **4h**; 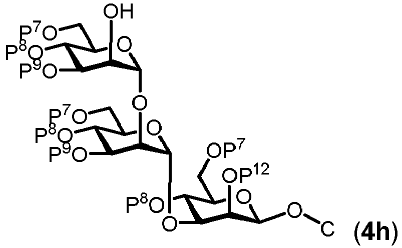 wherein P⁷- P⁹ and P¹² represent protecting groups and C represents -L-Eₚ with Ep being a solid support or a protected end group E, wherein E and L have the meanings as defined herein;
**H5)** optionally treating compound **4h** with building block **9** in presence of an activating agent 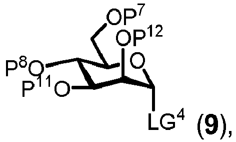 wherein P⁷, P⁸, P¹¹ and P¹² represent protecting groups and LG⁴ represents a leaving group, performing removal of protecting group P¹¹, and performing steps H2) -H4) n-1 times to obtain intermediate compound of formula **5h** 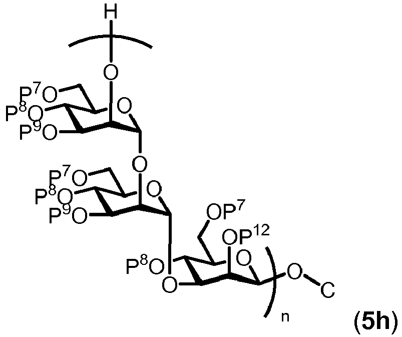 wherein P⁷- P⁹ and P¹² represent protecting groups and C represents -L-Eₚ with Ep being a solid support or a protected end group E, wherein n, E and L have the meanings as defined herein;
**H6)** performing removal of all protecting groups to obtain a saccharide of general formula (**I**).

A further method of the synthesis of a saccharide of general formula (**I**) comprises the following steps:
**11)** providing a monosaccharide **1** 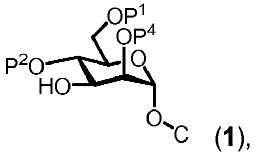 wherein P¹, P² and P⁴ represent protecting groups and C represents -L-Eₚ with Ep being a solid support or a protected end group E, wherein E and L have the meanings as defined herein;
**12)** treating monosaccharide **1** with building block **2** in presence of an activating agent 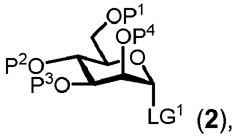 wherein P¹ - P⁴ represent protecting groups and LG¹ represents a leaving group;
**13)** performing removal of protecting group P³;
**14)** treating the product of step 13) with a building block 3 in presence of an activating agent 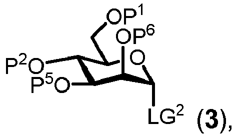 wherein P¹, P², P⁵ and P⁶ represent protecting groups and LG² represents a leaving group;
**15)** performing removal of protecting group P⁶ to obtain intermediate compound **4i**; 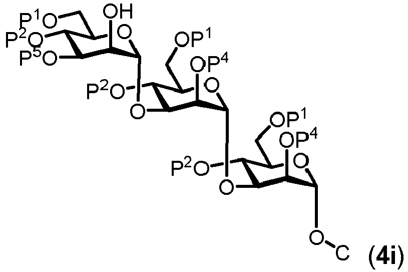 wherein P¹, P², P⁴ and P⁵ represent protecting groups and C represents -L-Eₚ with Eₚ being a solid support or a protected end group E, wherein E and L have the meanings as defined herein;
**16)** optionally repeating steps 12) - 15) in the following order I2)→I3)→I2)→I3)→I4)→ 15) n-1 times to obtain intermediate compound of formula **5i** 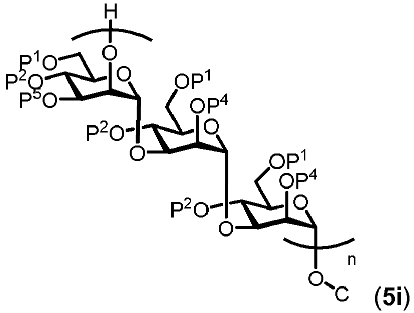 wherein P¹, P², P⁴ and P⁵ represent protecting groups and C represents -L-Eₚ with Ep being a solid support or a protected end group E, wherein n, E and L have the meanings as defined herein;
**17)** performing removal of all protecting groups to obtain a saccharide of general formula (**I**).

A further method of the synthesis of a saccharide of general formula (**I**) comprises the following steps:
**J1)** providing a monosaccharide **1** 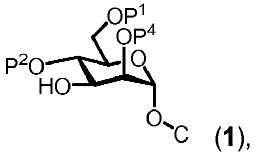 wherein P¹, P² and P⁴ represent protecting groups and C represents -L-Eₚ with Ep being a solid support or a protected end group E, wherein E and L have the meanings as defined herein;
**J2)** treating monosaccharide **1** with a building block **3** in presence of an activating agent 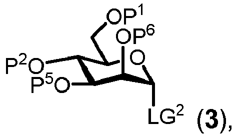 wherein P¹, P², P⁵ and P⁶ represent protecting groups and LG² represents a leaving group;
**J3)** performing removal of protecting group P⁶
**J4)** treating the product of step J3) with building block **2** in presence of an activating agent 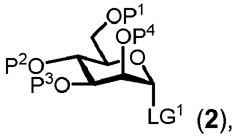 wherein P¹ - P⁴ represent protecting groups and LG¹ represents a leaving group;
**J5)** performing removal of protecting group P³ to obtain intermediate compound **4j;** 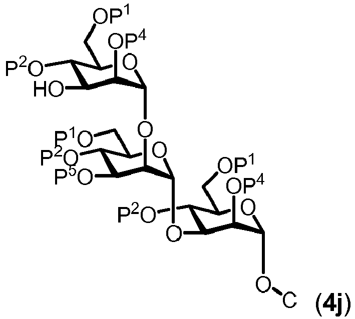 wherein P¹, P², P⁴ and P⁵ represent protecting groups and C represents -L-Eₚ with Eₚ being a solid support or a protected end group E, wherein E and L have the meanings as defined herein;
**J6)** optionally repeating steps J2) - J5) in the following order J4)→J5)→J2)→J3)→J4) →J5) n-1 times to obtain intermediate compound of formula **5j** 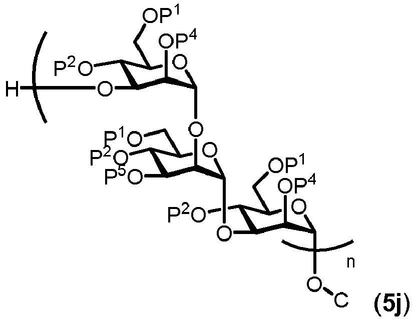 wherein P¹, P², P⁴ and P⁵ represent protecting groups and C represents -L-Eₚ with Ep being a solid support or a protected end group E, wherein n, E and L have the meanings as defined herein;
**J7)** performing removal of all protecting groups to obtain a saccharide of general formula (**I**).

A further method of the synthesis of a saccharide of general formula (**I**) comprises the following steps:
**K1)** providing a monosaccharide **6** 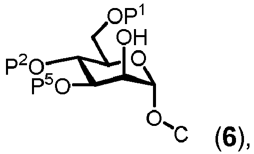 wherein P¹, P² and P⁵ represent protecting groups and C represents -L-Eₚ with Eₚ being a solid support or a protected end group E, wherein E and L have the meanings as defined herein;
**K2)** treating monosaccharide **6** with building block **2** in presence of an activating agent 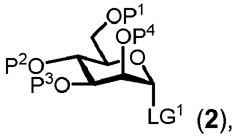 wherein P¹ - P⁴ represent protecting groups and LG¹ represents a leaving group;
**K3)** performing removal of protecting group P³;
**K4)** repeating steps K2) and K3) to obtain intermediate compound **4k;** 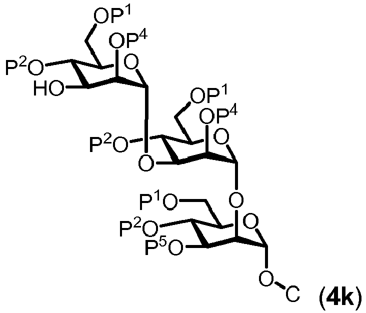 wherein P¹, P², P⁴ and P⁵ represent protecting groups and C represents -L-Eₚ with Eₚ being a solid support or a protected end group E, wherein E and L have the meanings as defined herein;
**K5)** optionally treating compound 4k with a building block **3** in presence of an activating agent 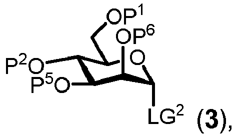 wherein P¹, P², P⁵ and P⁶ represent protecting groups and LG² represents a leaving group, performing removal of protecting group P⁶ and repeating steps K2) - K4) n-1 times to obtain intermediate compound of formula **5k** 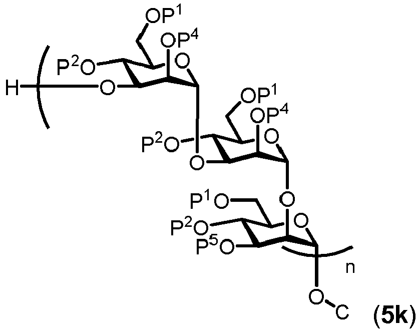 wherein P¹, P², P⁴ and P⁵ represent protecting groups and C represents -L-Eₚ with Ep being a solid support or a protected end group E, wherein n, E and L have the meanings as defined herein;
**K7)** performing removal of all protecting groups to obtain a saccharide of general formula (**I**).

Eₚ represents a solid support or a protected end group. E represents -NH₂, -N₃, -CN, -O-NH₂, -CH=CH₂, -C=CH, -Br, -CI, -I, -CO₂R', -CONHNH₂, -SH, or -SAc; and the corresponding protected end group Eₚ represents -N(P¹³)(P¹⁴), -N₃, -CN, -O-N(P¹³)(P¹⁴), -CH=CH₂, -C=CH, -Br, -CI, -I, -CO₂R', -CONHN(P¹³)(P¹⁴), -SPₛ, or -SAc

P¹, P², P³, P⁴, P⁵, P⁶, P⁷, P⁸, P⁹, P¹⁰, P¹¹, P¹², P¹³ and P¹⁴ represent protecting groups. The term "protecting group" as used herein refers to commonly used groups in organic synthesis, preferably used for protection hydroxyl groups, and thiols.
More preferably, P¹, P², P³, P⁴, P⁵, P⁶, P⁷, P⁸, P⁹, P¹⁰, P¹¹ and P¹² are suitable protecting groups for hydroxyl groups, more preferably different suitable protecting groups for hydroxyl groups capable of being removed subsequently one after another by a suitable sequence of deprotection reactions. Preferred protecting groups for hydroxyl groups are acetyl, phenyl, benzyl, isopropylidene, benzylidene, benzoyl, *p*-methoxybenzyl, *p*-methoxybenzylidene, *p*-methoxyphenyl, *p*-bromobenzyledene, *p*-nitrophenyl, allyl, acetyl, isopropyl, *p*-bromobenzyl, dimethoxytrityl, trityl, 2-naphthylmethyl, pivaloyl, triisopropylsilyl, *tert*-butyldimethylsilyl, *tert*-butyldiphenylsilyl, *tert*-butylmethoxphenylsilyl, triethylsilyl, trimethylsilyl, 2-trimethylsilylethoxymethyl, 9-fluorenylmethoxycarbonyl, benzyloxymethyl, methyloxymethyl, *tert*-butyloxymethyl, methoxyethyloxymethyl, levulinoyl.

The protecting groups can be differentiated in permanent protecting groups and temporary protecting groups. Permanent protecting groups are protecting groups that are stable during the entire synthesis and that can be efficiently removed at the late stage of the synthesis. In this case, permanent protecting groups include P¹, P², P⁴ P⁵, P⁷, P⁸, P⁹, P¹², P¹³ and P¹⁴. P¹, P², P⁴ P⁵, P⁷, P⁸, P⁹ and P¹² are masking the hydroxyl groups during the entire synthesis, while protecting groups P¹³ and P¹⁴ are masking the terminal amino group present in the end group Eₚ. Preferably protecting groups P¹, P², P⁵, P⁷, P⁸ and P⁹ are benzyl groups, protecting group P⁴ is a benzoyl group, protecting group P¹² is a benzyl group, protecting group P¹³ is a benzyl group and protecting group P¹⁴ is a benzyloxycarbonyl group (Cbz).

The temporary protecting groups are generally orthogonal protecting groups that can be selectively removed at different levels of the synthesis to free hydroxyl groups for subsequent introduction of different substituents, including monosaccharides, other protecting groups or other residues present on the molecule. In this case, temporary protecting groups include P³, P⁶, P¹⁰ and P¹¹.

The ingenious choice of protecting groups allows expedient access to a library of saccharides of general formulae (**I**), (**II**), (**II-a**), (**II-b**), (**II-c**), (**II-d**), (**II-e**), (**II-f**), (**II-g**), (**II-h**), (**II-i**), (**II-j**), (**II-k**), (**III**), (**III-a**), (**III-b**), (**III-c**), (**III-d**), (**III-e**), (**III-f**), (**III-g**), (**III-h**), (**III-i**), (**III-j**) or (**III-k**) functionalized with a terminal group for subsequent conjugation to an immunogenic carrier or a solid support. Moreover, the choice of leaving groups affects the stereochemical outcome of the glycosylation reactions in steps A2), A4), B2), B5), C2), C4), D2), D5), E2), E5), F2), F4), H2), H5), J2), J4), 12), 14), K2) and K5). From the prior art it is apparent for a skilled person to choose the protecting group and reaction conditions in order to obtain the desired mannose saccharides of general formulae (**I**), (**II**), (**II-a**), (**II-b**), (**II-c**), (**II-d**), (**II-e**), (**II-f**), (**II-g**), (**II-h**), (**II-i**), (**II-j**), (**II-k**), (**III**), (**III-a**), (**III-b**), (**III-c**), (**III-d**), (**III-e**), (**III-f**), (**III-g**), (**III-h**), (**III-i**), (**III-j**) or (**III-k**) (see J. Chem. Soc., Perkin Trans. 1, 2000, 1471-1491 and Eur. J. Org. Chem. 2009, 870-888).

Temporary protecting groups P³, P⁶, P¹⁰ and P¹¹ are preferably selected from, but are not restricted to: allyl, p-methoxybenzyl, 2-naphthylmethyl, tri-isopropylsilyl, *tert-*butyldimethylsilyl, *tert*-butylmethoxyphenylsilyl, triethylsilyl, trimethylsilyl, 2-trimethylsilylethoxymethyl, 9-fluorenylmethoxycarbonyl and levulinoyl. Preferably, protecting groups P³, P⁶, P¹⁰ and P¹¹ can be selectively removed in presence of protecting groups P¹, P², P⁴ P⁵, P⁷, P⁸, P⁹, P¹², P¹³ and P¹⁴. Preferably, P³, P⁶, P¹⁰ and P¹¹ are 9-fluorenylmethoxycarbonyl or levulinoyl, and more preferably P³ and P¹¹ are the same and P⁶ and P¹⁰ are the same. In a preferred embodiment, protecting groups P⁶ and P¹⁰ represent 9-fluorenylmethoxycarbonyl and protecting groups P³ and P¹¹ represent -fluorenylmethoxycarbonyl or levulinoyl.

Building blocks **2, 3, 8** and **9** are glycosylating agents. As used herein, the term glycosylating agent refers to a monosaccharide functionalized at the anomeric position with a leaving group that upon activation with a suitable activating agent provide an oxocarbenium intermediate able to react with a nucleophile, such as a hydroxyl group. Hence, glycosylating agents **2, 3, 8** and **9** are functionalized at the anomeric position with leaving groups LG¹, LG², LG³ and LG⁴. Examples of leaving groups suitable for the present synthesis are well known to the person skilled in carbohydrate chemistry and include halides, thioethers, imidates, acetate, and phosphate.

Preferably, leaving groups LG¹, LG², LG³ and LG⁴ are selected from the group of leaving groups consisting of:

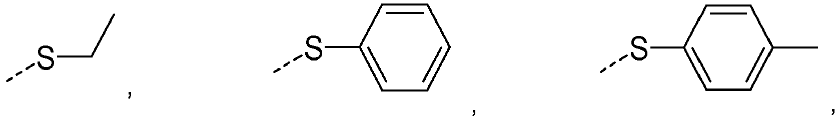

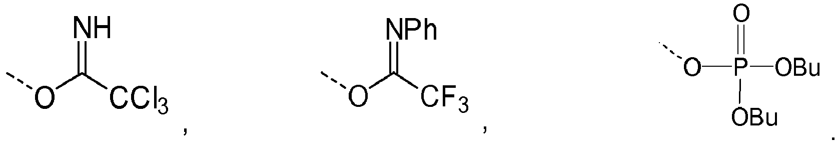

As mentioned, the provision of an oxocarbenium intermediate relies on the activation of the leaving group installed at the anomeric position of the glycosylating agent with an appropriate or suitable activating agent. It is common knowledge for the skilled person that suitable activating agents for phosphate (i.e. phosphate activating agents) and imidate (i.e. imidate activating agents) are Lewis acids, such as silyl triflate or silver triflate, while suitable activating agents for thioether i.e. thioether activating agents include, but are not restricted to: NIS/TfOH, NIS/TMSOTf, NIS/BF₃^{.}Et₂O, NIS/AgOTf, DMTST/Tf₂O, IDPC, BSP/Tf₂O, Ph₂SO/Tf₂O. Examples of silyl triflate include, but are not restricted to trimethylsilyl trifluoromethanesulfonate, *tert*-butyl dimethyl trifluoromethanesulfonate, triiospropyl trifluoromethanesulfonate.

Preferably, LG¹, LG², LG³ and LG⁴ are thioethers and even more preferred is when LG¹, LG², LG³ and LG⁴ are selected from the group consisting of:

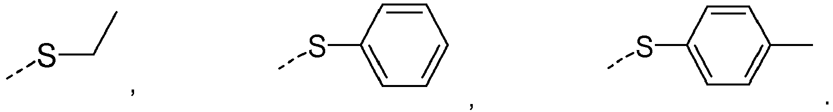

It is preferred that the coupling reaction between saccharides in the steps **A2), A4), B2), B5), C2), C4), D2), D5), E2), E5), F2), F4), G2), G4), H2), H5), J2), J4), 12), 14), K2)** and **K5)** is performed by activation with NIS/TfOH or TMSOTf, in a mixture of apolar solvent and polar aprotic solvent at a temperature of between - 10 °C and 10 °C. Even more preferred is that said reaction is performed in a mixture of apolar solvent and polar aprotic solvent, by treatment with NIS/TfOH at a temperature of about 0 °C

Preferred polar aprotic solvents are tetrahydrofuran, diethyl ether and dioxane. Preferred apolar solvents are toluene, halogenated solvents such as chloroform and methylene chloride. Preferred mixtures of apolar and polar aprotic solvent are: methylene chloride / tetrahydrofuran, methylene chloride / diethyl ether, toluene / diethyl ether, toluene/ tetrahydrofuran.

The removal of protecting groups P¹, P², P⁴ P⁵, P⁷, P⁸, P⁹, P¹², P¹³ and P¹⁴ performed at steps **A8), B8), C9), D9), E8), F7), F4), G7), H8), 17), J7)** and K7) involves:
- first cleavage of the base-labile protecting groups by treatment with a base in presence of hydrogen peroxide in a mixture of solvents. Preferably, the base is NaOMe or LiOH; and
- second cleavage of the protecting groups sensitive to hydrogenation by subjecting the compound to hydrogen in presence of a palladium catalyst in a mixture of solvents.

A further aspect according to the present invention refers to an intermediate compound for preparing a saccharide of the general formulae **(I), (II), (II-a), (II-b), (II-c), (II-d), (II-e), (II-f), (II-g), (II-h), (II-i), (II-j), (II-k), (III), (III-a), (III-b), (III-c), (III-d), (III-e), (III-f), (III-g), (III-h), (III-i), (III-j)** or **(III-k)** , wherein the intermediate compound has any one of general formulae **(I2a), (I2b), (I2c), (I2d), (I2e), (I2f), (I2g), (I2h), (I3a), (I3b), (I3c), (I3d), (I3e), (I3f), (I3g), (I3h), (I3i), (I3j), (I3k), (I3l), (I3m), (I3n), (I4a), (I4b), (I4c), (I4d), (I4e), (I4f), (I4g), (I4h), (I4i), (I4j), (I5a), (I5b), (I5c), (I5d), (I5e), (I5f), (I5g), (I5h), (I5i)** or **(15j):** 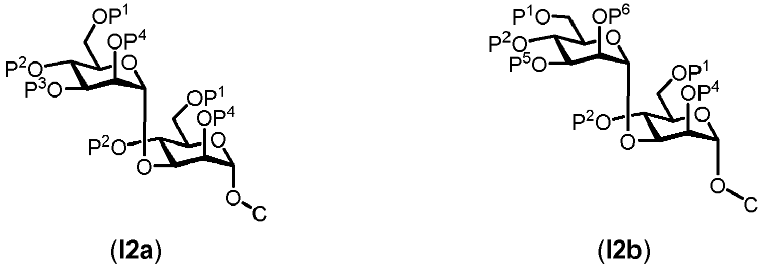 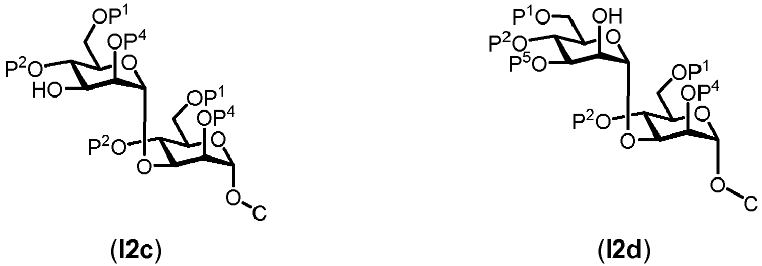 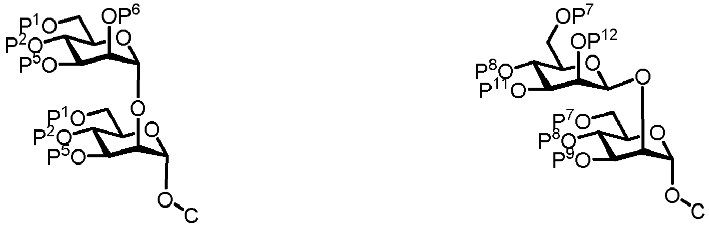 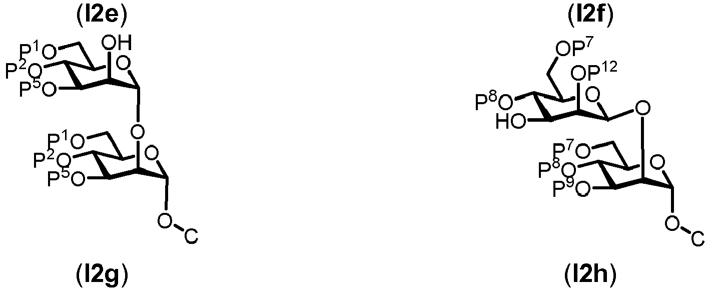 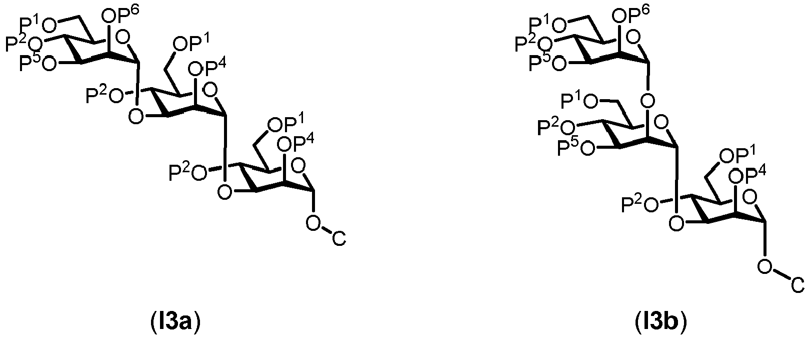 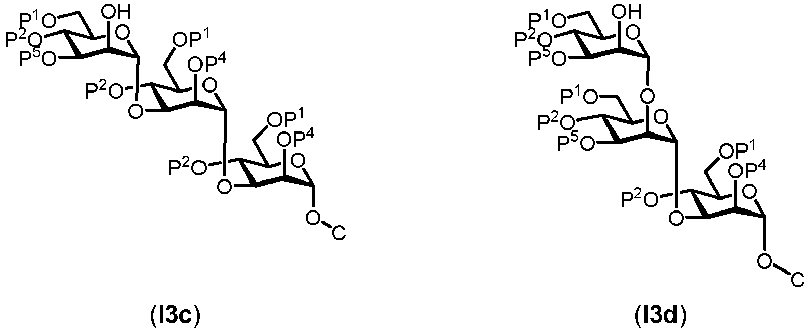 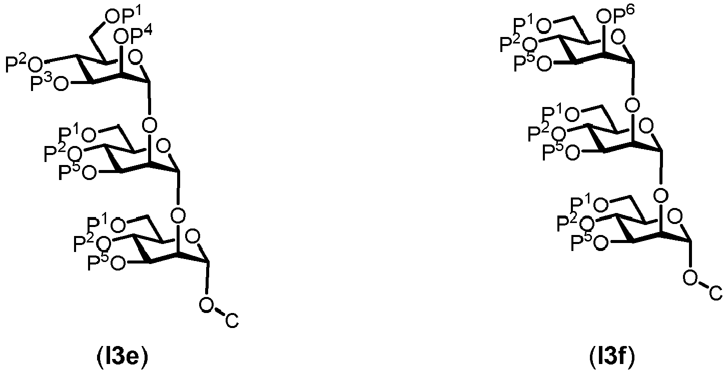 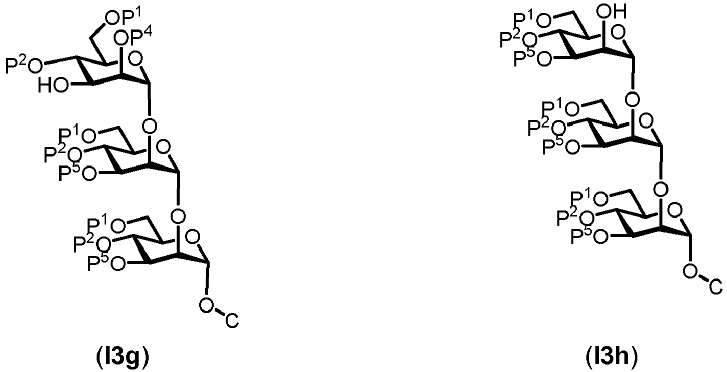 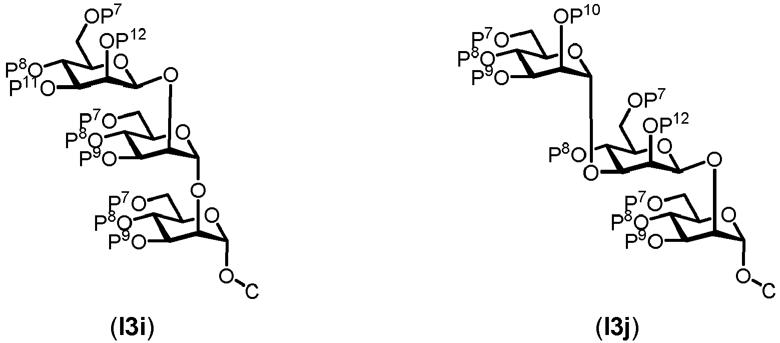 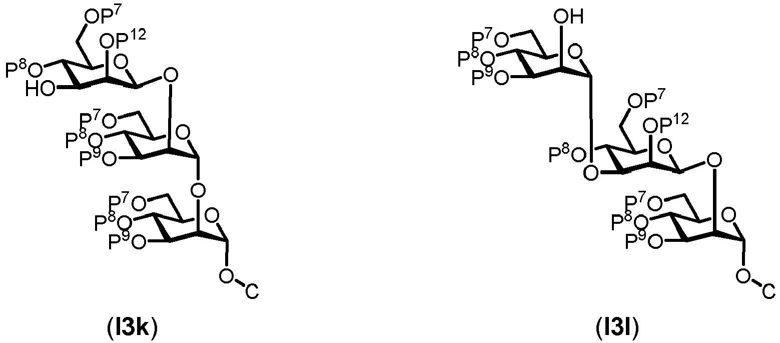 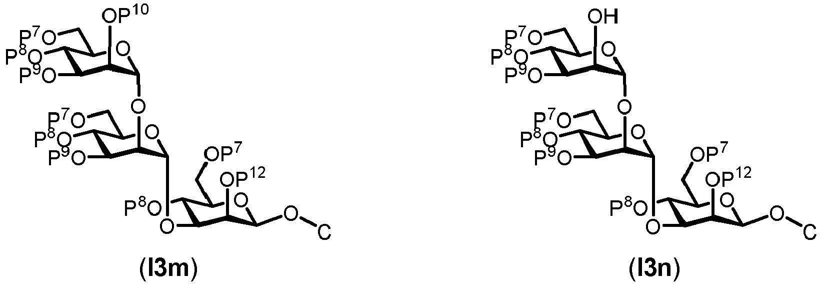 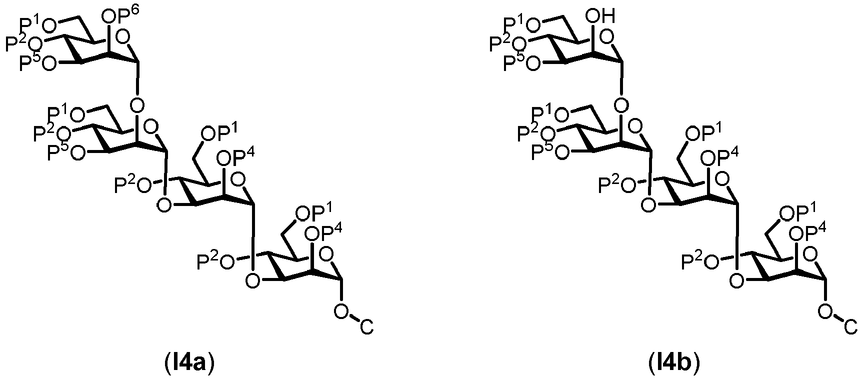 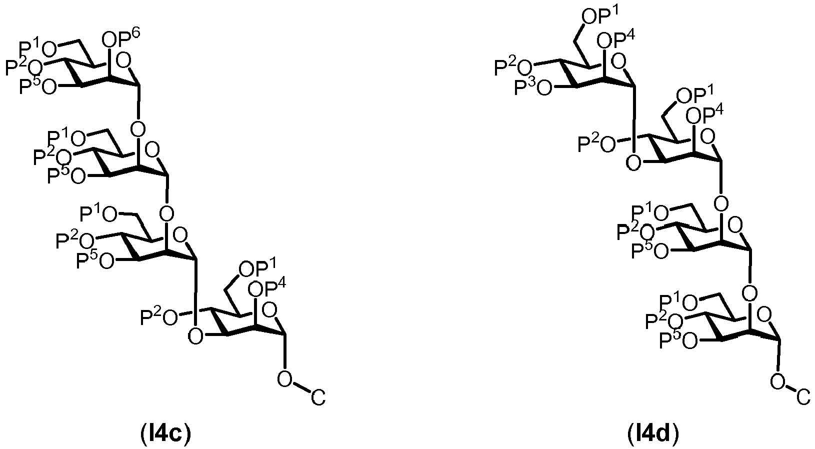 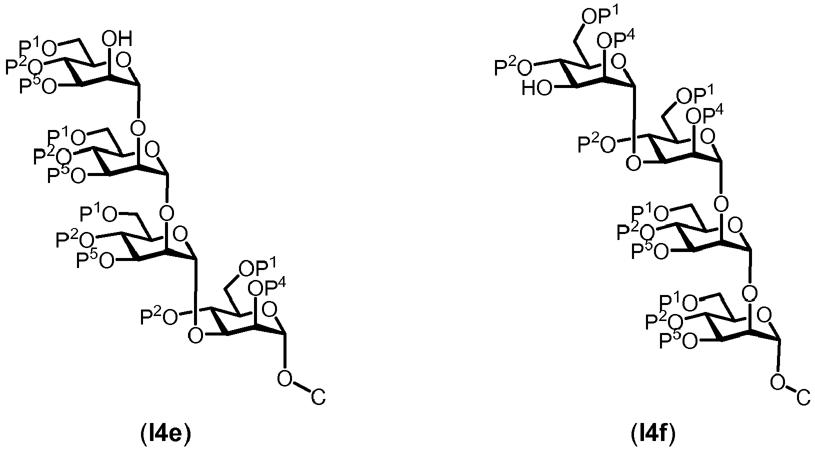 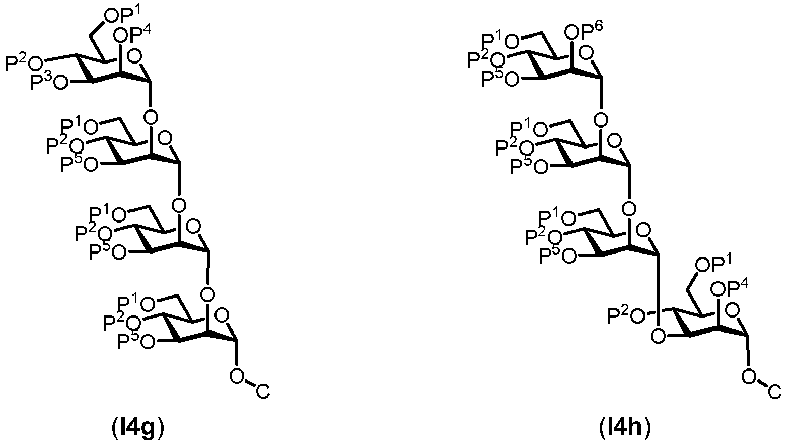 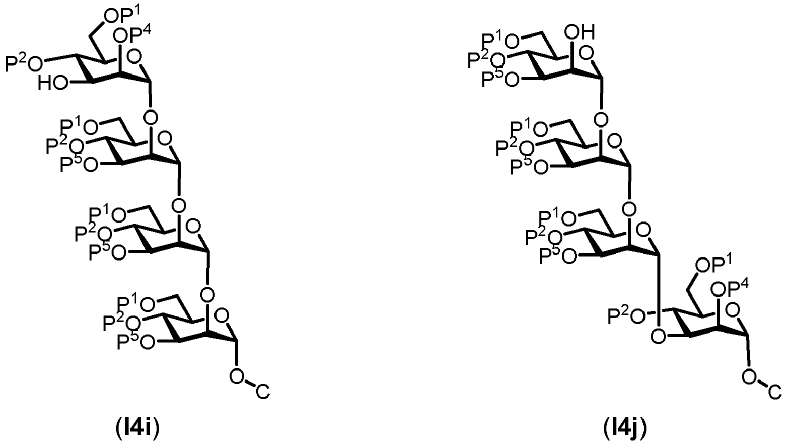 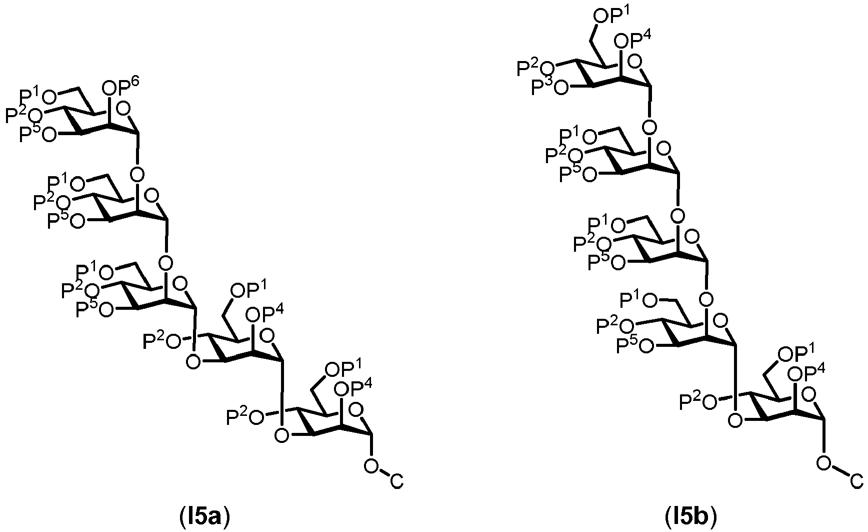 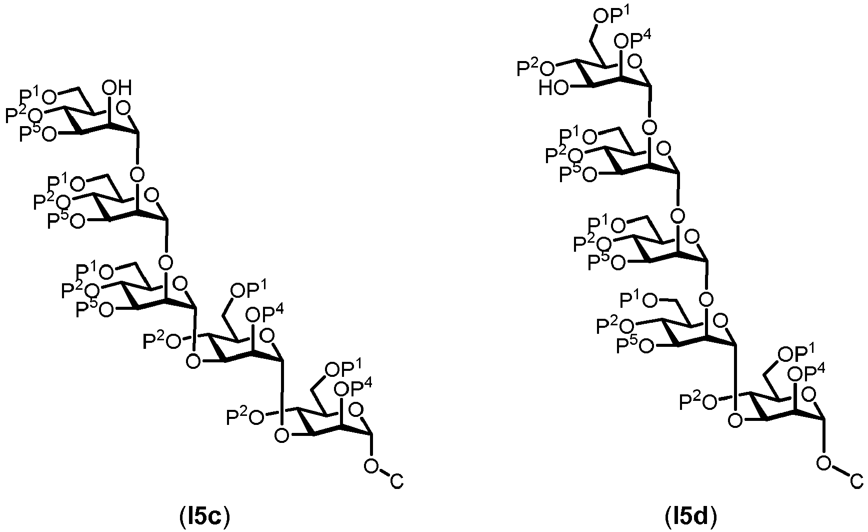 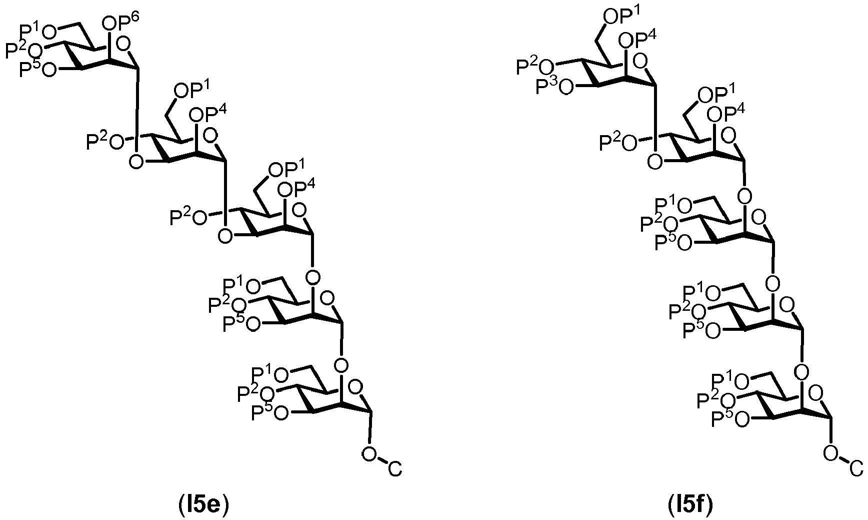 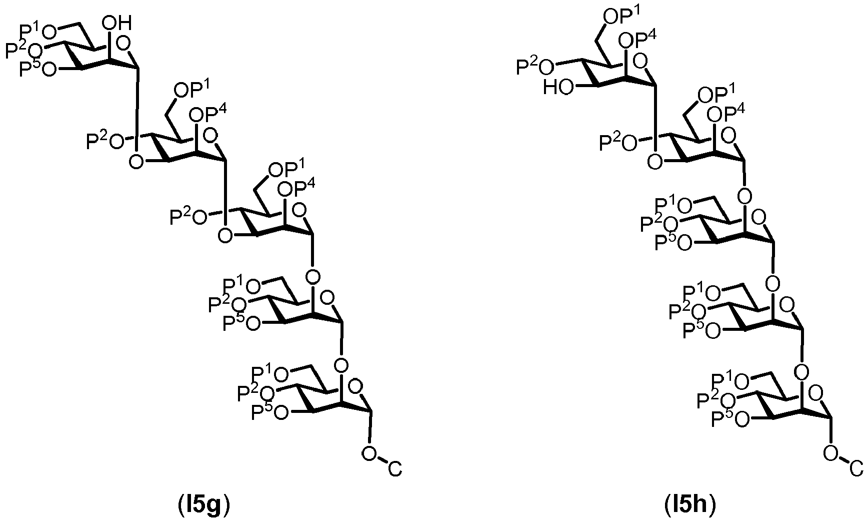 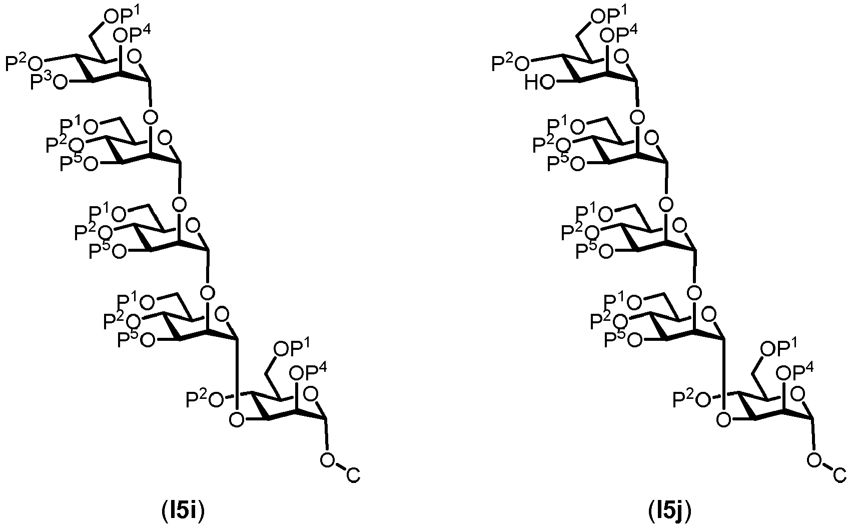 wherein C represents -L-Eₚ with Ep being a solid support or a protected end group E, P¹, P², P³, P⁴, P⁵, P⁶, P⁷, P⁸, P⁹, P¹⁰, P¹¹ and P¹² represent protecting groups, and E and L have the same meanings as defined above.

In formulae **(12a),** (**12b**), (**12c**), **(I2d), (I2e), (I2f), (I2g), (I2h), (13a), (13b), (13c), (13d), (I3e), (I3f), (I3g), (I3h), (I3i),** (**I3j**), **(I3k), (I3l), (I3m), (I3n), (I4a), (I4b), (I4c), (I4d), (I4e), (I4f), (I4g), (I4h), (I4i), (I4j), (15a), (15b), (15c), (I5d), (I5e), (I5f), (I5g), (I5h), (I5i)** or (**I5j**), preferably the linker-**L**- represents **-L^{a}-, -L^{a}-L^{e}- -L^{a}-L^{b}-L^{e}-,** or **-L^{a}-L^{d}-L^{e}-;** -**L**^{a}- represents -(CH₂)ₒ-, -(CH₂-CH₂-O)ₒ-C₂H₄-, or -(CH₂-CH₂-O)ₒ-CH₂; -**L^{b}**- represents -O-;
-**L^{d}**- represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂-;
-**L**^{e}- represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂-; and
**o**, **q**, **p1** and **p2** are independently of each other an integer selected from 1, 2, 3, 4, 5, and 6

An especially preferred intermediate is an intermediate of formula **(I2a), (I2b), (I2c), (I2d), (I2e), (I2f), (I2g), (I2h), (I3a), (I3b), (I3c), (I3d), (I3e), (I3f), (I3g), (I3h), (I3i),** (**I**3j), **(I3k), (I3l), (I3m), (I3n), (I4a), (I4b), (I4c), (I4d), (I4e), (I4f), (I4g), (I4h), (I4i), (I4j), (I5a), (I5b), (I5c), (I5d), (I5e), (I5f), (I5g), (I5h), (I5i)** or (**I**5j), wherein -L-represents -(CH₂)ₒ- and o is an integer selected from 2, 5 and 6.

P¹, P², P³, P⁴, P⁵, P⁶, P⁷, P⁸, P⁹, P¹⁰, P¹¹ and P¹² are suitable protecting groups for hydroxyl groups, more preferably different suitable protecting groups for hydroxyl groups capable of being removed subsequently one after another by a suitable sequence of deprotection reactions. Preferred protecting groups for hydroxyl groups are acetyl, phenyl, benzyl, isopropylidene, benzylidene, benzoyl, p-methoxybenzyl, *p*-methoxybenzylidene, p-methoxyphenyl, p-bromobenzyledene, p-nitrophenyl, allyl, acetyl, isopropyl, p-bromobenzyl, dimethoxytrityl, trityl, 2-naphthylmethyl, pivaloyl, triisopropylsilyl, *tert*-butyldimethylsilyl, *tert*-butyldiphenylsilyl, *tert*-butylmethoxphenylsilyl, triethylsilyl, trimethylsilyl, 2-trimethylsilylethoxymethyl, 9-fluorenylmethoxycarbonyl, benzyloxymethyl, methyloxymethyl, *tert*-butyloxymethyl, methoxyethyloxymethyl, levulinoyl.

Thus, intermediates **(I2a), (I2b), (I2c), (I2d), (I2e), (I2f), (I2g), (I2h), (I3a), (I3b), (I3c), (I3d), (I3e), (I3f), (I3g), (I3h), (I3i),** (**I**3j), **(I3k), (I3l), (I3m), (I3n), (I4a), (I4b), (I4c), (I4d), (I4e), (I4f), (I4g), (I4h), (I4i), (I4j), (I5a), (I5b), (I5c), (I5d), (I5e), (I5f), (I5g), (I5h), (I5i)** or (**I**5j), are especially preferred when protecting groups P¹, P², P⁵, P⁷, P⁸ and P⁹ are benzyl groups, protecting group, P³, P⁶, P¹⁰ and P¹¹ are 9-fluorenylmethoxycarbonyl or levulinoyl groups, P⁴ and P¹² are benzoyl groups, protecting group P¹³ is a benzyl group and protecting group P¹⁴ is a benzyloxycarbonyl group (Cbz).

### Glycoconjugates

Another aspect of the present invention refers to a conjugate comprising a saccharide of general formula (I) covalently bound or covalently linked to an immunogenic carrier through the terminal group E of the -O-L-E group. In other words, another aspect of the present invention is directed to a saccharide of any of the general formulae (I), (II), **(II-a)-(II-k), (III), (III-a)-(III-j)** or **(III-k)** conjugated with an immunogenic carrier through the terminal group E of the -O-L-E group. A conjugate comprising a synthetic saccharide of the general formula (I), (II), **(II-a)-(II-k), (III), (III-a)-(III-j)** or **(III-k),** covalently bound or covalently linked to an immunogenic carrier through the terminal group E of the -O-L-E group is also defined as a conjugate obtained by reacting a saccharide of any of the general formulae **(I), (II), (II-a)-(II-k), (III), (III-a)-(III-j)** or **(III-k)** with an immunogenic carrier. Surprisingly, said conjugate proved to be efficient as a vaccine for immunization against diseases associated with *Klebsiella pneumoniae* serotype 03, O3b and/or 05 bacteria.

Saccharides are known by the person skilled in the art as generally TI-2 (T cell independent-2) antigens and poor immunogens. TI-2 antigens are antigens, which are recognized only by mature B cells through the cross linking of surface exposed immunoglobulin receptors. Without T cell help, no immunological memory is generated and neither isotype switching from IgM to other IgG subclasses, nor B cells affinity maturation occurs. Moreover, saccharides are known poor immunogens in humans due to the structural homology to human glycolipids and glycoproteins. Due to their poor immunogenic properties, saccharides manifest poor ability to produce both antibody production by B cells, as well as the formation of memory cells, features which are essential for the production of potent vaccines.

Therefore, to produce a potent saccharide-based vaccine, the saccharides of general formulae **(I), (II), (II-a)-(II-k), (III), (III-a)-(III-j)** or **(III-k)** are conjugated to an immunogenic carrier to provide conjugates, which present increased immunogenicity in comparison with the saccharide. Hence, under the scope of the present application is covered also a conjugate comprising a saccharide fragment

**T*-[(-Uₓ₊₄-Uₓ₊₃-Uₓ₊₂-Uₓ₊₁-Uₓ)ₘ-(Vₓ₊₂-Vₓ₊₁-Vₓ)₁₋ₘ]ₙ-T-O-**

wherein m, n, x, Uₓ₊₁, Uₓ₊₂, Uₓ₊₃, Uₓ₊₄, Vₓ, Vₓ₊₁, Vₓ₊₂, T and T* have the meanings defined herein, covalently linked through the O atom to an immunogenic carrier.

Said conjugate comprises at least one synthetic saccharide of the general formula **(I)** and an immunogenic carrier to which the at least one saccharide **(I)** is covalently bound.

Surprisingly it was found that immunization with a conjugate comprising a saccharide of general formula (I) covalently linked to an immunogenic carrier results in the production of high titers of antibodies specific to the carbohydrate part of the saccharide of general formula (I). Said antibodies are cross-reacting with the natural *Klebsiella pneumoniae* serotype 03, O3b and/or 05 lipopolysaccharides and present opsonophagocytosis and bactericidal activity, thus conferring protection against *Klebsiella pneumoniae* serotype 03, O3b and/or 05 bacteria.

In this context the term "immunogenic carrier" is defined as a structure, which is conjugated to the saccharide to form a conjugate that presents an increased immunogenicity in comparison with the saccharide *per se.* Thus, the conjugation of the saccharides of the general formulae **(I), (II), (II-a)-(II-k), (III), (III-a)-(III-j)** or **(III-k)** to the immunogenic carrier has as effect the stimulation of the immune response against the saccharide of general formula **(I)** without inducing an immune response against said immunogenic carrier.

Preferred immunogenic carriers are carrier proteins or glycosphingolipids with immunomodulatory properties. For the person skilled in the art, a carrier protein is a protein selected from the group comprising or consisting of: a diphtheria toxoid, a mutated diphtheria toxoid, a modified diphtheria toxoid, a mutated and modified diphtheria toxoid, a tetanus toxoid, a modified tetanus toxoid, a mutated tetanus toxoid, non-lipidated cell-surface liporotein (protein D) of non-typeable *Haemophilus influenzae,* outer membrane protein (OMP) complex of *Neisseria meningitidis,* bovine serum albumin (BSA), keyhole limpet hemocyanine (KLH) or cholera toxoid (CT). The term "toxoid" as used herein refers to a bacterial toxin (usually an exotoxin), whose toxicity has been inactivated or suppressed either by chemical (formalin) or heat treatment, while other properties, typically immunogenicity, are maintained. A mutated toxoid as used herein is a recombinant bacterial toxin, which has been amended to be less toxic or even non-toxic by amending the wild-type amino acid sequence. Such a mutation could be a substitution of one or more amino acids. Such a mutated toxoid presents on its surface a functionality that can react with the functional group Y of the interconnecting molecule to provide a modified toxoid. Said functionality is known to the person skilled in the art and includes, but is not restricted to the primary amino functionality of a lysine residue that can react with activated esters, an isocyanate group or an aldehyde in presence of a reducing agent, to the carboxylate functionality of a glutamate or aspartate residue that can be activated by carbodiimides or to the thiol functionality of a cysteine residue.

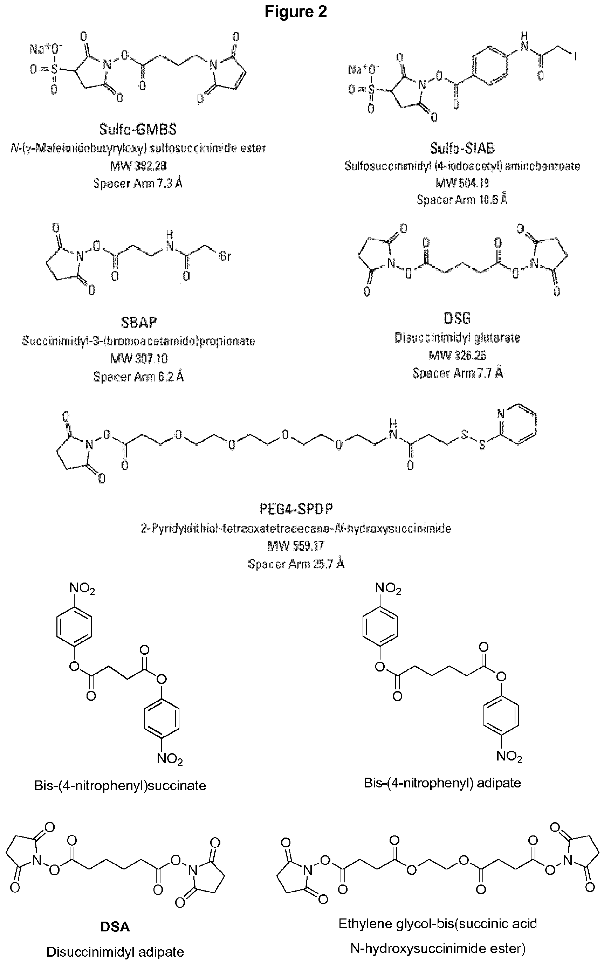

Activated esters include N-(γ-maleimidobutyryloxy) sulfosuccinimide ester (sulfo-GMBS), succinimidyl (4-iodoacetyl) aminobenzoate (sulfo-SIAB), succinimidyl-3-(bromoacetamido)propionate (SBAP), disuccinimidyl glutarate (DSG), disuccinimidyl adipate (DSA), 2-pyridyldithiol-tetraoxatetradecane-*N*-hydroxysuccinimide (PEG-4-SPDP) (see **Figure 2**).

The cysteine residue on the carrier protein can be converted to the corresponding dehydroalanine that can be further reacted with a suitable interconnecting molecule to provide modified carrier protein having on their surface the functional group X of the interconnecting molecule.

It is especially preferred that the saccharides of general formula I are conjugated to the non-toxic mutated diphtheria toxin CRM₁₉₇ presenting as a functionality a primary amine functionality of a lysine residue.

CRM₁₉₇ like wild-type diphtheria toxin is a single polypeptide chain of 535 amino acids (58 kD) consisting of two subunits linked by disulfide bridges having a single amino acid substitution of glutamic acid for glycine. It is utilized as a carrier protein in a number of approved conjugate vaccines for diseases such as Prevnar.

Thus, in a preferred embodiment of the present invention the carrier protein presents on its surface primary amino functionalities of lysine residues that are able to react with the functional group Y of the interconnecting molecule to provide modified carrier protein having on their surface said functional group X of the interconnecting molecule, which is able to react with the terminal amino group of the linker of the compounds of general formula (**I**).

Said functional group X of the interconnecting molecules is selected of the group comprising or consisting of maleimide; α-iodoacetyl; α-bromoacetyl; and *N*-hydroxysuccinimide ester (NHS), aldehyde, imidoester, carboxylic acid, alkyl sulfonate, sulfonyl chloride, epoxide, anhydride, carbonate (see **Figure 3**).

Preferably, the saccharide of general formula **I** is conjugated to the non-toxic mutated diphtheria toxin CRM₁₉₇, which is modified by maleimide. In yet another preferred embodiment, the saccharide of general formula **I** is conjugated to the non-toxic mutated diphtheria toxin CRM₁₉₇, which is modified by α-bromoacetamide. In the most preferred embodiment, the saccharide of general formula **I** is conjugated to the non-toxic mutated diphtheria toxin CRM₁₉₇, which is modified by *N*-hydroxysuccinimide adipate.

Preferred is a conjugate of general formula (**IV**)

**[T*-((-Uₓ₊₄-Uₓ₊₃-Uₓ₊₂-Uₓ₊₁-Uₓ)ₘ-(Vₓ₊₂-Vₓ₊₁-Vₓ)₁₋ₘ)ₙ-T-O-L-E₁-W]_{c}-CP** **(IV)**

wherein
c is comprised between 2 and 18;
-**E₁**- represents a covalent bond, -NH-, -O-NH-, -O-, -S-, -CO-, -CH=CH-, -CONH-, -CO-NHNH-, 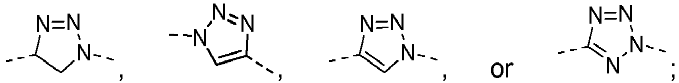
-W- is selected from: 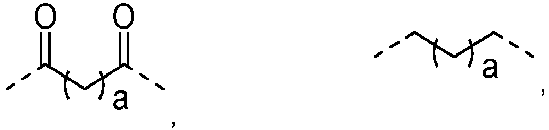 and 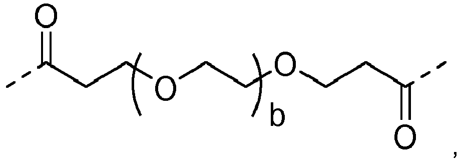
   a represents an integer selected from 1, 2, 3, 4, 5, 6, 7, 8, 9 and 10,
   b represents an integer selected from 1, 2, 3 and 4,
   CP is a carrier protein; and
   m, n, x, L, Uₓ₊₁, Uₓ₊₂, Uₓ₊₃, Uₓ₊₄, Vₓ, Vₓ₊₁, Vₓ₊₂, T and T* have the meanings as defined herein.

Preferably **E₁** is a covalent bond, -NH-, -CH=CH-, -CONH-, 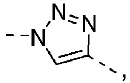 or 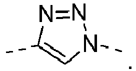 Preferably CP is CRM₁₉₇. Thus, in one embodiment of the present invention the conjugate is of general formula (**IV**), wherein CP is CRM₁₉₇ and c, -E₁-, W, m, n, x, L, Uₓ₊₁, Uₓ₊₂, Uₓ₊₃, Uₓ₊₄, Vₓ, Vₓ₊₁, Vₓ₊₂, T and T* have the meanings as defined herein.

Preferably, in general formula (**IV**) the linker -L- is selected from: -L^{a}-, -L^{a}-L^{e}-, -L^{a}-L^{b}-L^{e}-, and -L^{a}-L^{d}-L^{e}-;
-L^{a}- is selected from: -(CH₂)ₒ-, -(CH₂-CH₂-O)ₒ-C₂H₄-, -(CH₂-CH₂-O)ₒ₋CH₂; -L^{b}- represents -O-;
-L^{d}- is selected from: -(CH₂)_{q}-, -(CF₂)_{q}-, -(CH₂-CH₂-O)_{q}-C₂H₄-, and -(CH₂-CH₂-O)_{q}-CH₂-;
-L^{e}- is selected from: -(CH₂)ₚ₁-, -(CF₂)ₚ₁-, -C₂H₄-(O-CH₂-CH₂)ₚ₁-, -CH₂-(O-CH₂-CH₂)ₚ₁- and -(CH₂)ₚ₁-O-(CH₂)ₚ₂-;
and o, q, p1 and p2 are independently of each other an integer selected from 1, 2, 3, 4, 5, and 6.

Also a conjugate of general formula **(IV),** wherein -W- represents 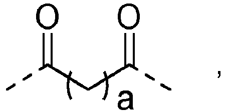 and a is an integer selected from 2, 3, 4, 5 and 6 is preferred.

A conjugate of general formula **(IV),** wherein
the linker-L- is selected from: -L^{a}-, -L^{a}-L^{e}-, -L^{a}-L^{b}-L^{e}-, and -L^{a}-L^{d}-L^{e}-; -L^{a}- is selected from: -(CH₂)ₒ-, -(CH₂-CH₂-O)ₒ-C₂H₄-, -(CH₂-CH₂-O)_{O}-CH₂; -L^{b}- represents -O-;
-L^{d}- is selected from: -(CH₂)_{q}-, -(CF₂)_{q}-, -(CH₂-CH₂-O)_{q}-C₂H₄-, and -(CH₂-CH₂-O)_{q}-CH₂-;
-L^{e}- is selected from: -(CH₂)ₚ₁-, -(CF₂)ₚ₁-, -C₂H₄-(O-CH₂-CH₂)ₚ₁-, -CH₂-(O-CH₂-CH₂)ₚ₁- and -(CH₂)ₚ₁-O-(CH₂)ₚ₂-;
o, q, p1 and p2 are independently of each other an integer selected from 1, 2, 3, 4, 5, and 6;
-W- represents 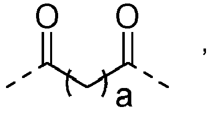 and a is an integer selected from 2, 3, 4, 5 and 6 is especially preferred.

Even more preferred is a conjugate of general formula **(IV),** wherein
x represents 1,
V*- represents H-,
the linker-L- is selected from: -L^{a}-, -L^{a}-L^{e}-, -L^{a}-L^{b}-L^{e}-, and -L^{a}-L^{d}-L^{e}-; -L^{a}- is selected from: -(CH₂)ₒ-, -(CH₂-CH₂-O)ₒ-C₂H₄-, -(CH₂-CH₂-O)ₒ-CH₂; -L^{b}- represents -O-;
-L^{d}- is selected from: -(CH₂)_{q}-, -(CF₂)_{q}-, -(CH₂-CH₂-O)_{q}-C₂H₄-, and -(CH₂-CH₂-O)_{q}-CH₂-;
-L^{e}- is selected from: -(CH₂)ₚ₁-, -(CF₂)ₚ₁-, -C₂H₄-(O-CH₂-CH₂)ₚ₁-, -CH₂-(O-CH₂-CH₂)ₚ₁- and -(CH₂)ₚ₁-O-(CH₂)ₚ₂-;
o, q, p1 and p2 are independently of each other an integer selected from 1, 2, 3, 4, 5, and 6;
-W- represents 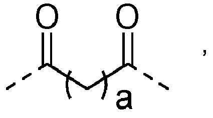 and a is an integer selected from 2, 3, 4, 5 and 6. Particularly preferred is a conjugate of general formula **(IV),** wherein the linker -L-represents -(CH₂)ₒ-,
o is an integer selected from 2, 3, 4, 5 and 6;
-W- represents 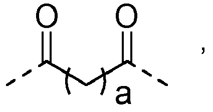 and a is an integer selected from 2, 3, 4, 5 and 6.

Also preferred is a conjugate of general formula (**IV**), wherein x represents 1,
V*- represents H-,
the linker -L- represents -(CH₂)ₒ- ,
o is an integer selected from 2, 3, 4, 5 and 6;
-W- represents 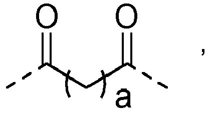 and a is an integer selected from 2, 3, 4, 5 and 6.

Preferably c is comprised between 2 and 18, more preferably between 5 and 15, even more preferably between 8 and 12. It is also preferred that n represents 1.

Preferred is also a conjugate of general formula (**V**)

**[H-((-Uₓ₊₄-Uₓ₊₃-Uₓ₊₂-Uₓ₊₁-Uₓ)ₘ-(Vₓ₊₂-Vₓ₊₁-Vₓ)₁₋ₘ)ₙ-O-L-E₁-W]_{c}-CRM₁₉₇** (**V**)

wherein
c is comprised between 2 and 18;
-**E₁**- represents a covalent bond, -NH-, -O-NH-, -O-, -S-, -CO-, -CH=CH-, -CONH-, -CO-NHNH-, 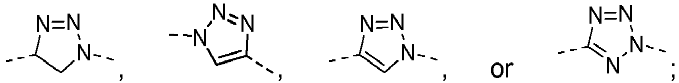
-W- is selected from: 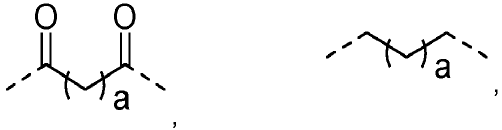 and 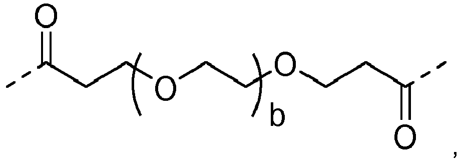
   a represents an integer selected from 1, 2, 3, 4, 5, 6, 7, 8, 9 and 10,
   b represents an integer selected from 1, 2, 3 and 4; and
   m, n, x, L, Uₓ₊₁, Uₓ₊₂, Uₓ₊₃, Uₓ₊₄, Vₓ, Vₓ₊₁ and Vₓ₊₂ have the meanings as defined herein.

In another embodiment, said immunogenic carrier is preferably a glycosphingolipid with immunomodulatory properties, and more preferably (2S,3*S*,4*R*)-1-(α-D-galactopyranosyl)-2-hexacosanoylaminooctadecane-3,4-diol. The term glycosphingolipid with immunomodulatory properties, as used herein, refers to a suitable glycosphingolipid capable of stimulating the immune system's response to a target antigen, but which does not in itself confer immunity as defined above.

Glycosphingolipids as used herein are compounds containing a carbohydrate moiety α-linked to a sphingolipid. Preferably, the carbohydrate moiety is a hexopyranose and most preferably is α-D-galactopyranose. For the person skilled in the art, sphingolipids are a class of lipids containing a C18 amino alcohol connected *via* an amide bond to a fatty acid. The C18 amino alcohol is preferably mono-, di- or polysubstituted with hydroxyl groups. Especially preferred, the C18 amino alcohol is phytosphingosine. The fatty acid is preferably a monocarboxylic acid having a saturated alkyl chain of a number of carbons ranging from 16 to 28 and more preferably from 18 to 26. Glycosphingolipids with immunomodulatory properties include, but they are not restricted to (2*S*,3*S*,4*R*)-1-(α-D-galactopyranosyl)-2-hexacosanoylaminooctadecane-3,4-diol, which can stimulate natural killer (NK) activity and cytokine production by natural killer T (NKT) cells and exhibits potent antitumor activity *in vivo (*Proc. Natl Acad. Sci. USA, 1998, 95, 5690).

The conjugates of the saccharides of general formula **I** with a glycosphingolipid with immunomodulatory properties have the advantage of being heat stable. To be suitable for conjugation, on the glycosphingolipid with immunomodulatory properties a functionality is introduced. Said functionality is prone to react directly with the terminal amino group of the linker of the saccharides of general formula **I** to provide conjugates of the saccharides of general formula **I**, or with the functional group Y of the interconnecting molecule to provide the modified glycosphingolipid with immunomodulatory properties.

Preferably, said functionality is introduced at the C6 of the carbohydrate moiety of the glycosphingolipid with immunomodulatory properties. Thus, the glycosphingolipid with immunomodulatory properties is functionalized with a functionality, which is prone of reacting with the terminal amino group of the saccharides or with the functional group Y of the interconnecting molecule. A functionality prone to react with an amino group includes, but it is not restricted to activated ester, isocyanate group, aldehyde, epoxide, imidoester, carboxylic acid, alkyl sulfonate and sulfonyl chloride. A functionality prone to react with the functional group Y of the interconnecting molecule so that to provide the modified glycosphingolipid with immunomodulatory properties presenting the functional group X of the interconnecting molecule includes, but it is not restricted to amine, alcohol, thiol, activated ester, isocyanate group, aldehyde, epoxide, vinyl, imidoester, carboxylic acid, alkyl sulfonate, sulfonyl chloride, vinyl group, alkynyl group and azido group.

Preferably, the functionality introduced at the C6 of the carbohydrate moiety of the glycosphingolipid with immunomodulatory properties is selected from the group comprising or containing an amine, a thiol, an alcohol, a carboxylic acid, a vinyl, maleimide, α-iodoacetyl, α-bromoacetyl, *N*-hydroxysuccinimide ester (NHS), 2-pyridyldithiols.

Said functional group X of the interconnecting molecules is selected of the group comprising or consisting of maleimide, α-iodoacetyl, α-bromoacetyl, *N*-hydroxysuccinimide ester (NHS), aldehyde, carboxylic acid, epoxyde, alkyl sulfonate, sulfonyl chloride, anhydride, carbonate.

As used herein, the term "interconnecting molecule" refers to a bifunctional molecule containing functional group X and functional group Y, wherein functional group X is capable of reacting with the terminal amino group on the linker -L- and the functional group Y is capable of reacting with a functionality present on the immunogenic carrier or on the solid support.

Vaccines containing at least one conjugate of the present invention cause fewer side effects and/or non-protective immune responses in comparison to vaccines containing isolated (and not synthesized) mixtures of saccharides obtained by non-selective cleavage of the capsular polysaccharide of *Klebsiella pneumoniae* or conjugates thereof. Moreover the inventive vaccines can be easier manufactured in accordance with the GMP regulations than the vaccines containing isolated mixtures of non-selectively cleaved capsular polysaccharides and are easier characterized, which makes stability and purity control easier as well as detection of kind and amount of impurities.

More preferred is a conjugate of any one of the formulae **(V-1)** - (**V-11**): 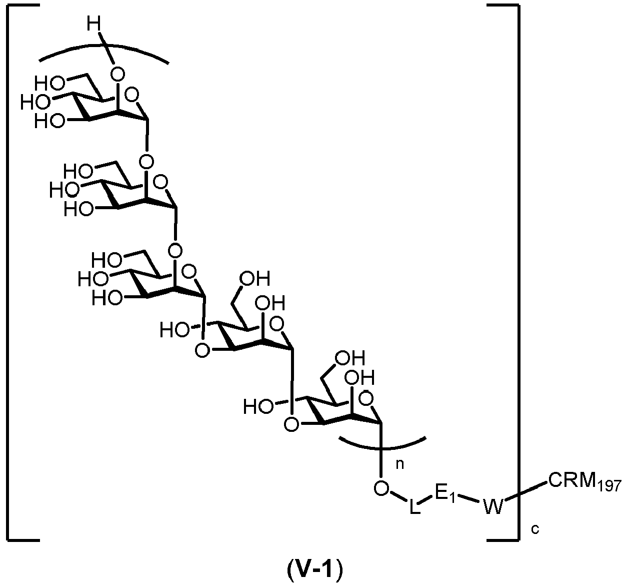 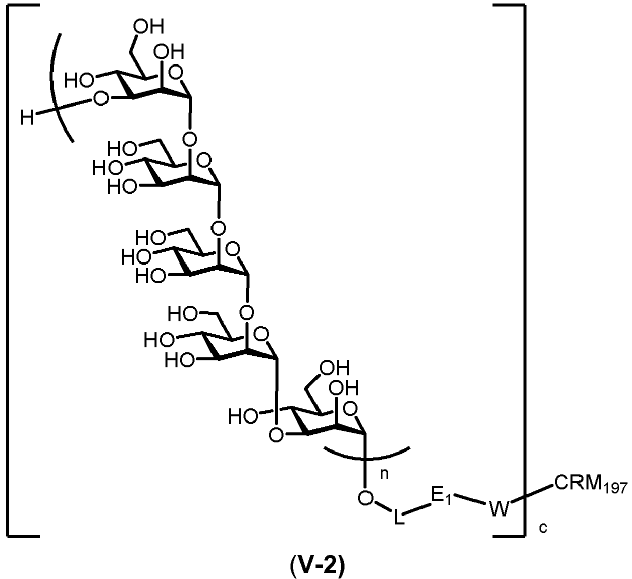 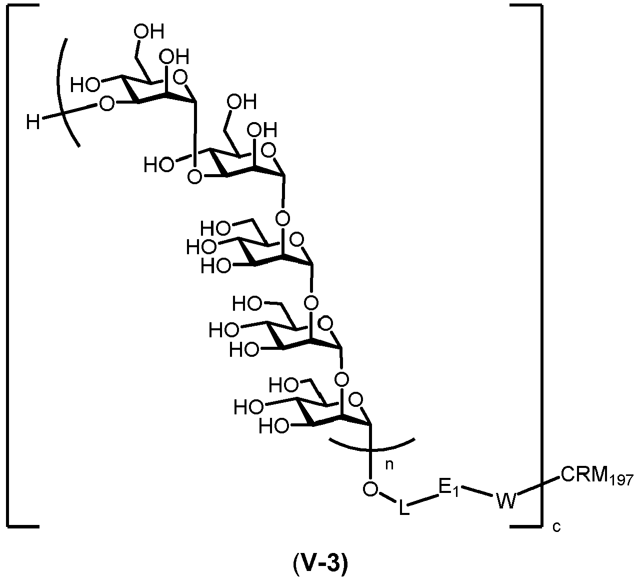 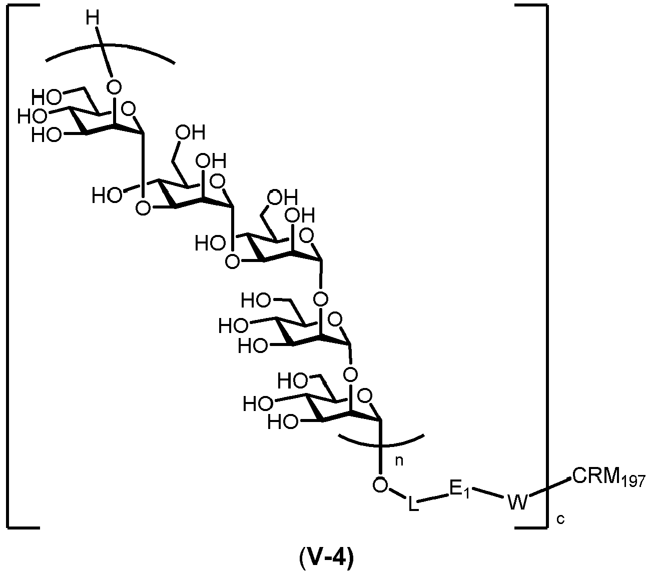 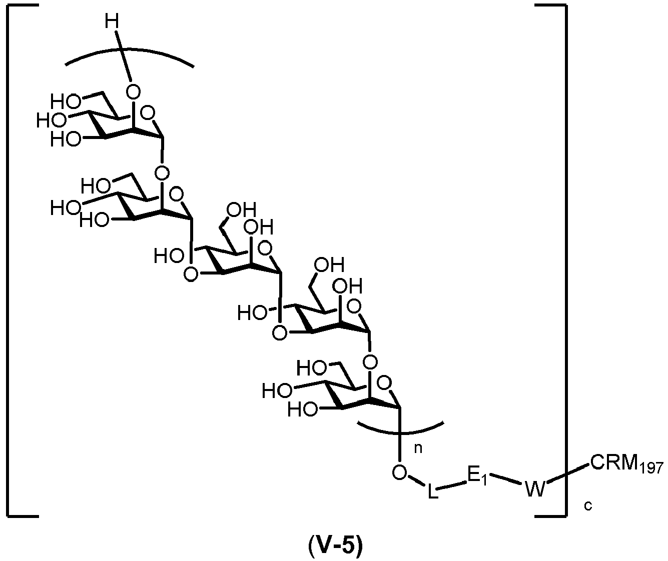 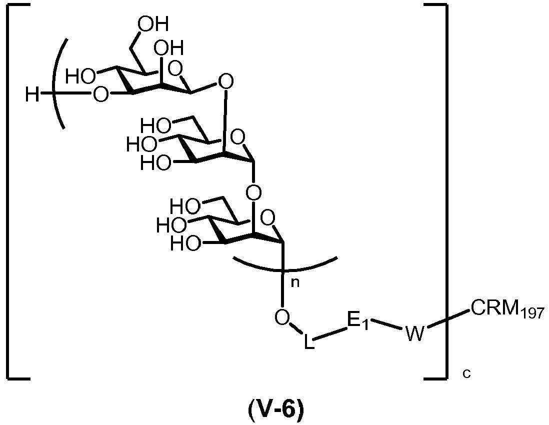 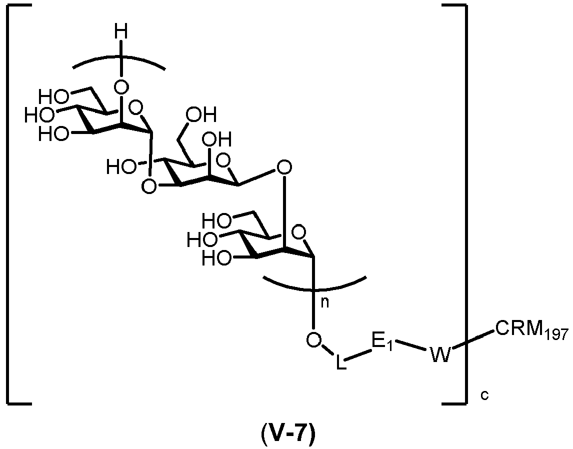 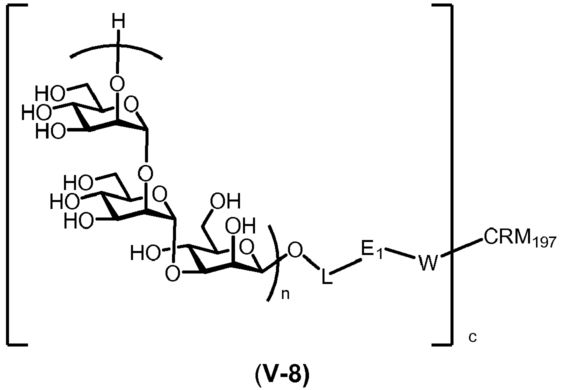 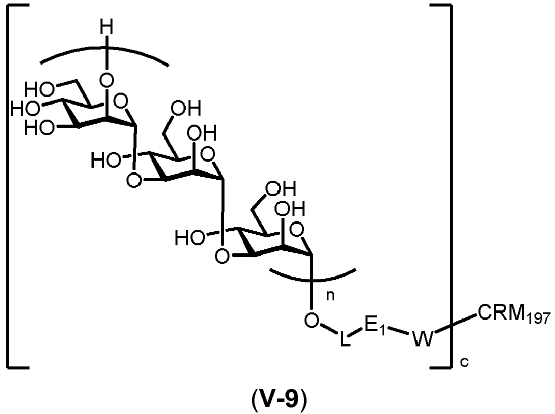 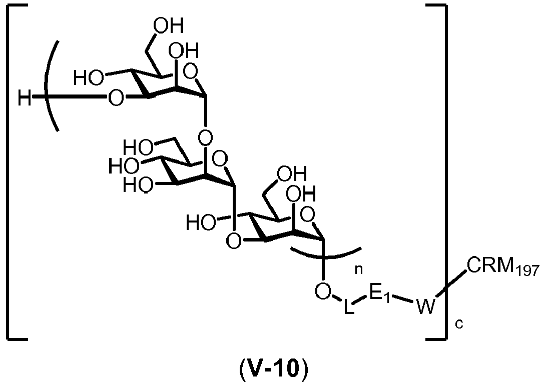 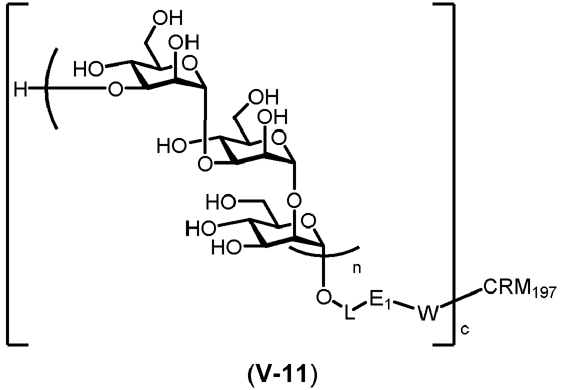 wherein L, E₁, W, c, and n have the same meanings as defined above.

More preferred is a conjugate of any one of the formulae **(IV), (V)** and **(V-1)** - **(V-11),** wherein n is an integer from 2 to 10.
More preferred the conjugate of any one of the formulae **(IV), (V)** and **(V-1)** - **(V-11),** wherein **c** is selected from 4 to 10.

Preferably -W- represents 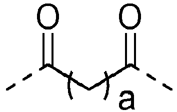 and a is an integer selected from 2, 3, 4, 5 and 6.

Thus, a conjugate of general formula **(IV), (V)** and **(V-1)** - **(V-11),** wherein -W-represents 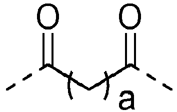 and a is an integer selected from 2, 3, 4, 5 and 6 is especially preferred.

Preferably, the linker**-L-** represents **-L^{a}-, -L^{a}-L^{e}-, -L^{a}-L^{b}-L^{e}-,** or **-L^{a}-L^{d}-L^{e}-; -L^{a}-** represents -(CH₂)ₒ-, -(CH₂-CH₂-O)ₒ-C₂H₄-, or -(CH₂-CH₂-O)ₒ-CH₂; **-L^{b}-** represents -O-;
**-L^{d}-** represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂-;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂-; and
**o, q, p1** and **p2** are independently of each other an integer selected from 1, 2, 3, 4, 5, and 6

In the most preferred embodiment, E₁ is a covalent bond, -NH-, -CH=CH-, -CONH-,

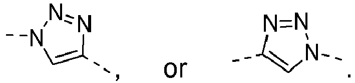

It was found that a conjugate comprising a saccharide of any one of general formulae **(I), (II), (II-a)-(II-k), (III), (III-a)-(III-j)** or **(III-k),** and particularly a conjugate of any one of general formulae (**IV**), **(V)** and **(V-1)** - (**V-11**), elicits a protective immune response in a human and/or animal host, and therefore is useful for prevention and/or treatment of diseases associated with *Klebsiella pneumoniae* serotype 03, O3b and/or 05 bacteria. Thus, the conjugates comprising the saccharides of general formula (**I**) conjugated to an immunogenic carrier are useful for prevention and/or treatment of diseases associated with *Klebsiella pneumoniae* bacteria containing in their lipopolysaccharide one of the following saccharide fragments:
-2)-α-D-Man-(1, 2)-α-D-Man-(1, 2)-α-D-Man-(1, 3)-α-D-Man-(1, 3)-α-D-Man-(1-;
-3)-α-D-Man-(1, 2)-α-D-Man-(1, 2)-α-D-Man-(1, 2)-α-D-Man-(1, 3)-α-D-Man-(1-;
-3)-α-D-Man-(1, 3)-α-D-Man-(1, 2)-α-D-Man-(1, 2)-α-D-Man-(1, 2)-α-D-Man-(1-;
-2)-α-D-Man-(1, 3)-α-D-Man-(1, 3)-α-D-Man-(1, 2)-α-D-Man-(1, 2)-α-D-Man-(1-;
-2)-α-D-Man-(1, 2)-α-D-Man-(1, 3)-α-D-Man-(1, 3)-α-D-Man-(1, 2)-α-D-Man-(1-;
-3)-β-D-Man-(1, 2)-α-D-Man-(1, 2)-α-D-Man-(1-;
-2)-α-D-Man-(1, 3)-β-D-Man-(1, 2)-α-D-Man-(1-;
-2)-α-D-Man-(1, 2)-α-D-Man-(1, 3)-β-D-Man-(1-;
-2)-α-D-Man-(1, 3)-α-D-Man-(1, 3)-α-D-Man-(1-;
-3)-α-D-Man-(1, 2)-α-D-Man-(1, 3)-α-D-Man-(1-;
-3)-α-D-Man-(1, 3)-α-D-Man-(1, 2)-α-D-Man-(1-.

Preferably, the bacterium containing in the lipopolysaccharide one of the above mentioned saccharide fragments is *Klebsiella pneumoniae* serotype 03, O3b and/or 05.
In a preferred embodiment, the conjugates comprising the saccharides of general formula **I** conjugated to an immunogenic carrier are useful for prevention and/or treatment of diseases associated with bacteria, and particularly with diseases associated with bacteria containing in their O-polysaccharide one of the following saccharide fragments: -2)-α-D-Man-(1, 2)-α-D-Man-(1, 2)-α-D-Man-(1, 3)-α-D-Man-(1, 3)-α-D-Man-(1-;-3)-α-D-Man-(1, 2)-α-D-Man-(1, 2)-α-D-Man-(1, 2)-α-D-Man-(1, 3)-α-D-Man-(1-; -3)-α-D-Man-(1, 3)-α-D-Man-(1, 2)-α-D-Man-(1, 2)-α-D-Man-(1, 2)-α-D-Man-(1-; -2)-α-D-Man-(1, 3)-α-D-Man-(1, 3)-α-D-Man-(1, 2)-α-D-Man-(1, 2)-α-D-Man-(1-; -2)-α-D-Man-(1, 2)-α-D-Man-(1, 3)-α-D-Man-(1, 3)-α-D-Man-(1, 2)-α-D-Man-(1-; -3)-β-D-Man-(1, 2)-α-D-Man-(1, 2)-α-D-Man-(1-; -2)-α-D-Man-(1, 3)-β-D-Man-(1, 2)-α-D-Man-(1-; -2)-α-D-Man-(1, 2)-α-D-Man-(1, 3)-β-D-Man-(1-; -2)-α-D-Man-(1, 3)-α-D-Man-(1, 3)-α-D-Man-(1-; -3)-α-D-Man-(1, 2)-α-D-Man-(1, 3)-α-D-Man-(1-; -3)-α-D-Man-(1, 3)-α-D-Man-(1, 2)-α-D-Man-(1-, and preferably with *Klebsiella pneumoniae* serotype 03, O3b and/or 05, wherein said diseases include pneumonia, bronchitis, meningitis, urinary tract infection, wound infection, osteomyelitis, bacteremia, septicemia and ankylosing spondylitis.

### Pharmaceutical compositions

Another aspect of the present invention is directed to a pharmaceutical composition or a vaccine comprising at least one conjugate that comprises a saccharide of general formula (**I**) conjugated to an immunogenic carrier and/or at least one saccharide of general formula (**I**) together with at least one pharmaceutically acceptable adjuvant and/or excipient. Said pharmaceutical composition can be used for raising a protective immune response in a human and/or animal host. Ideally, the pharmaceutical composition is suitable for use in humans.

In another aspect of the present invention, said pharmaceutical composition or vaccine further comprises at least one of capsular polysaccharides, O-polysaccharides and/or capsular polysaccharides, O-polysaccharide fragments and/or protein conjugates thereof of *Klebsiella pneumoniae* bacteria selected from the group comprising or consisting of *Klebsiella pneumoniae* serotypes 01, 02, O2a, O2ac, 04, 07, 08, 012 and carbapenem-resistant *Klebsiella pneumoniae* ST258.

The term "adjuvant" as used herein refers to an immunological adjuvant i.e. a material used in a vaccine composition that modifies or augments the effects of said vaccine by enhancing the immune response to a given antigen contained in the vaccine without being antigenically related to it. For the persons skilled in the art, classically recognized examples of immunological adjuvants include, but are not restricted to oil emulsions (e.g. Freund's adjuvant), saponins, aluminum or calcium salts (e.g. alum), non-ionic block polymer surfactants, and many others.

Pharmaceutical compositions are preferably in aqueous form, particularly at the point of administration, but they can also be presented in non-aqueous liquid forms or in dried forms e.g. as gelatin capsules, or as lyophilisates, etc.

Pharmaceutical compositions may include one or more preservatives, such as thiomersal or 2-phenoxyethanol. Mercury-free compositions are preferred, and preservative-free vaccines can be prepared.

Pharmaceutical compositions may include a physiological salt, such as a sodium salt e.g. to control tonicity. Sodium chloride (NaCl) is typical and may be present at between 1 and 20 mg/ml. Other salts that may be present include potassium chloride, potassium dihydrogen phosphate, disodium phosphate dehydrate, magnesium chloride, calcium chloride, etc..

Pharmaceutical compositions can have an osmolality of between 200 mOsm/kg and 400 mOsm/kg.

Pharmaceutical compositions may include compounds (with or without an insoluble metal salt) in plain water (e.g. w.f.i.), but will usually include one or more buffers. Typical buffers include: a phosphate buffer; a Tris buffer; a borate buffer; a succinate buffer; a histidine buffer (particularly with an aluminium hydroxide adjuvant); or a citrate buffer. Buffer salts will typically be included in the 5-20 mM range.

Pharmaceutical compositions typically have a pH between 5.0 and 9.5 e.g. between 6.0 and 8.0.

Pharmaceutical compositions are preferably sterile and gluten free.

Pharmaceutical compositions are suitable for administration to animal (and, in particular, human) patients, and thus include both human and veterinary uses. They may be used in a method of raising an immune response in a patient, comprising the step of administering the composition to the patient.

The pharmaceutical compositions of the present invention may be administered before a subject is exposed to a *Klebsiella pneumoniae* serotype 03, O3b and/or 05 and/or after a subject is exposed to a *Klebsiella pneumoniae* serotype 03, O3b and/or 05.

In another aspect of the present invention, the present invention is directed to the use of at least one conjugate that comprises at least one saccharide of general formula (**I**) conjugated to an immunogenic carrier and/or at least one saccharide of general formula (**I**) for the manufacture of said pharmaceutical composition or said vaccine for prevention and/or treatment of diseases associated with *Klebsiella pneumoniae* serotype O3, O3b or O5 bacteria, particularly, diseases associated with *Klebsiella pneumoniae* serotype O3, O3b or O5 bacteria is selected from the group comprising or consisting of pneumonia, bronchitis, meningitis, urinary tract infection, wound infection, osteomyelitis, bacteremia, septicemia and ankylosing spondylitis.

Preferred, the present invention refers to the use of at least one saccharide of any one of general formulae **(I), (II), (II-a)-(II-k), (III), (III-a)-(III-j)** or **(III-k)** and/or at least one of the conjugates comprising at least one saccharide of any one of general formulae **(I), (II), (II-a)-(II-k), (III), (III-a)-(III-j)** or **(III-k)** for the manufacture of said pharmaceutical composition or said vaccine.
More preferred, the present invention refers to the use of at least one of the saccharides **I'a-1 - I'a-11, I'a-1 - I'b-11, I'b-1 - I'c-11, I'c-1 - I'c-11, I'd-1 - I'd-11, I'e-1 - I'e-11** and **I'f-1 - I'f-11** and/or at least one of the conjugates comprising at least one of the saccharides **I'a-1** - **I'a-11, I'a-1** - **I'b-11, I**'**b-1 - I'c-11, I'c-1 - I'c-11, I'd-1 - I'd-11, I'e-1 - I'e-11** and **I'f-1 - I'f-11** for the manufacture of said pharmaceutical composition or said vaccine.

Particularly, the present invention refers to the use of at least one conjugate of any one of general formulae **(IV), (V)** and **(V-1)** - **(V-11)** for the manufacture of said pharmaceutical composition or said vaccine.
Pharmaceutical compositions may be prepared in unit dose form. In some embodiments a unit dose may have a volume of between 0.1-1.0 mL e.g. about 0.5 mL.

The invention also provides a delivery device (e.g. syringe, nebuliser, sprayer, inhaler, dermal patch, etc.) containing a pharmaceutical composition of the invention e.g. containing a unit dose. This device can be used to administer the composition to a vertebrate subject.

The invention also provides a sterile container (e.g. a vial) containing a pharmaceutical composition of the invention e.g. containing a unit dose.

The invention also provides a unit dose of a pharmaceutical composition of the invention.

The invention also provides a hermetically sealed container containing a pharmaceutical composition of the invention. Suitable containers include e.g. a vial.

Pharmaceutical compositions of the invention may be prepared in various forms. For example, the compositions may be prepared as injectables, either as liquid solutions or suspensions. Solid forms suitable for solution in, or suspension in, liquid vehicles prior to injection can also be prepared (e.g. a lyophilised composition or a spray-freeze dried composition). The composition may be prepared for topical administration e.g. as an ointment, cream or powder. The composition may be prepared for oral administration e.g. as a tablet or capsule, as a spray, or as a syrup (optionally flavoured). The composition may be prepared for pulmonary administration e.g. by an inhaler, using a fine powder or a spray. The composition may be prepared as a suppository. The composition may be prepared for nasal, aural or ocular administration e.g. as a spray or drops. Injectables for intramuscular administration are typical.

The pharmaceutical compositions may comprise an effective amount of an adjuvant i.e. an amount which, when administered to an individual, either in a single dose or as part of a series, is effective for enhancing the immune response to a co-administered *Klebsiella pneumoniae* serotype O3, O3b and/or O5 antigen.

This amount can vary depending upon the health and physical condition of the individual to be treated, age, the taxonomic group of individual to be treated (e.g. non-human primate, primate, etc.), the capacity of the individual's immune system to synthesize antibodies, the degree of protection desired, the formulation of the vaccine, the treating doctor's assessment of the medical situation, and other relevant factors. The amount will fall in a relatively broad range that can be determined through routine trials.

Techniques for the formulation and administration of the vaccine of the present invention may be found in "Remington's Pharmaceutical Sciences" Mack Publishing Co., Easton PA.

A therapeutically effective dosage of one conjugate according to the present invention or of one saccharide of general formula (**I**) refers to that amount of the compound that results in an at least a partial immunization against a disease. Toxicity and therapeutic efficacy of such compounds can be determined by standard pharmaceutical, pharmacological, and toxicological procedures in cell cultures or experimental animals. The dose ratio between toxic and therapeutic effect is the therapeutic index. The actual amount of the composition administered will be dependent on the subject being treated, on the subject's weight, the severity of the affliction, the manner of administration and the judgment of the prescribing physician.

Another aspect of the present invention is directed to a method of inducing immune response against *Klebsiella pneumoniae* serotype 03, O3b and/or 05 in a human and/or animal host, said method comprising administering of the saccharide of general formula (**I**) and/or salt thereof and/or a conjugate thereof or pharmaceutical composition thereof to said human and/or animal host. A method of treating or preventing diseases caused by *Klebsiella pneumoniae* serotype 03, O3b and/or 05, in a human and/or animal host according to the present invention comprises administering of at least one saccharide of general formula (**I**) and/or salt thereof and/or a conjugate thereof or pharmaceutical composition thereof to said human and/or animal host.

### Immunological assays

Yet another aspect of the present invention refers to saccharide of general formula (**I**) for use as marker in immunological assays for detection of antibodies against bacteria containing in their O-polysaccharide one of the following saccharide fragments:
- 2)-α-D-Man-(1, 2)-α-D-Man-(1, 2)-α-D-Man-(1, 3)-α-D-Man-(1, 3)-α-D-Man-(1-;
- 3)-α-D-Man-(1, 2)-α-D-Man-(1, 2)-α-D-Man-(1, 2)-α-D-Man-(1, 3)-α-D-Man-(1-;
- 3)-α-D-Man-(1, 3)-α-D-Man-(1, 2)-α-D-Man-(1, 2)-α-D-Man-(1, 2)-α-D-Man-(1-;
- 2)-α-D-Man-(1, 3)-α-D-Man-(1, 3)-α-D-Man-(1, 2)-α-D-Man-(1, 2)-α-D-Man-(1-;
- 2)-α-D-Man-(1, 2)-α-D-Man-(1, 3)-α-D-Man-(1, 3)-α-D-Man-(1, 2)-α-D-Man-(1-;
- 3)-β-D-Man-(1, 2)-α-D-Man-(1, 2)-α-D-Man-(1-;
- 2)-α-D-Man-(1, 3)-β-D-Man-(1, 2)-α-D-Man-(1-;
- 2)-α-D-Man-(1, 2)-α-D-Man-(1, 3)-β-D-Man-(1-;
- 2)-α-D-Man-(1, 3)-α-D-Man-(1, 3)-α-D-Man-(1-;
- 3)-α-D-Man-(1, 2)-α-D-Man-(1, 3)-α-D-Man-(1-;
- 3)-α-D-Man-(1, 3)-α-D-Man-(1, 2)-α-D-Man-(1-.

Such assays comprise, for instance, microarray and ELISA useful for detection of antibodies against bacteria containing in their O-polysaccharide one of the above mentioned saccharide fragments, such as *Klebsiella pneumoniae* serotype 03, O3b and/or 05.
The O-antigens of *K. Pneumoniae* 03, O3b and 05 are shared respectively by *E. coli* 09, and 08. The O5-antigen is shared by *Burkholderia ceparcia* 02 and E, and *Serratia marcescens* 028. Therefore, O-polysaccharide one of the above mentioned saccharide fragments can be used for detection of antibodies against *E*. *coli* 08, and O9. *Burkholderia ceparcia* O2 and E and *Serratia marcescens* O28.

The saccharides of the present invention can be easily conjugated to solid supports for providing immunological assays useful for detection of antibodies against *Klebsiella pneumoniae* serotype O3, O3b and/or O5. Said solid supports present on their surface a functionality that is prone to react with the amino group of saccharides of general formula (**I**) or with the functional group Y of the interconnecting molecule to provide modified solid supports, presenting on their surface the functional group X of the interconnecting molecule that can further react with the amino group of saccharides of general formula (**I**). In an embodiment according to the present invention the solid supports are microarray slides, which present on their surface a functionality that is prone to react with the functional group Y of the interconnecting molecule to provide modified microarray slides, presenting of their surface the functional group X of the interconnecting molecule. Examples of such microarray slides include, but are not restricted to Corning® epoxide coated slides or Corning® GAPS™ II coated slides.

In a preferred embodiment the solid supports are microarray slides presenting on their surface a functionality that is prone to react with the amino group of saccharides of general formula (**I**), and more preferably an *N*-hydroxysuccinimide (NHS) activated ester. Such microarray slides are for example CodeLink® NHS slides.

### Description of the figures

**Figure 1** shows the chemical structure of the repeating unit of *Klebsiella pneumoniae* serotype O3, O3b and O5 O-polysaccharide.
**Figure 2** provides examples of functional group X of the interconnecting molecule according to the present invention.
**Figure 3** provides examples of functional group X of the interconnecting molecule according to the present invention.
**Figure 4** shows a CRM₁₉₇ conjugate of the present invention.

The following examples are included to demonstrate preferred embodiments of the invention. It should be appreciated by those skilled in the art that the techniques disclosed in the examples, which follow represent techniques discovered by the inventor to function well in the practice of the invention, and thus can be considered to constitute preferred modes for its practice. However, those skilled in the art should, in light of the present disclosure, appreciate that many changes can be made in the specific embodiments, which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Examples

### A. Chemical synthesis

### General information:

Commercial grade solvents were used unless stated otherwise. Dry solvents were obtained from a Waters Dry Solvent System. Solvents for chromatography were distilled prior to use. Sensitive reactions were carried out in heat-dried glassware and under an argon atmosphere. Analytical thin layer chromatography (TLC) was performed on Kieselgel 60 F254 glass plates precoated with a 0.25 mm thickness of silica gel. Spots were visualized by staining with vanillin solution (6% (w/v) vanillin and 10% (v/v) sulfuric acid in 95% EtOH) or Hanessian's stain (5% (w/v) ammonium molybdate, 1% (w/v) cerium(II) sulfate and 10% (v/v) sulfuric acid in water). Silica column chromatography was performed on Fluka Kieselgel 60 (230-400 mesh).

¹H, ¹³C and two-dimensional NMR spectra were measured with a Varian 400-MR spectrometer at 296 K. Chemical shifts (d) are reported in parts per million (ppm) relative to the respective residual solvent peaks (CDCl₃: d 7.27 in ¹H and 77.23 in ¹³C NMR; CD₃OD: d 3.31 in ¹H and 49.15 in ¹³C NMR). The following abbreviations are used to indicate peak multiplicities: s singlet; d doublet; *dd* doublet of doublets; *t* triplet; *dt* doublet of triplets; q quartet; m multiplet. Coupling constants (*J*) are reported in Hertz (Hz). Optical rotation (OR) measurements were carried out with a Schmidt & Haensch UniPol L1000 polarimeter at λ = 589 nm and a concentration (c) expressed in g/100 mL in the solvent noted in parentheses. High resolution mass spectrometry (HRMS) was performed at the Free University Berlin, Mass Spectrometry Core Facility, with an Agilent 6210 ESI-TOF mass spectrometer. Infrared (IR) spectra were measured with a Perkin Elmer 100 FTIR spectrometer.

### Abbreviations

- AcOH: Acetic acid
- Alloc: Allyloxycarbonyl
- aq.: aqueous
- BH₃: borane
- BBr₃: boron tribromide
- Boc: tert-Butoxycarbonyl
- br.: broad
- CAS: CAS Registry Number (CAS = Chemical Abstracts Service)
- CHCl₃: chloroform
- cHex: cyclohexane
- d: doublet
- dd: doublet of doublets
- DCM: dichloromethane
- DEAD: diethyl azodicarboxylate
- DIPEA: N,N-diisopropyl-ethylamine
- DME: dimethoxyethane
- DMF: dimethylformamide
- DMSO: dimethylsulfoxide
- DPPA: diphenylphosphoryl azide
- EDC•HCl: N1 -((ethylimino)methylene)-N3,N3-dimethylpropane-1 ,3-diamine hydrochloride
- ES: electrospray
- Et₂O: diethyl ether
- EtOAc: ethyl acetate
- FCS: fetal calf serum
- GSDMD: Gasdermin-D
- h: hour
- HCI: hydrochloric acid
- HEK293T: embryonic kidney fibroblast cell line
- H₂O: water
- HOBt.H₂O: 1H-benzo[d][1,2,3]triazol-1-ol hydrate
- hPBMC: human Peripheral Blood Mononuclear Cells
- IC₅₀: half maximal inhibitory concentration
- K₂CO₃: potassium carbonate
- LDH: lactate dehydrogenase
- LiAlH₄: lithium aluminium hydride
- m: multiplet
- MeCN: acetonitrile
- MeOH: methanol
- Mel: methyl iodide
- MgSO₄: magnesium sulphate
- min: minutes
- MS: mass spectrometry
- Na₂CO₃: sodium carbonate
- NaCNBH₃: sodium cyanoborohydride
- NaHCO₃: sodium hydrogencarbonate
- NaH: sodium hydride
- NaOH: sodium hydroxyde
- Na₂SO₄: sodium sulphate
- NCS: N-chlorosuccinimide
- NET: neutrophil extracellular traps
- NMR: nuclear magnetic resonance
- PBS: phosphate-buffered saline
- Pd/C: palladium on carbon
- Pd(PPh₃)₄: Tetrakis(triphenylphosphine)palladium(0)
- PMA: phorbol 12-myristate 13-acetate
- PPh₃: triphenylphosphine
- q: quartet
- rt: room temperature
- s: singlet
- sat.: saturated
- sep: septet
- t: triplet
- TFA: trifluoroacetic acid
- THF: tetrahydrofuran
- THP1: acute monocytic leukaemia cancer cell line
- TsOH: tosic acid
- Wt: weight.

### Example 1A: Synthesis of compound 1*

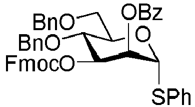

### Example 1B: Synthesis of compound 2*

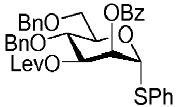

### Example 1C: Synthesis of compound 3*

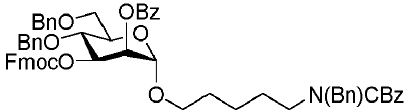

### Example 1D: Synthesis of compound 4*

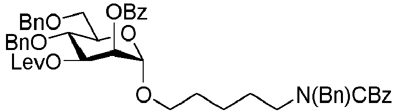

### Example 1E: Synthesis of compound 5*

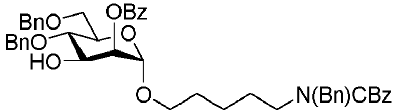

### Example 1F: Synthesis of compound 6*

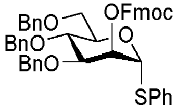

### Example 1G: Synthesis of compound 7*

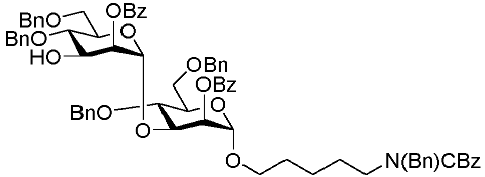

### Example 1H: Synthesis of compound 8*

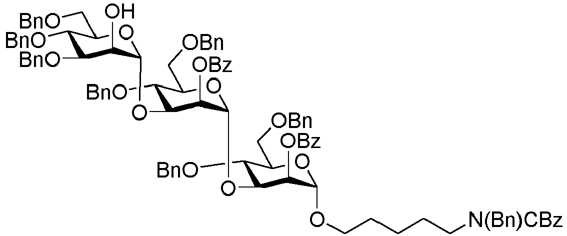

### Example 1G: Synthesis of compound 9*

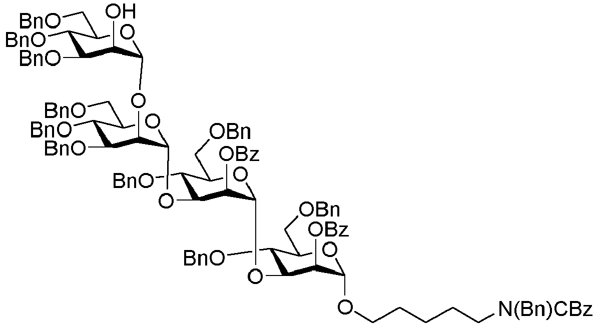

### Example 11: Synthesis of compound 10*

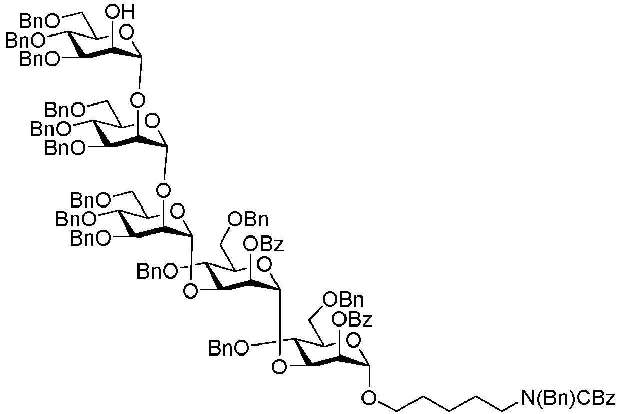

### Example 1J: Synthesis of compound 11*

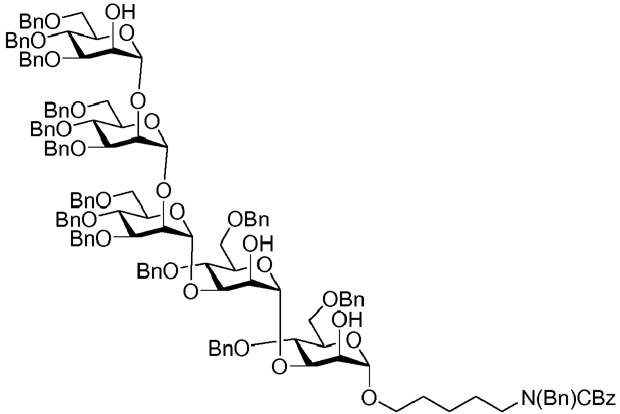

### Example 1K: Synthesis of compound 12*

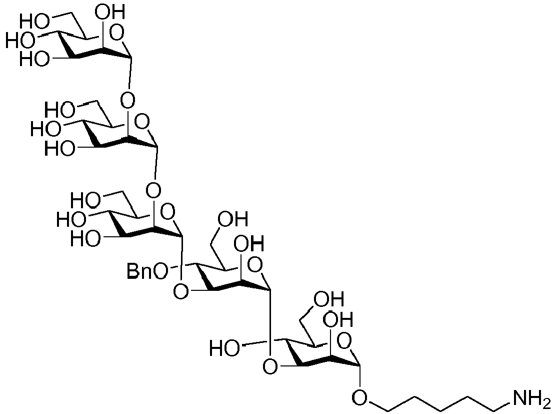

**Glycan microarray analysis:** Saccharides in PBS were printed on N-Hydroxy succinimide activated glass slides. Slides were incubated overnight. Serum dilutions were applied to the slide and incubated. Secondary antibodies (anti-rabbit IgG FITC, anti-rabbit IgM AlexaFluor 647, anti-human IgG-Fc AlexaFluor 488 and IgM AlexaFluor 594) were incubated on the slides and after drying by centrifugation, the slide was scanned using a GenePix 4300A (Molecular Devices) microarray reader.

The primary immune response was assessed by glycan array screening of serum samples retrieved at day 0, day 21 and day 35. Oligosaccharides of the present invention as well as natural O-polysaccharides of *K. pneumoniae* serotypes 03, O3b and 05 were printed on NHS ester-activated microarray slides. After immunization with a conjugate of the present invention, antibodies of the IgG and IgM subtypes against oligosaccharides of the present invention as well as against the natural O-polysaccharides of *K. pneumoniae* serotypes O3, O3b and O5 were detected in all immunized rabbits. Antibodies cross-reacting with the natural O-polysaccharides of *K. pneumoniae* serotypes O3, O3b and O5 indicates the potential of these antibodies to bind to *K. pneumoniae* bacteria and to confer protection against *K. pneumoniae* infection.

### Analysis of the antibody response by ELISA

ELISA was performed using polystyrene 96-well plates. Plates were coated using native O-polysaccharides of *K. pneumoniae.* Plates were blocked and washed once with PBS-T. After applying cell culture supernatants or mAb dilutions (30-50 µL), plates were incubated, washed with PBS-T three times and treated with a horseradish peroxidase (HRP)-labeled secondary antibody. Plates were washed with PBS-T three times and HRP activity was measured with TMB substrate according to the manufacturer's instructions. Endpoint titers were determined as the reciprocal of the highest dilution resulting in an absorbance above a value of 0.1.

The immune response was analyzed at different time points. The specific immunoglobulins by end point titer were quantified. The ELISA data proves that the conjugates of the present invention are immunogenic and induce high antibody titers. The booster response after immunization in terms of fold change was further analyzed. Hence, ELISA analysis shows that the saccharides of formula (I) of the present invention are immunogenic in rabbits and generate cross reactive antibodies.

## Claims

1. A saccharide of general formula (I)
**T*-[(-Uₓ₊₄-Uₓ₊₃-Uₓ₊₂-Uₓ₊₁-Uₓ)ₘ-(Vₓ₊₂-Vₓ₊₁-Vₓ)₁₋ₘ]ₙ- T-O-L-E** (**I**)
wherein
m is an integer selected from 0 and 1;
x is an integer selected from 1 to 2 × m + 3;
n is an integer selected from 1, 2, 3, 4, 5, 6 ,7 ,8, 9 and 10; 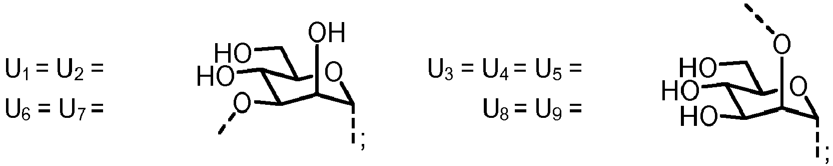 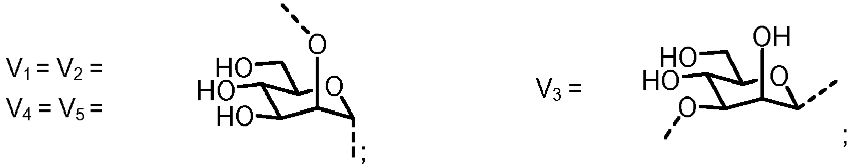 or 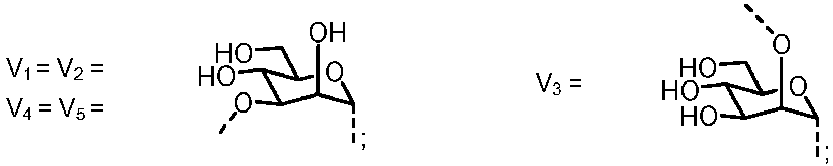
-**T**- represents a bond, -(Uₓ₊₄)ₘ-(Vₓ₊₂)₁₋ₘ-, -(Uₓ₊₄-Uₓ₊₃)ₘ-(Vₓ₊₂-Vₓ₊₁)₁₋ₘ-, -(Uₓ₊₄-Uₓ₊₃-Uₓ₊₂)ₘ- or -(Uₓ₊₄-Uₓ₊₃-Uₓ₊₂-Uₓ₊₁)ₘ-;
**T***- represents H-, H-(Uₓ)ₘ-(Vₓ)₁₋ₘ-, H-(Uₓ₊₁-Uₓ)ₘ-(Vₓ₊₁-Vₓ)₁₋ₘ-H-(Uₓ₊₂-Uₓ₊₁-Uₓ)ₘ- or H-(Uₓ₊₃-Uₓ₊₂-Uₓ₊₁-Uₓ)ₘ-;
**L** represents a linker and;
**E** represents -NH₂, -N₃, -CN, -O-NH₂, -CH=CH₂, -C=CH, -Br, -CI, -I, -CO₂R', -CONH-NH₂, -SH, or -SAc;
**R**' represents -H, -Me, -Et, 4-nitrophenyl, pentafluorophenyl, or *N*-hydroxysuccinimidyl;
or a diastereoisomer or a pharmaceutically acceptable salt thereof.

2. The saccharide according to claim 1 of general formula (II)
**T*-[(-Uₓ₊₄-Uₓ₊₃-Uₓ₊₂-Uₓ₊₁-Uₓ)ₘ-(Vₓ₊₂-Vₓ₊₁-Vₓ)₁₋ₘ]ₙ-O-L-E** **(II)**
wherein m, n, x, L, E, Uₓ₊₁, Uₓ₊₂, Uₓ₊₃, Uₓ₊₄, Vₓ, Vₓ₊₁, Vₓ₊₂ and T* have the meanings as defined in claim 1.

3. The saccharide according to claim 1 of general formula **(III)**
**T*-[(-Uₓ₊₄-Uₓ₊₃-Uₓ₊₂-Uₓ₊₁-Uₓ)ₘ-(Vₓ₊₂-Vₓ₊₁₋Vₓ)₁₋ₘ]ₙ₋(Uₓ₊₄)ₘ-(Vₓ₊₂)₁₋ₘ-O-L-E** (**III**)
wherein m, n, x, L, E, Uₓ₊₁, Uₓ₊₂, Uₓ₊₃, Uₓ₊₄, Vₓ, Vₓ₊₁, Vₓ₊₂ and T* have the meanings as defined in claim 1.

4. The saccharide according to any one of the claims 1 to 3, wherein
**-L-** represents **-L^{a}-, -L^{a}-L^{e}-, -L^{a}-L^{b}-L^{e}-**, or **-L^{a}-L^{d}-L^{e}- ;**
**-L^{a}-** represents -(CH₂)ₒ-, -(CH₂-CH₂-O)ₒ-C₂H₄-, or -(CH₂-CH₂-O)ₒ-CH₂; **-L^{b}-** represents -O-;
**-L^{d}-** represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂-;
**-L^{e}-** represents -(CH₂)ₚ₁-, -(CF₂)ₚ₁-, -C₂H₄-(O-CH₂-CH₂)ₚ₁-,
-CH₂-(O-CH₂-CH₂)ₚ₁- or -(CH₂)ₚ₁-O-(CH₂)ₚ₂-; and
**o, q, p1** and **p2** are independently of each other an integer selected from 1, 2, 3, 4, 5, and 6.

5. The saccharide according to claim 1 selected from the group consisting of:

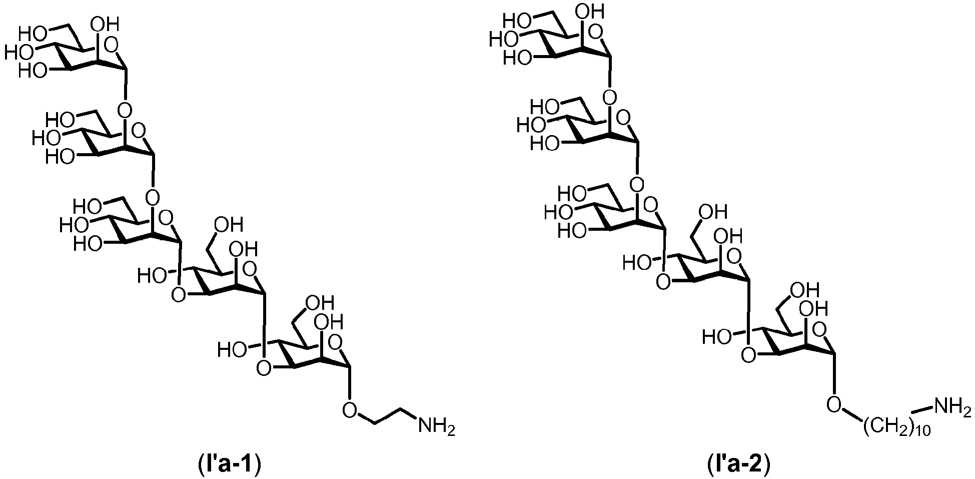

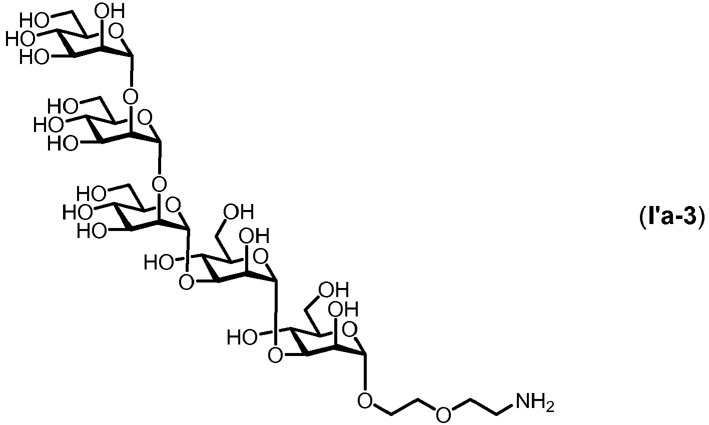

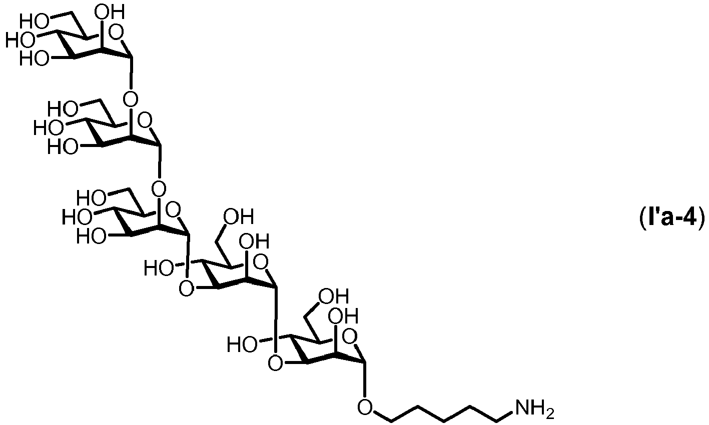

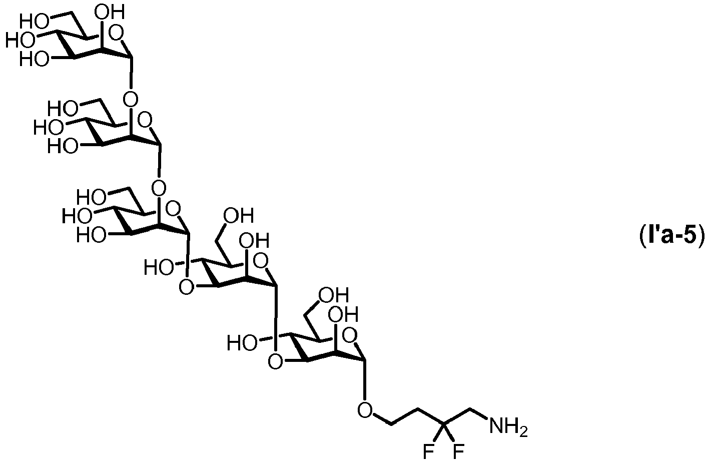

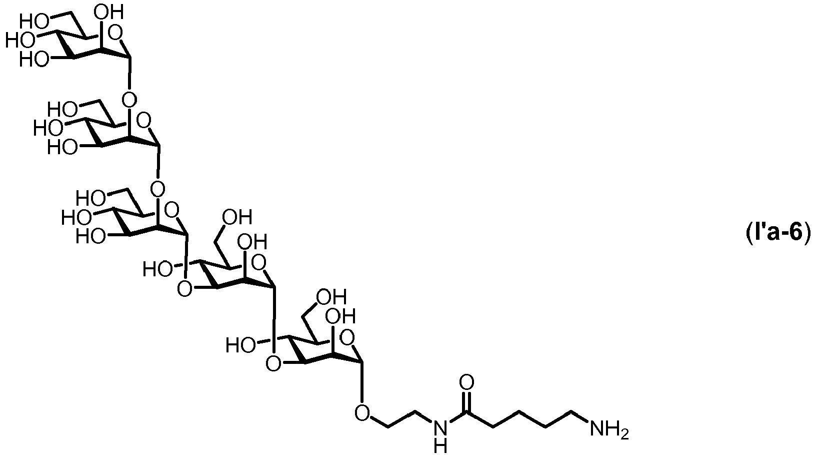

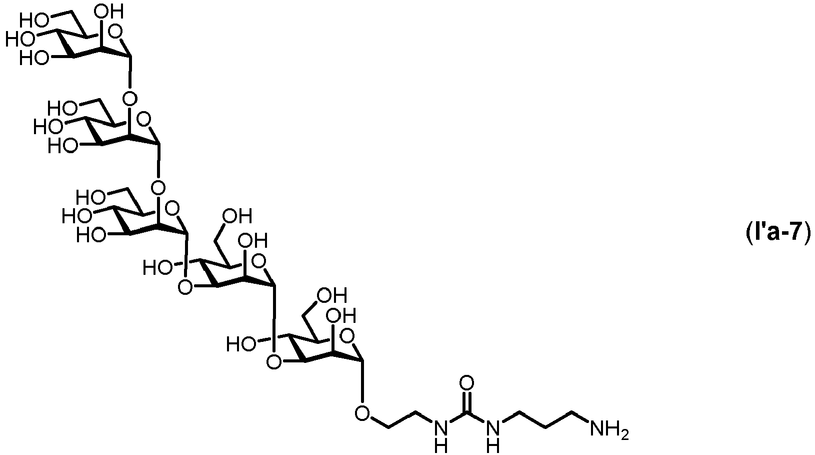

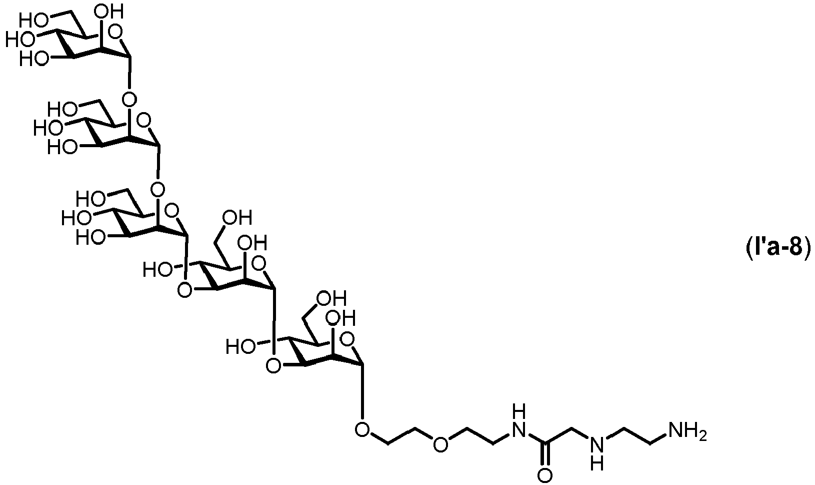

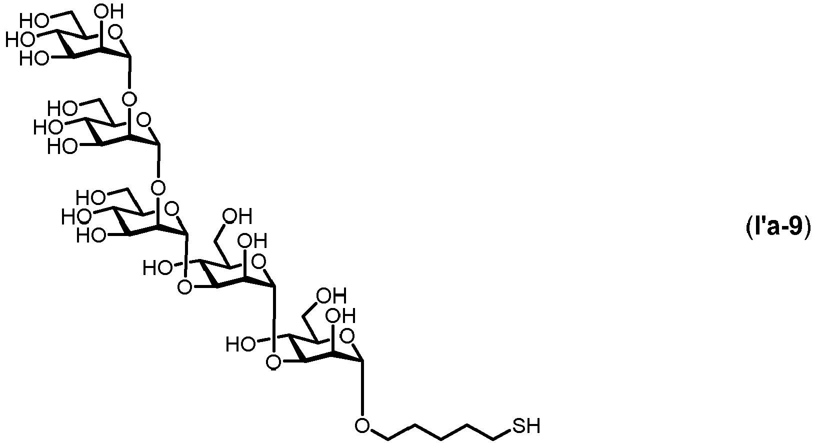

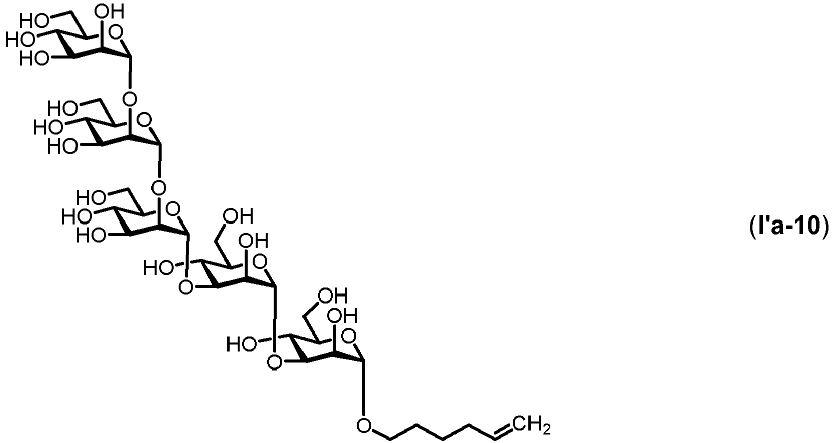

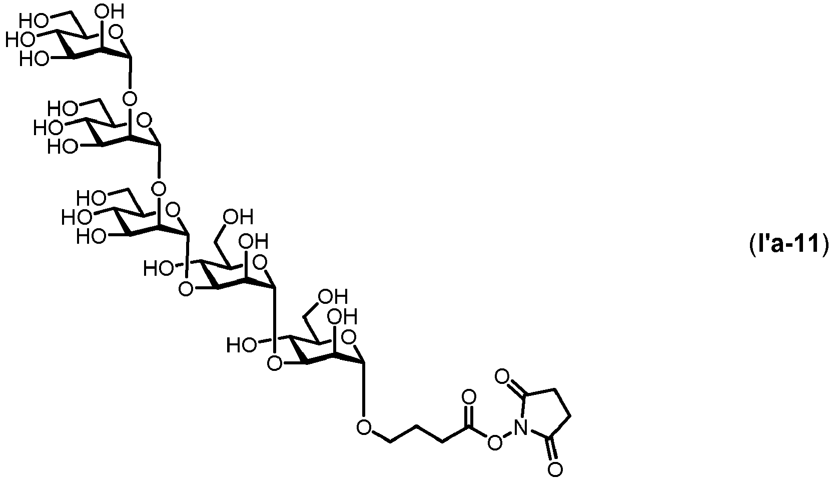

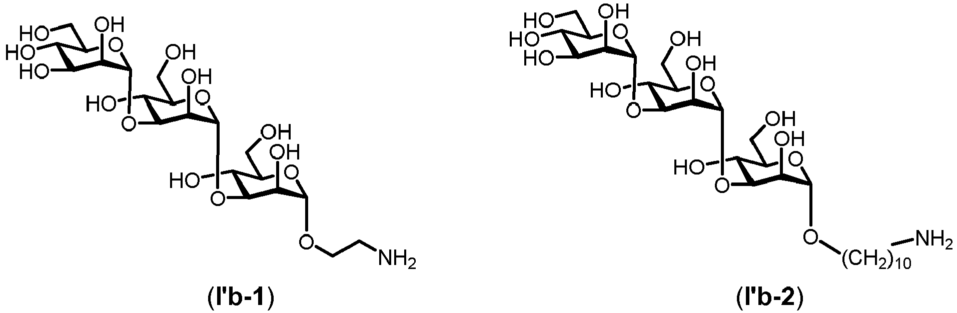

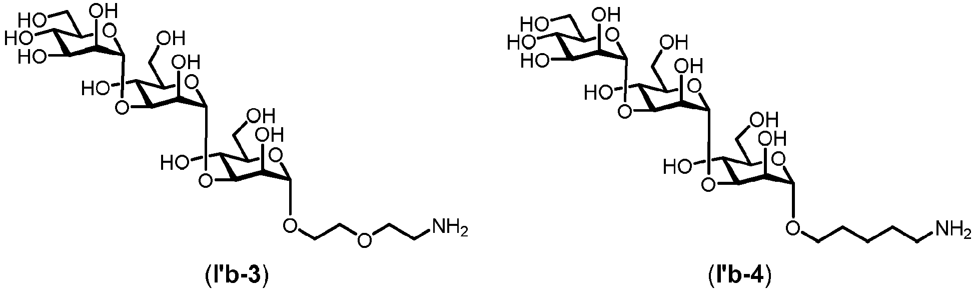

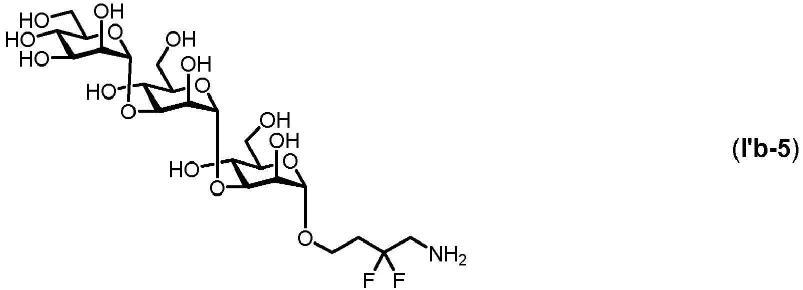

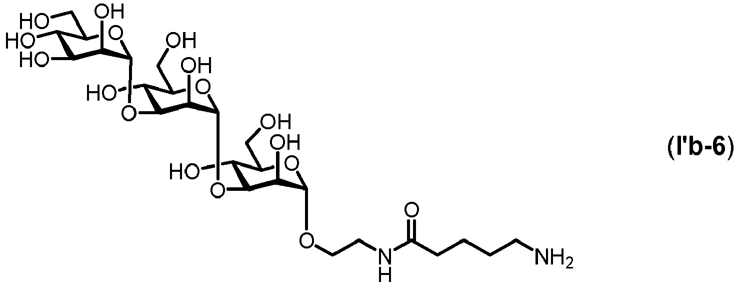

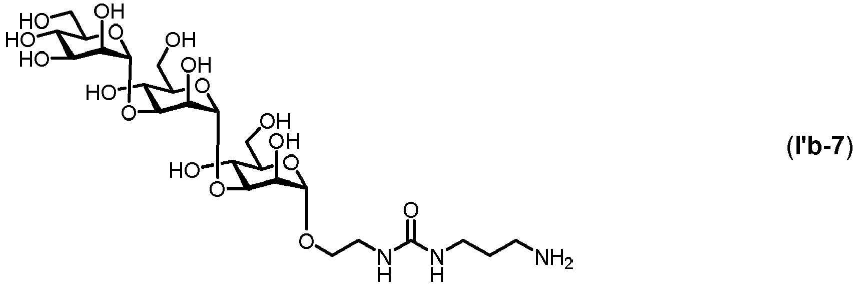

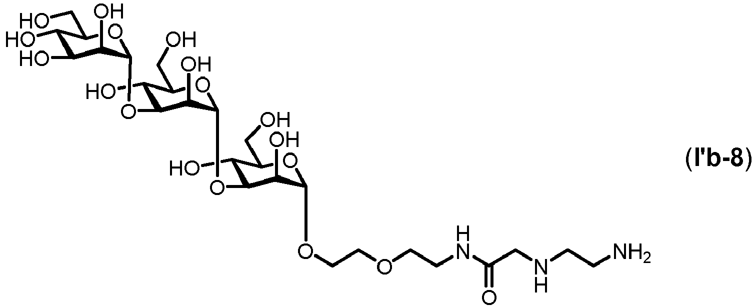

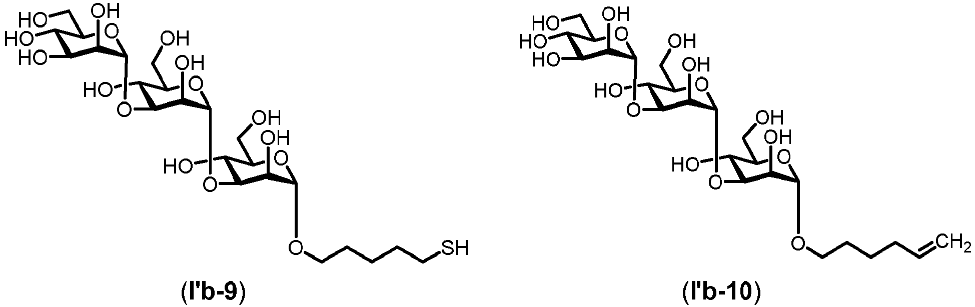

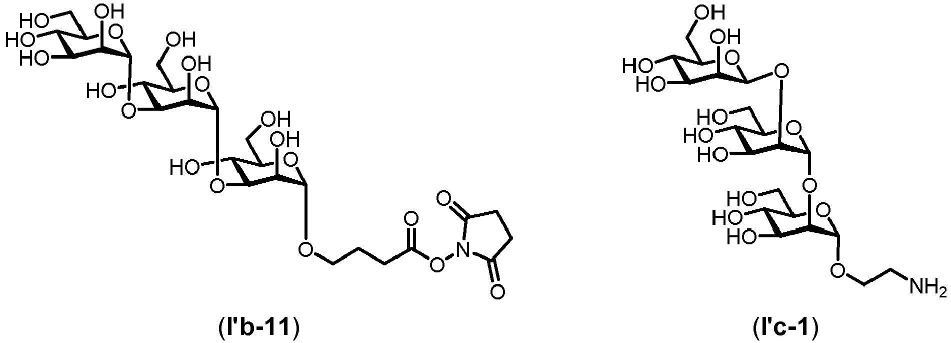

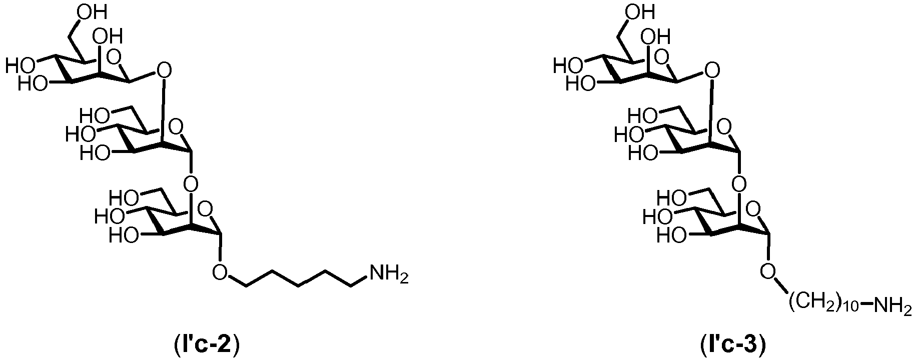

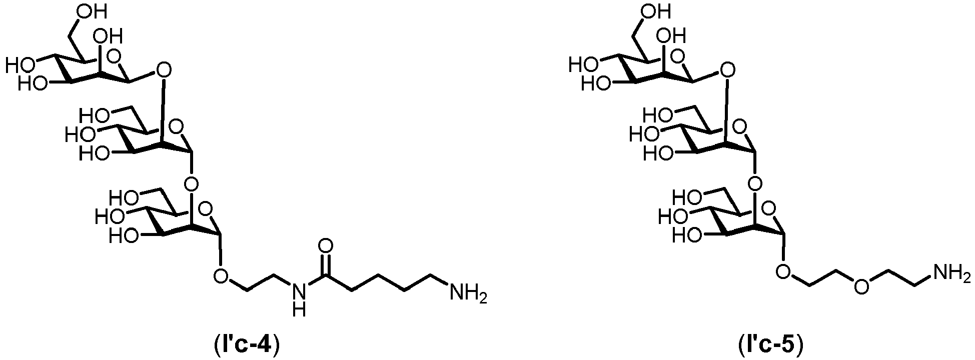

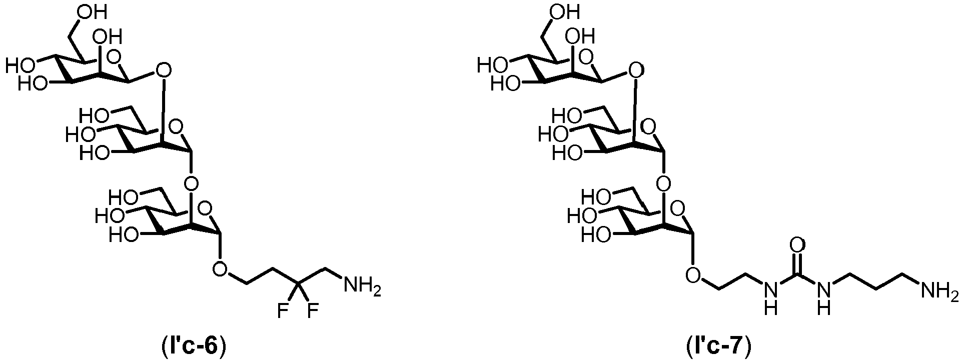

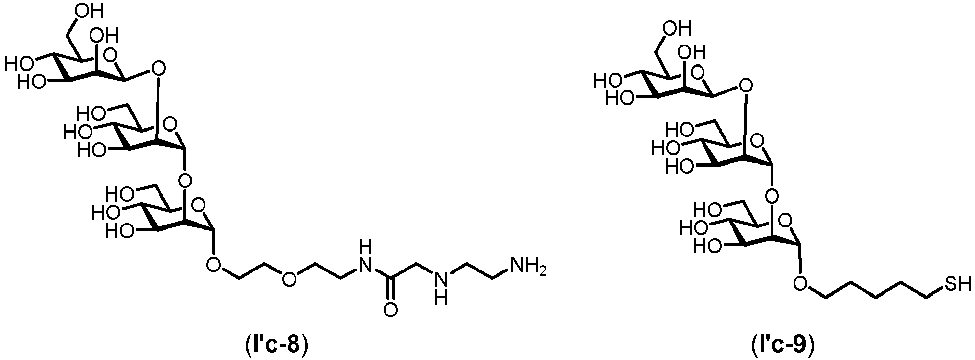

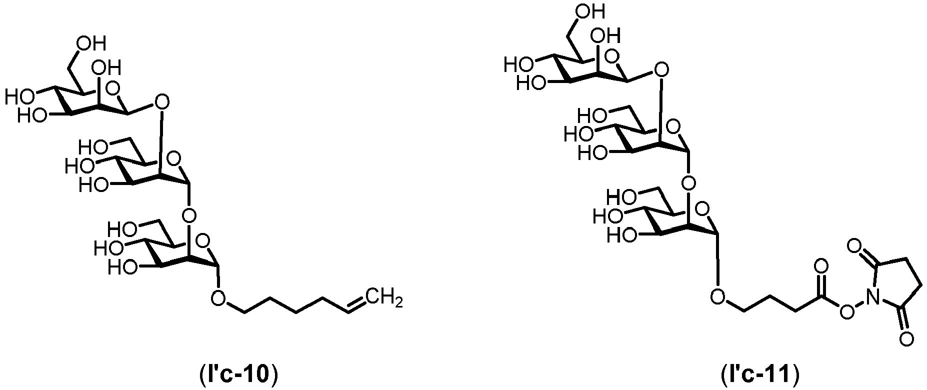

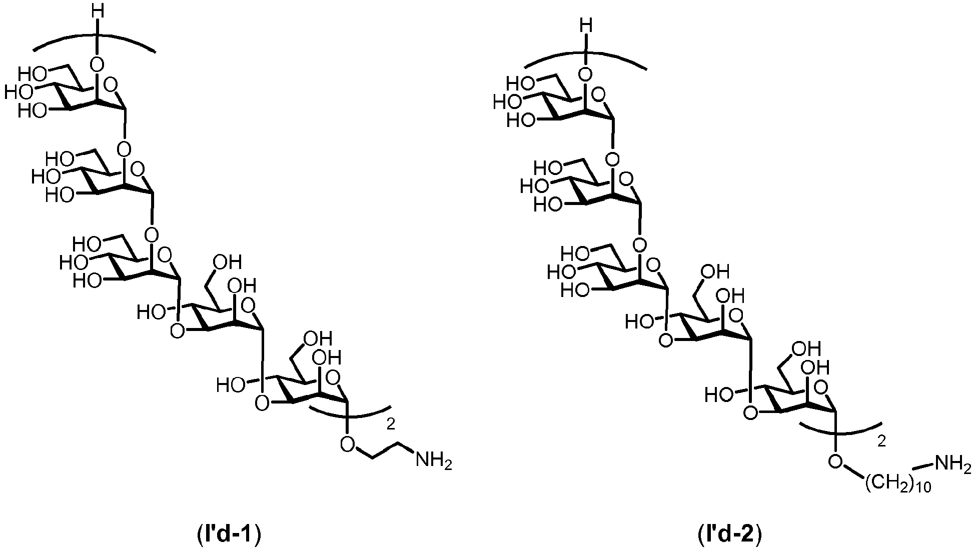

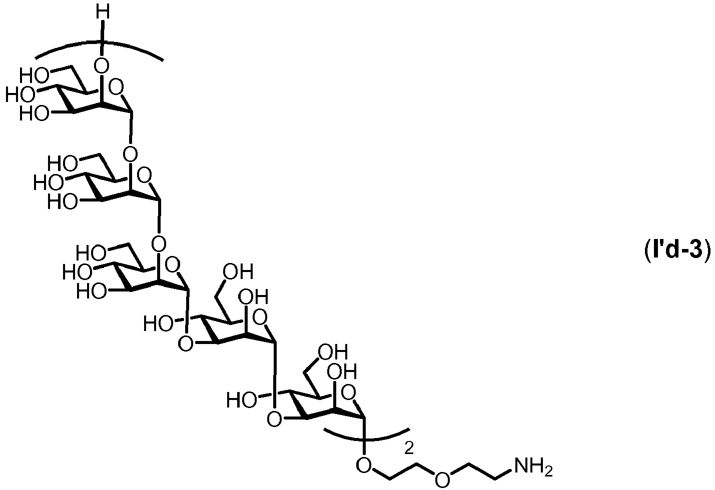

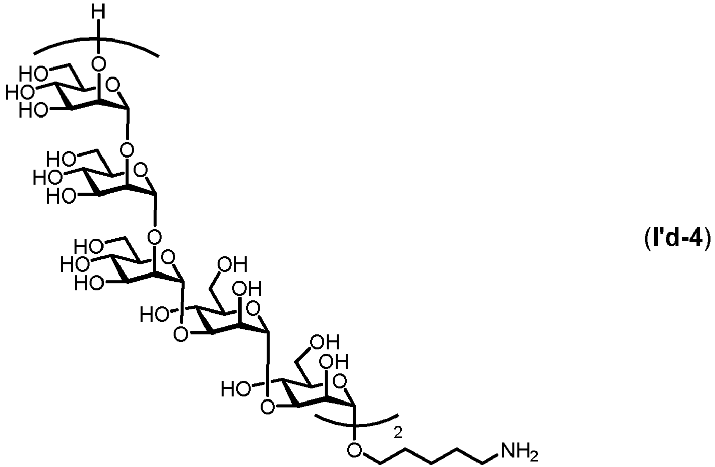

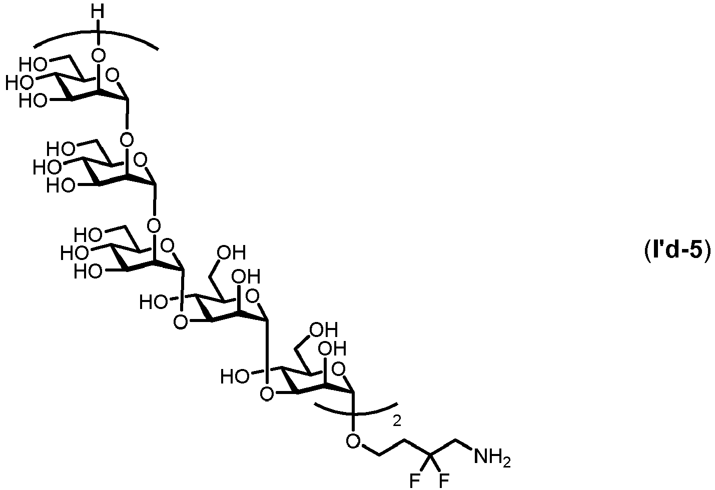

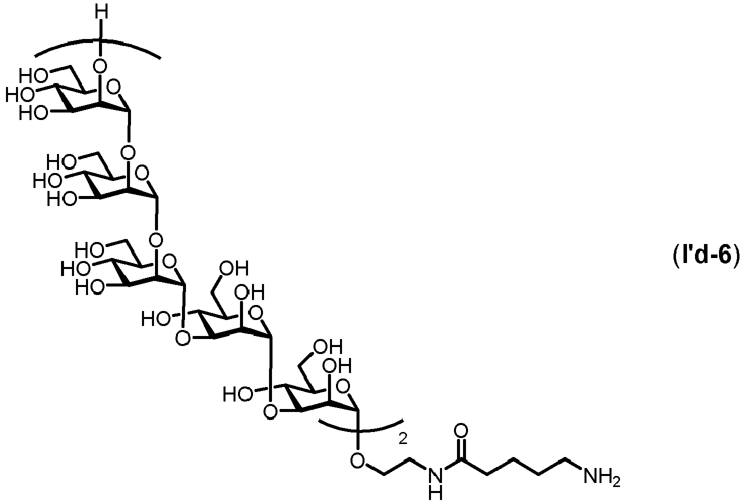

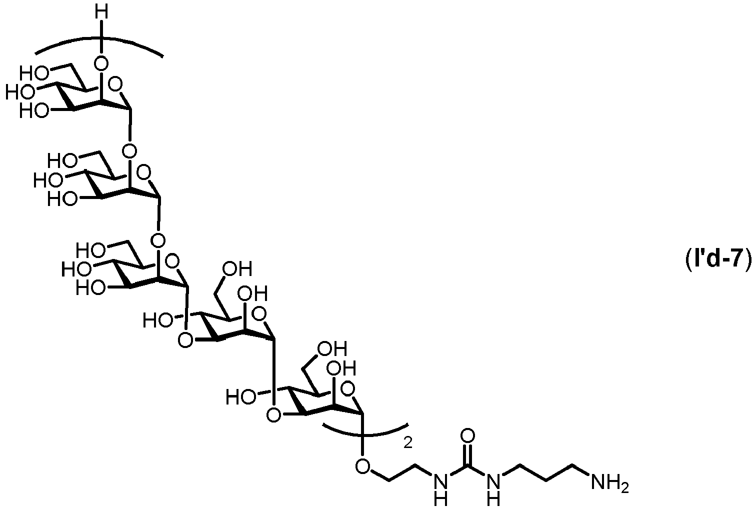

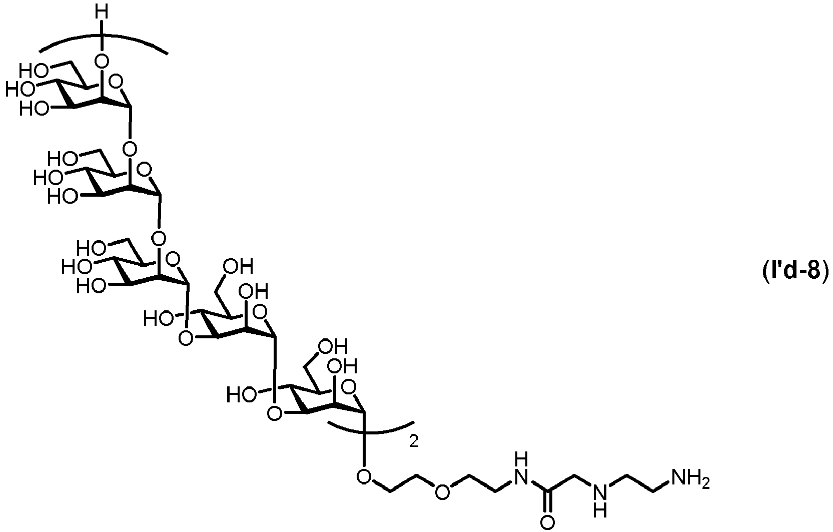

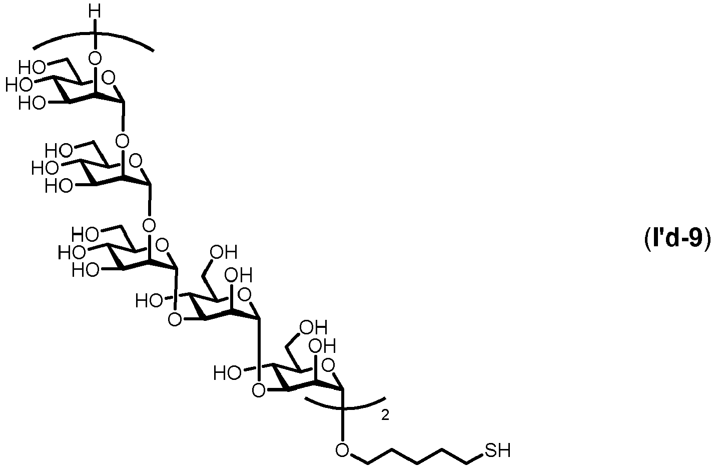

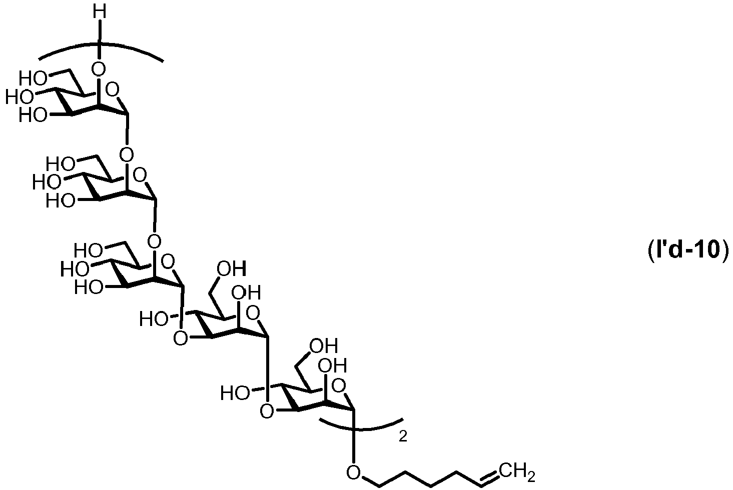

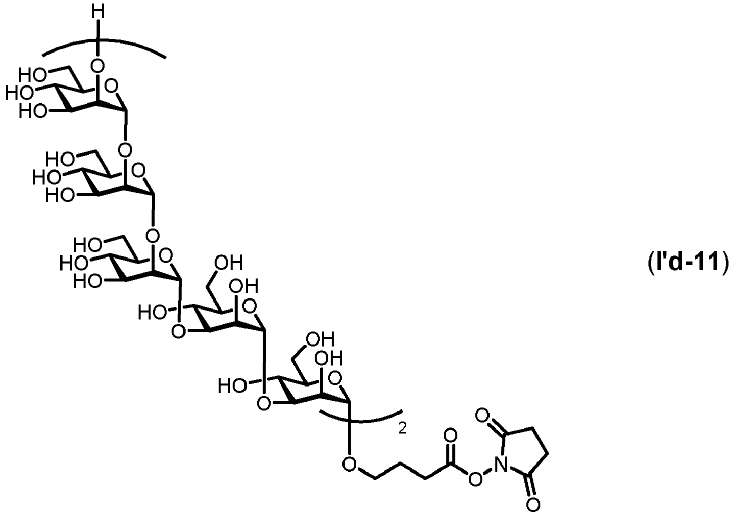

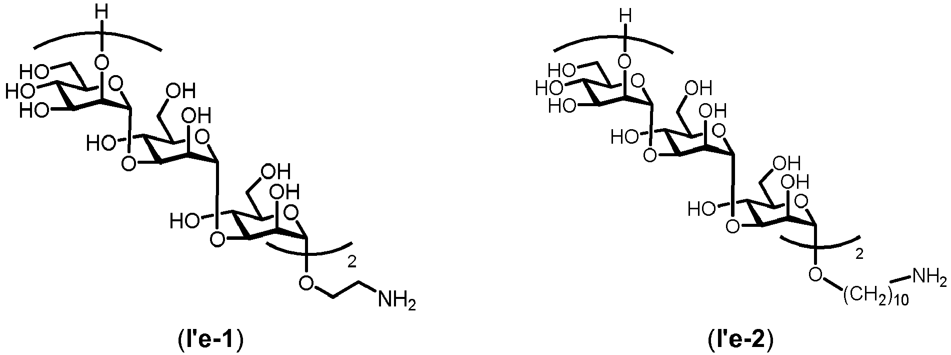

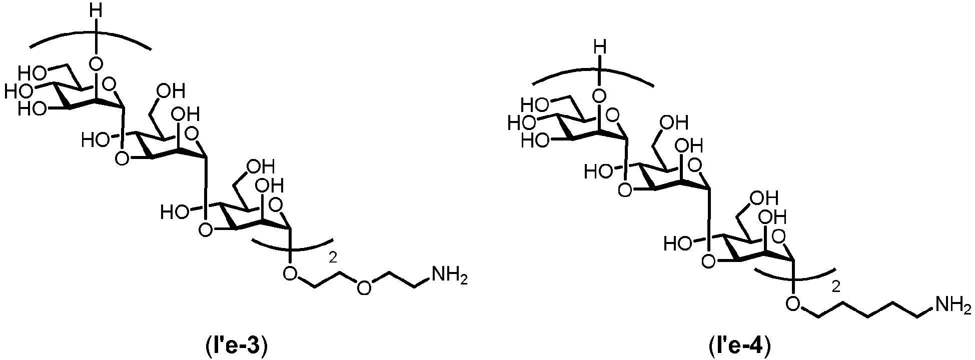

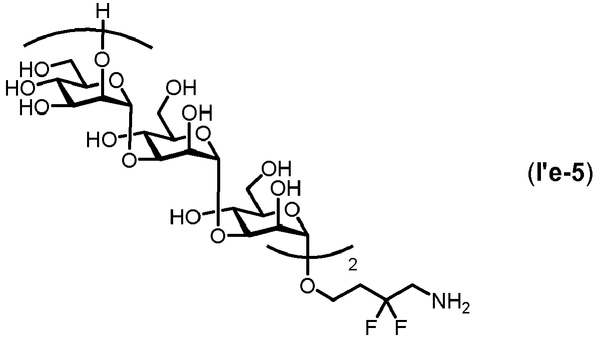

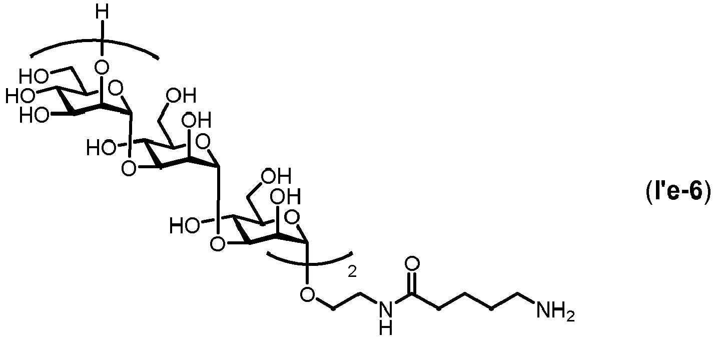

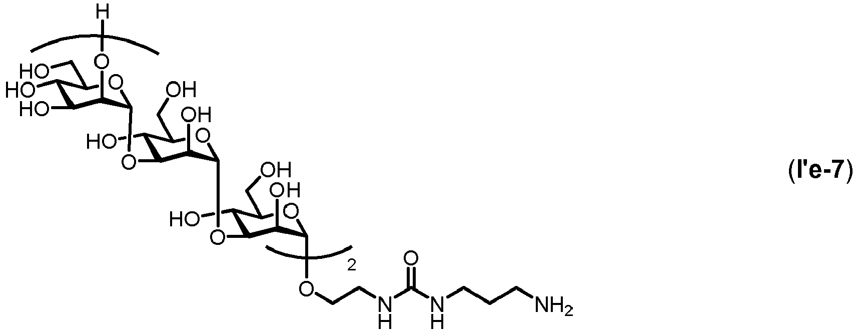

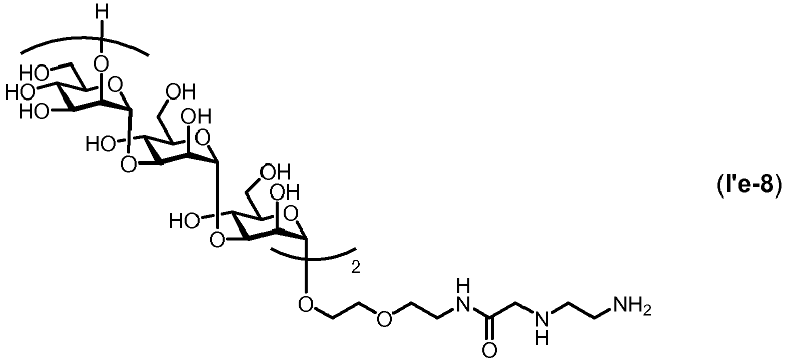

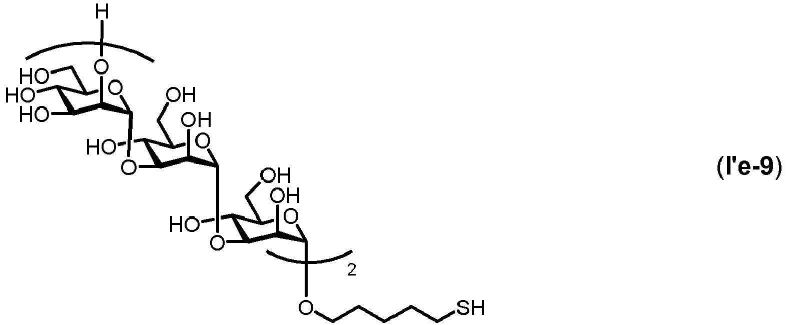

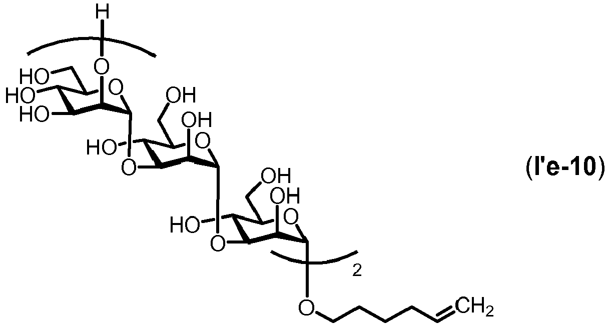

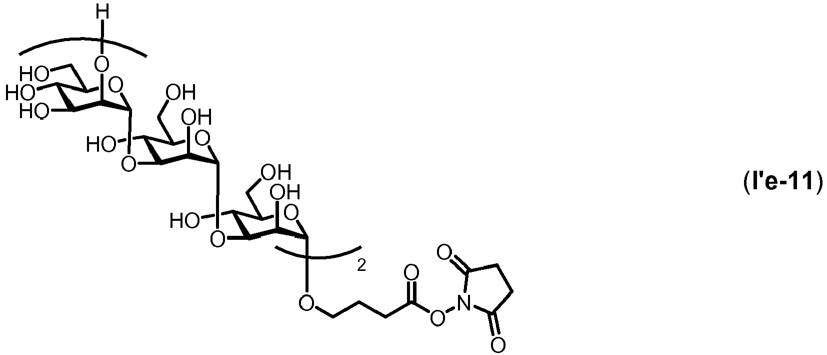

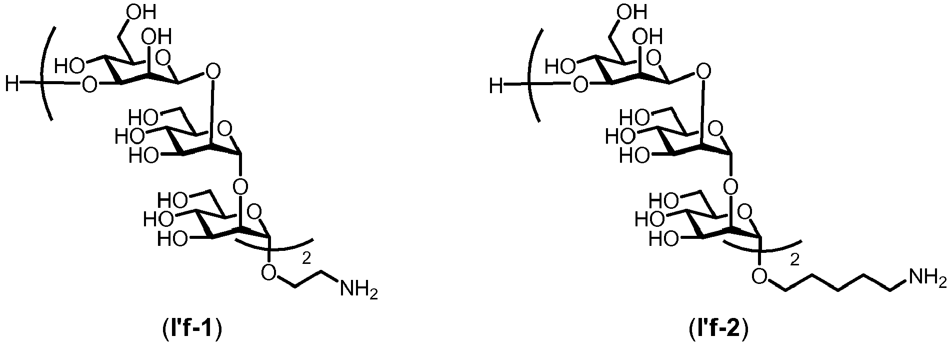

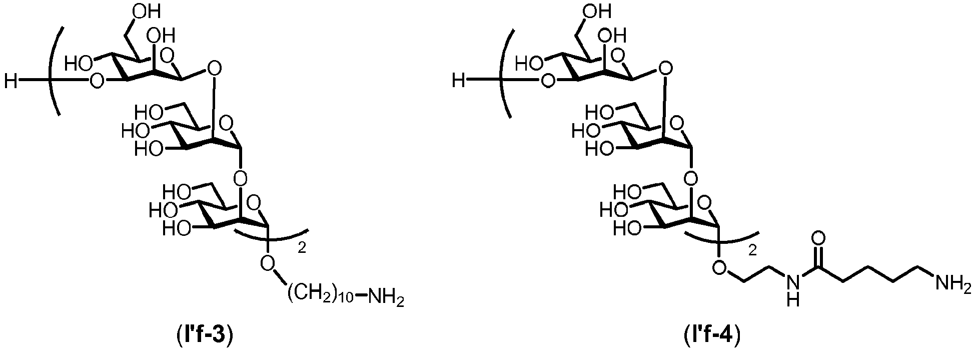

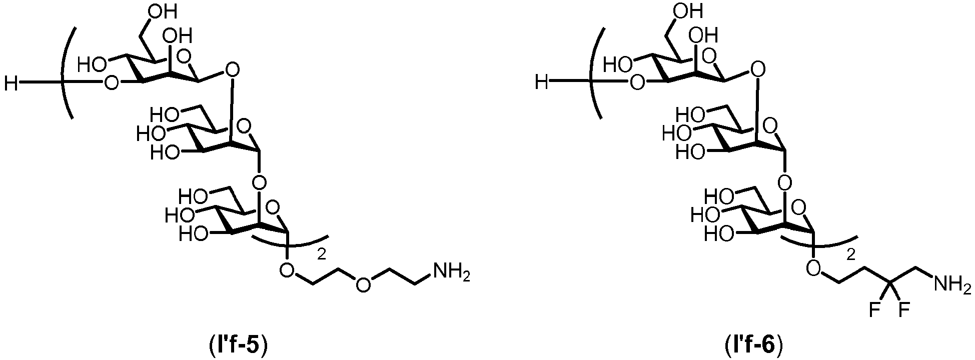

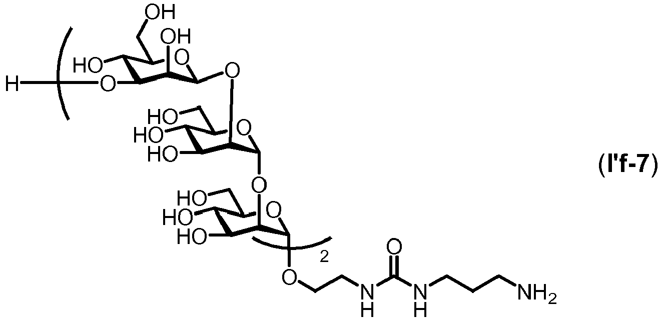

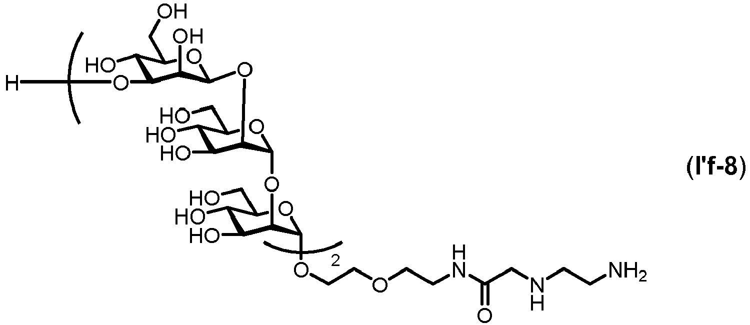

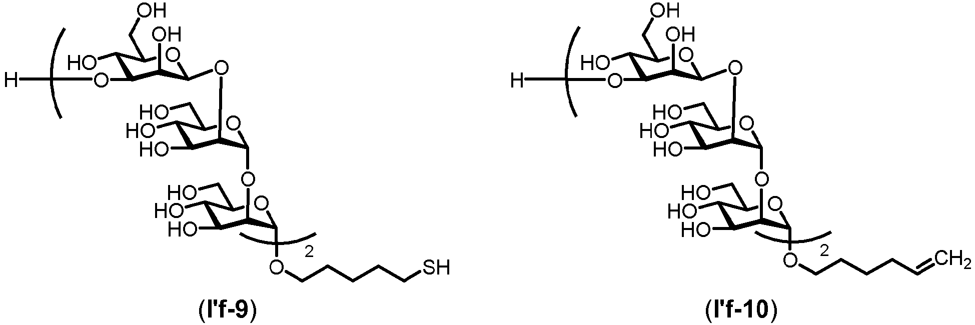

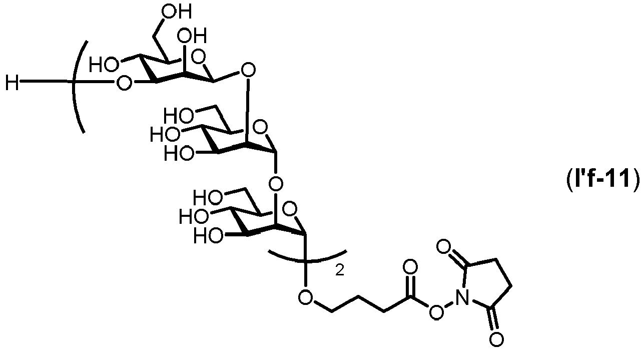

6. A conjugate comprising a saccharide according to any one of the claims 1 - 5 covalently linked to an immunogenic carrier through the residue **E** of the -O-L-**E** group.

7. A saccharide according to any one of the claims 1 - 5 or a conjugate according to claim 6 for use in raising a protective immune response in a human and/or animal host.

8. A saccharide according to any one of the claims 1 - 5 or a conjugate according to claim 6 for use in prevention and/or treatment of diseases associated with bacteria containing in their lipopolysaccharide one of the following saccharide fragments:
-2)-α-D-Man-(1, 2)-α-D-Man-(1, 2)-α-D-Man-(1, 3)-α-D-Man-(1, 3)-α-D-Man-(1-;
-3)-α-D-Man-(1, 2)-α-D-Man-(1, 2)-α-D-Man-(1, 2)-α-D-Man-(1, 3)-α-D-Man-(1-;
-3)-α-D-Man-(1, 3)-α-D-Man-(1, 2)-α-D-Man-(1, 2)-α-D-Man-(1, 2)-α-D-Man-(1-;
-2)-α-D-Man-(1, 3)-α-D-Man-(1, 3)-α-D-Man-(1, 2)-α-D-Man-(1, 2)-α-D-Man-(1-;
-2)-α-D-Man-(1, 2)-α-D-Man-(1, 3)-α-D-Man-(1, 3)-α-D-Man-(1, 2)-α-D-Man-(1-;
-3)-β-D-Man-(1, 2)-α-D-Man-(1, 2)-α-D-Man-(1-;
-2)-α-D-Man-(1, 3)-β-D-Man-(1, 2)-α-D-Man-(1-;
-2)-α-D-Man-(1, 2)-α-D-Man-(1, 3)-β-D-Man-(1-;
-2)-α-D-Man-(1, 3)-α-D-Man-(1, 3)-α-D-Man-(1-;
-3)-α-D-Man-(1, 2)-α-D-Man-(1, 3)-α-D-Man-(1-;
-3)-α-D-Man-(1, 3)-α-D-Man-(1, 2)-α-D-Man-(1-.

9. The saccharide for use or the conjugate for use according to claim 8, wherein the diseases associated with bacteria include pneumonia, bronchitis, meningitis, urinary tract infection, wound infection, osteomyelitis, bacteremia, septicemia and ankylosing spondylitis.

10. A pharmaceutical composition comprising the conjugate according to claim 6 and/or the saccharide according to any one of the claims 1 - 5 together with at least one pharmaceutically acceptable adjuvant and/or excipient.

11. A saccharide according to any one of the claims 1 - 5 for use as marker in immunological assays for detection of antibodies against bacteria containing in their O-polysaccharide one of the following saccharide fragments:
- 2)-α-D-Man-(1, 2)-α-D-Man-(1, 2)-α-D-Man-(1, 3)-α-D-Man-(1, 3)-α-D-Man-(1-;
- 3)-α-D-Man-(1, 2)-α-D-Man-(1, 2)-α-D-Man-(1, 2)-α-D-Man-(1, 3)-α-D-Man-(1-;
- 3)-α-D-Man-(1, 3)-α-D-Man-(1, 2)-α-D-Man-(1, 2)-α-D-Man-(1, 2)-α-D-Man-(1-;
- 2)-α-D-Man-(1, 3)-α-D-Man-(1, 3)-α-D-Man-(1, 2)-α-D-Man-(1, 2)-α-D-Man-(1-;
- 2)-α-D-Man-(1, 2)-α-D-Man-(1, 3)-α-D-Man-(1, 3)-α-D-Man-(1, 2)-α-D-Man-(1-;
- 3)-β-D-Man-(1, 2)-α-D-Man-(1, 2)-α-D-Man-(1-;
- 2)-α-D-Man-(1, 3)-β-D-Man-(1, 2)-α-D-Man-(1-;
- 2)-α-D-Man-(1, 2)-α-D-Man-(1, 3)-β-D-Man-(1-;
- 2)-α-D-Man-(1, 3)-α-D-Man-(1, 3)-α-D-Man-(1-;
- 3)-α-D-Man-(1, 2)-α-D-Man-(1, 3)-α-D-Man-(1-;
- 3)-α-D-Man-(1, 3)-α-D-Man-(1, 2)-α-D-Man-(1-.

12. A method for synthesis of saccharide of general formula (I) comprising of:
**A1)** providing a monosaccharide **1** 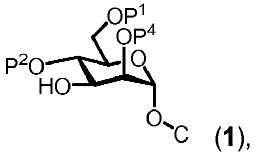 wherein P¹, P² and P⁴ represent protecting groups and C represents -L-Eₚ with Ep being a solid support or a protected end group E, wherein E and L have the meanings as defined in claim 1;
**A2)** treating monosaccharide **1** with a building block **2** in presence of an activating agent 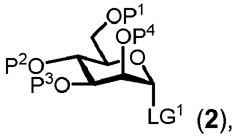 wherein P¹ - P⁴ represent protecting groups and LG¹ represents a leaving group;
**A3)** performing removal of protecting group P³;
**A4)** treating the product of step A3) with a building block **3** in presence of an activating agent 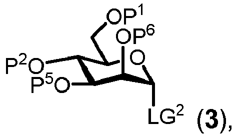 wherein P¹, P², P⁵ and P⁶ represent protecting groups and LG² represents a leaving group;
**A5)** performing removal of protecting group P⁶;
**A6)** repeating steps A4) and A5) two times to obtain intermediate compound **4a** 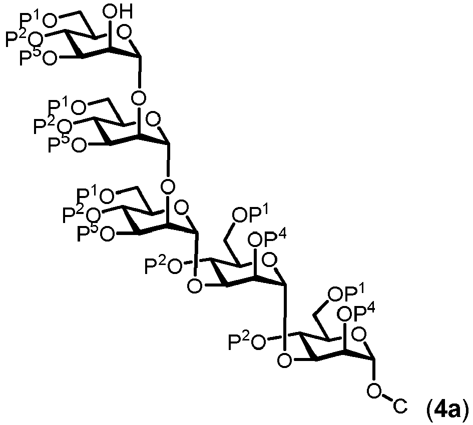 wherein P¹, P², P⁴ and P⁵ represent protecting groups and C represents -L-Eₚ with Ep being a solid support or a protected end group E, wherein E and L have the meanings as defined in claim 1;
**A7)** optionally repeating steps A2) - A6) in the following order A2)→A3)→A2) →A3)→A4)→A5)→A6) n-1 times to obtain intermediate compound of formula **5a,** 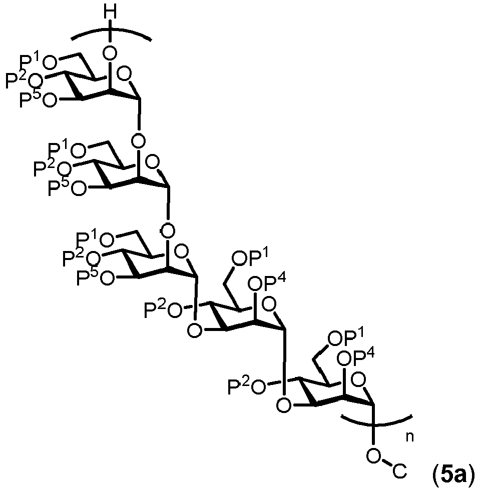 wherein P¹, P², P⁴ and P⁵ represent protecting groups and C represents -L-Eₚ with Ep being a solid support or a protected end group E, wherein n, E and L have the meanings as defined in claim 1;
**A8)** performing removal of all protecting groups to obtain a saccharide of general formula **(I).**
**OR**
a method for synthesis of saccharide of general formula (I) comprising of:
**F1)** providing a monosaccharide 7 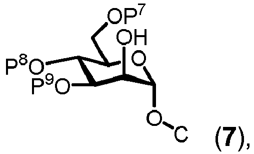 wherein P⁷, P⁸ and P⁹ represent protecting groups and C represents -L-Eₚ with Ep being a solid support or a protected end group E, wherein E and L have the meanings as defined in claim 1;
**F2)** treating monosaccharide **7** with a building block **8** in presence of an activating agent 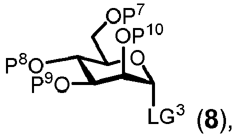 wherein P⁷-P¹⁰ represent protecting groups and LG³ represents a leaving group;
**F3)** performing removal of protecting group P¹⁰;
**F4)** treating the product of step F3) with building block **9** in presence of an activating agent 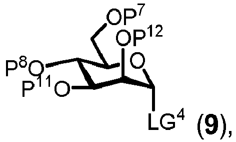 wherein P⁷, P⁸, P¹¹ and P¹² represent protecting groups and LG⁴ represents a leaving group;
**F5)** performing removal of protecting group P¹¹ to obtain intermediate compound **4f;** 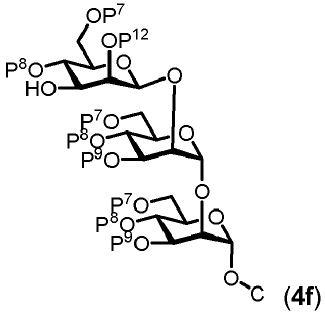 wherein P⁷- P⁹ and P¹² represent protecting groups and C represents -L-Eₚ with Ep being a solid support or a protected end group E, wherein E and L have the meanings as defined in claim 1;
**F6)** optionally repeating steps F2) - F5) in the following order F2)→F3)→F2) →F4)→F5) n-1 times to obtain intermediate compound of formula **5f,** 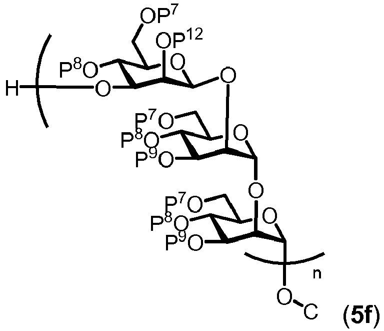 wherein P⁷- P⁹ and P¹² represent protecting groups and C represents -L-Eₚ with Ep being a solid support or a protected end group E, wherein n, E and L have the meanings as defined in claim 1;
**F7)** performing removal of all protecting groups to obtain a saccharide of general formula **(I).**

13. An intermediate compound for preparing a saccharide of the general formulae **(I),** wherein the intermediate compound has any one of general formulae **(I2a), (I2b), (12c), (I2d), (I2e), (I2f), (I2g), (I2h), (13a), (13b), (13c), (13d), (I3e), (I3f), (I3g), (I3h), (I3i),** (**I**3j), **(13k),** (I**3l**), **(13m), (I3n), (14a), (14b), (14c), (I4d), (I4e), (I4f), (I4g), (I4h), (I4i), (I4j), (15a), (15b), (15c), (I5d), (I5e), (I5f), (I5g), (I5h), (I5i)** or (**I5j**): 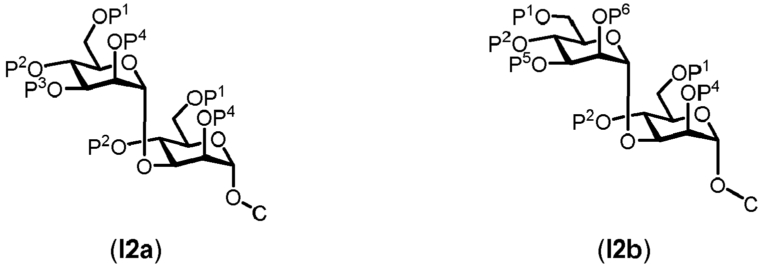 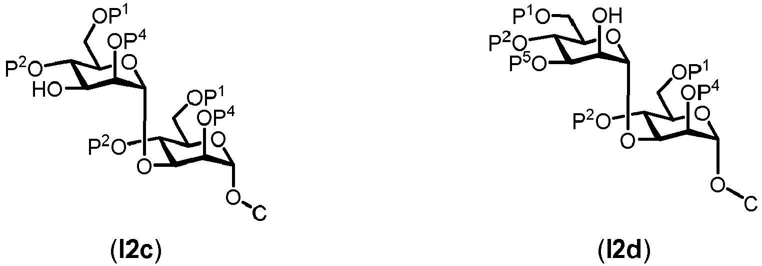 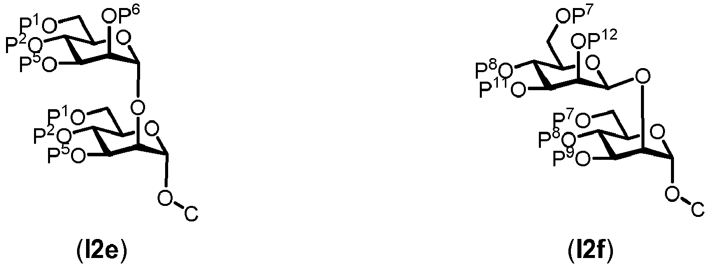 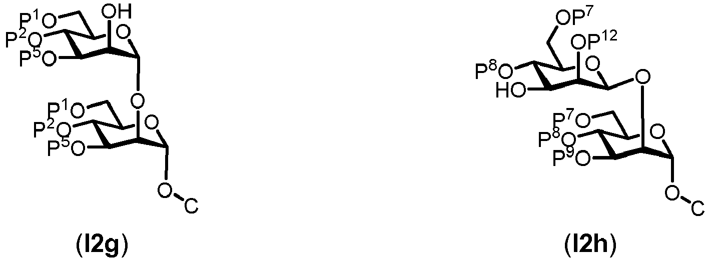 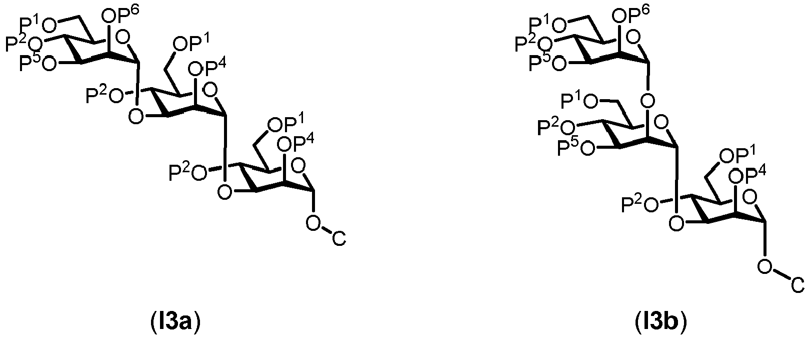 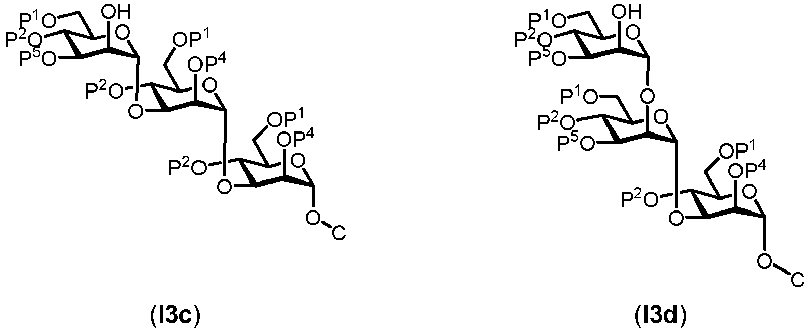 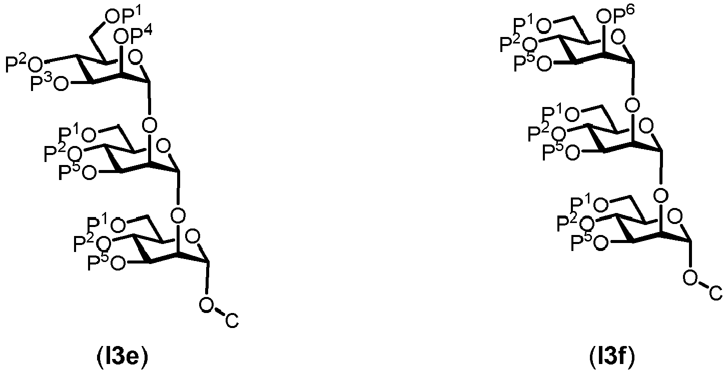 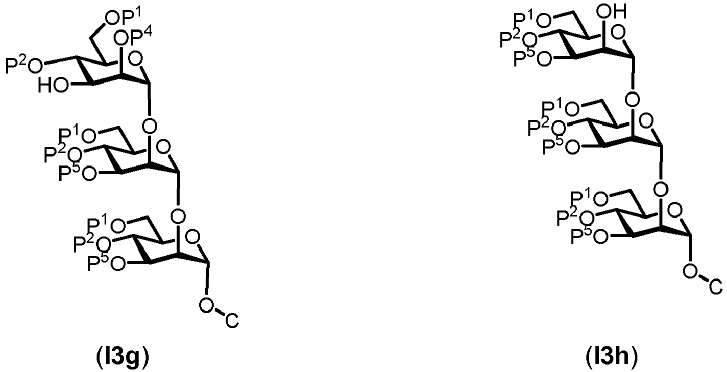 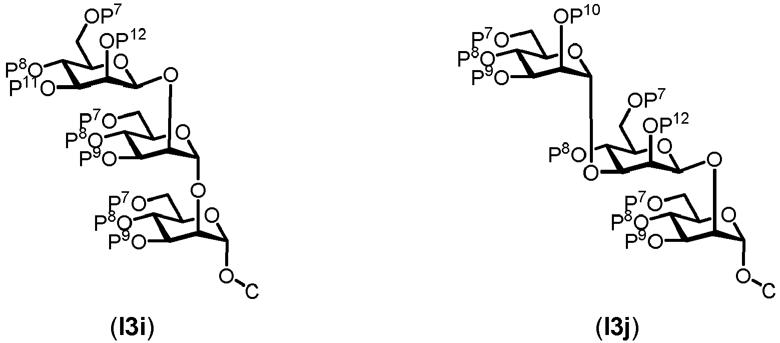 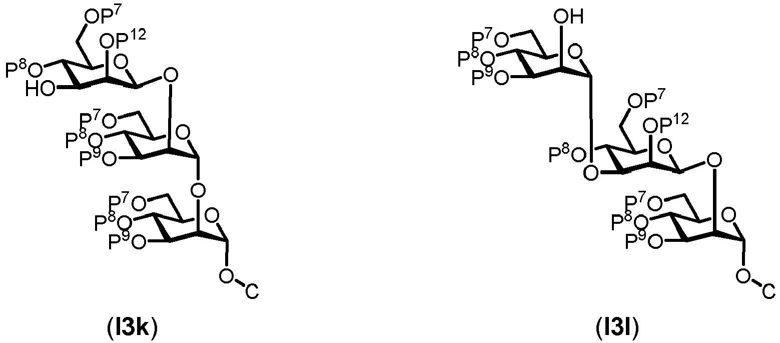 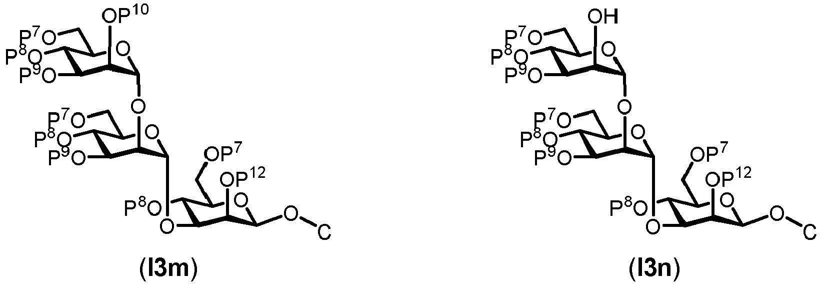 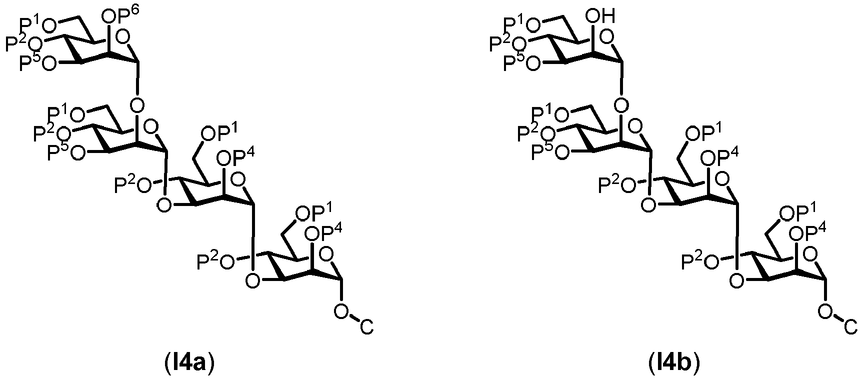 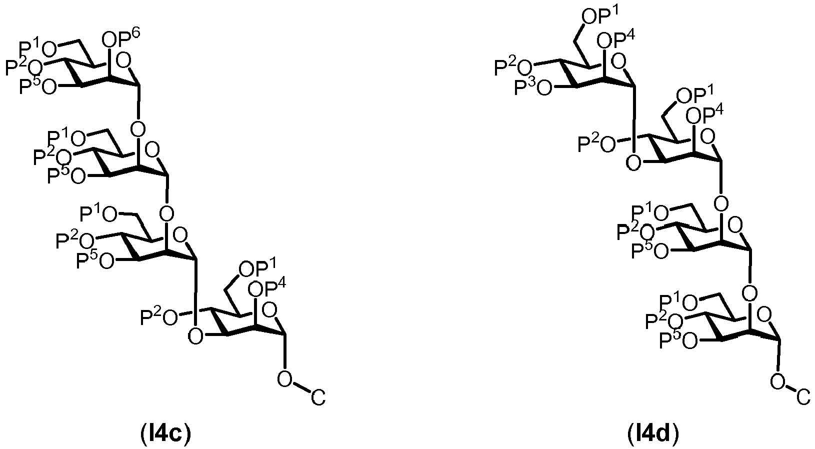 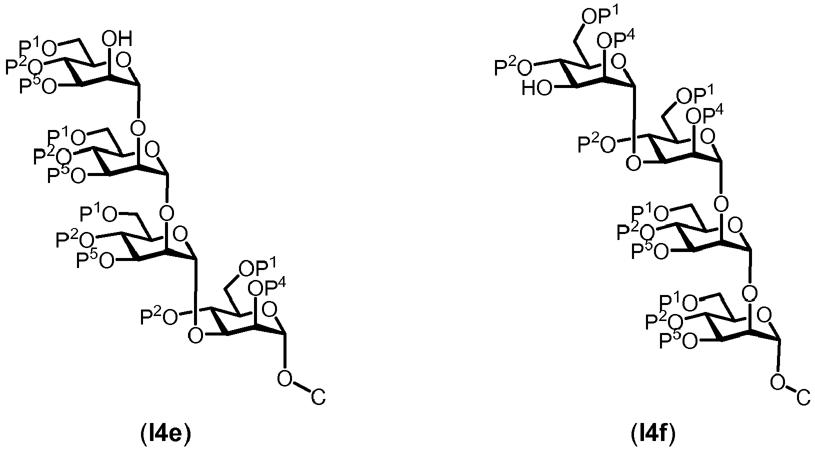 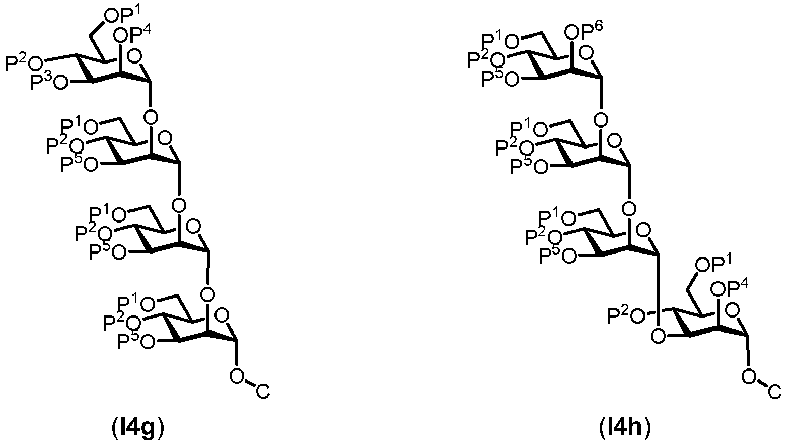 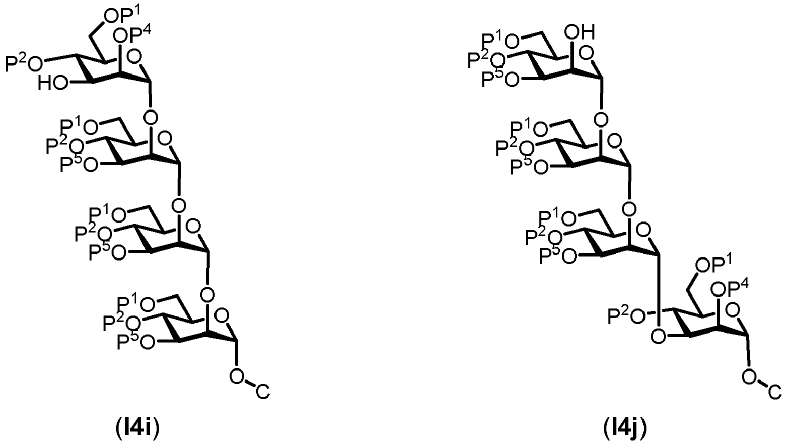 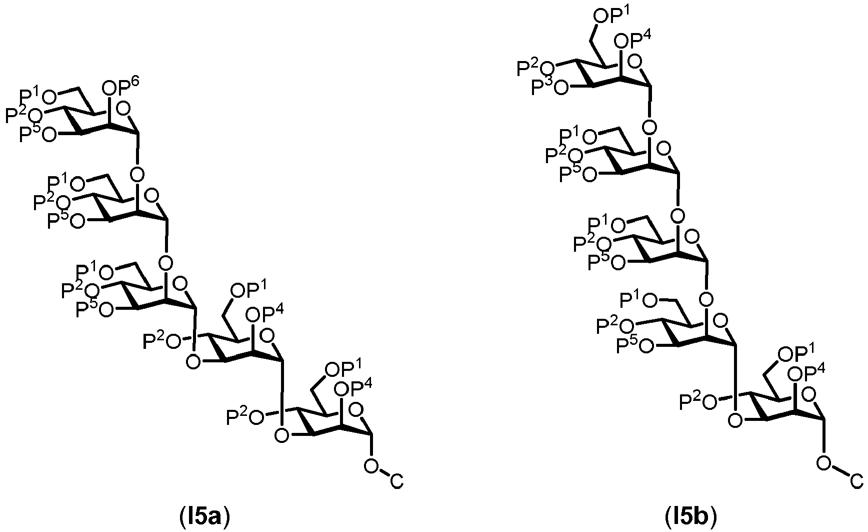 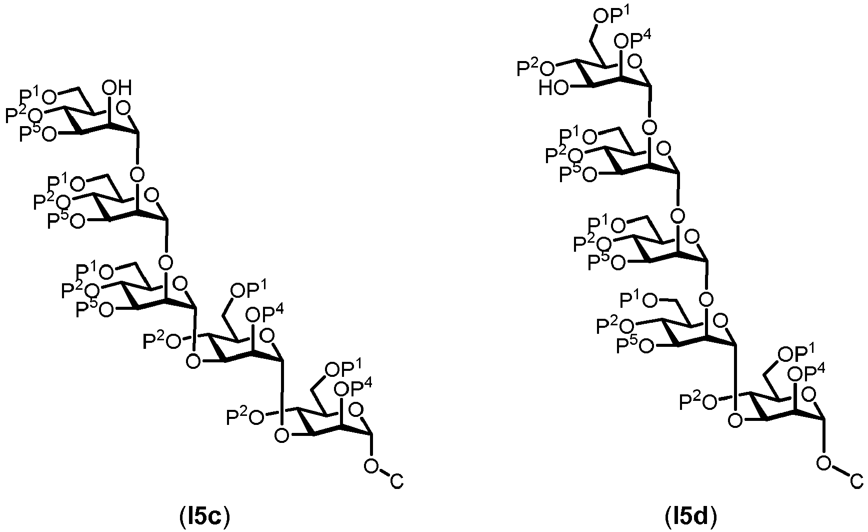 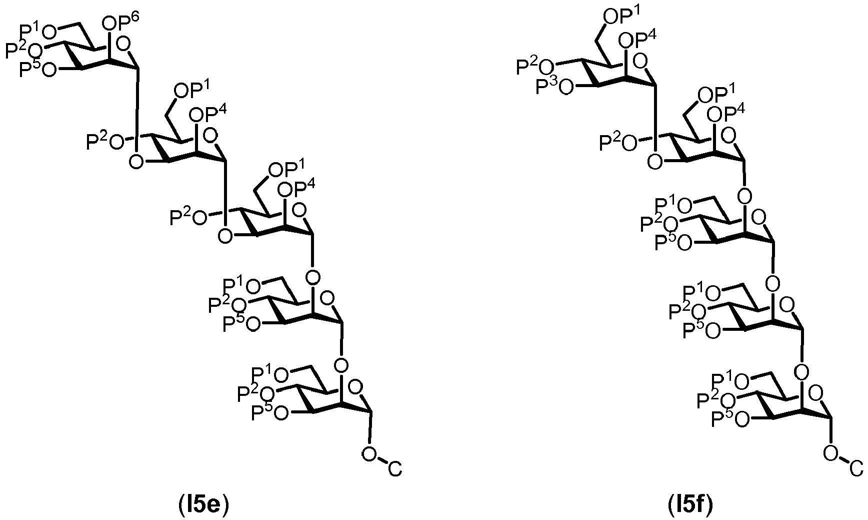 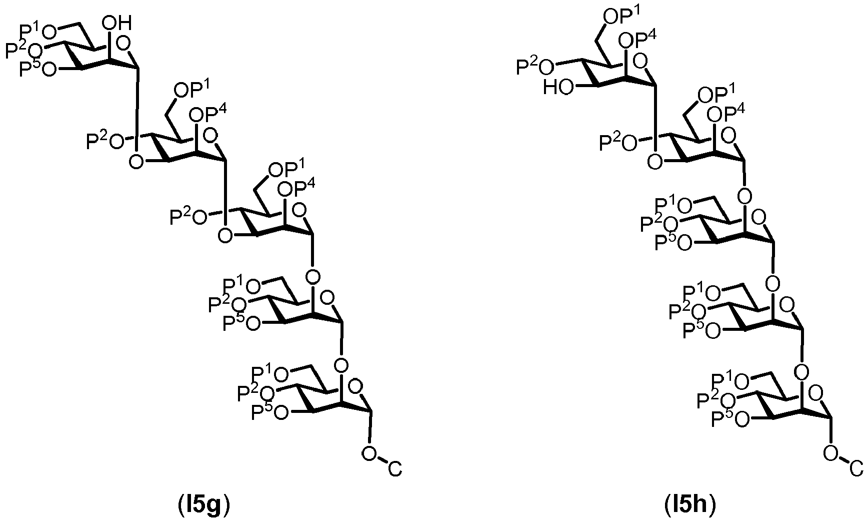 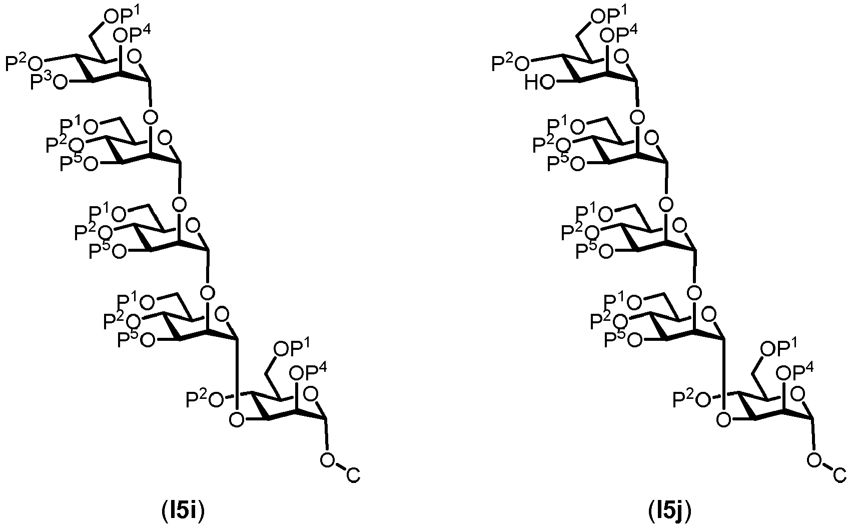 wherein P¹- P⁶ represent protecting groups and C represents -L-Eₚ with Ep being a solid support or a protected end group E, wherein E and L have the meanings as defined in claim 1.

14. The conjugate according to claim 6 of general formula **(V)**
**[H-((-Uₓ₊₄-Uₓ₊₃-Uₓ₊₂-Uₓ₊₁-Uₓ)ₘ-(Vₓ₊₂-Vₓ₊₁-Vₓ)₁₋ₘ)ₙ-O-L-E₁-W]_{c}-CRM₁₉₇** **(V)**
wherein
c is comprised between 2 and 18;
-E₁- represents a covalent bond, -NH-, -O-NH-, -O-, -S-, -CO-, -CH=CH-, -CONH-, -CO-NHNH-, 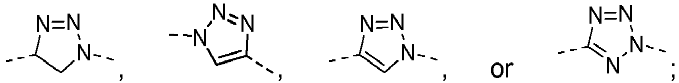
-W- is selected from: 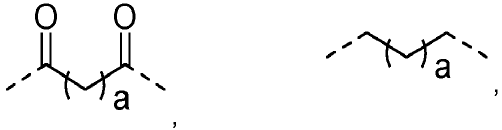 and 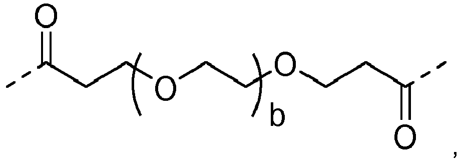
a represents an integer selected from 1, 2, 3, 4, 5, 6, 7, 8, 9 and 10,
b represents an integer selected from 1, 2, 3 and 4, and
m, n, x, L, Uₓ₊₁, Uₓ₊₂, Uₓ₊₃, Uₓ₊₄, Vₓ, Vₓ₊₁ and Vₓ₊₂ have the meanings as defined in claim 1.

15. The conjugate according to claim 14, wherein the conjugate has any one of the following formula **(V-1)** - **(V-11)** 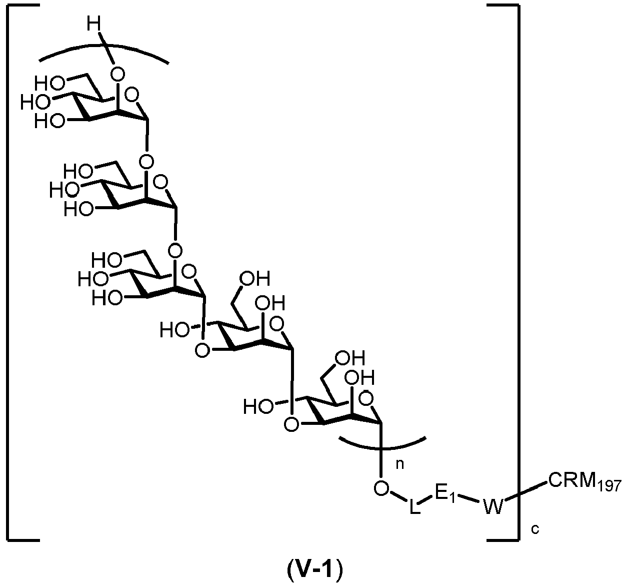 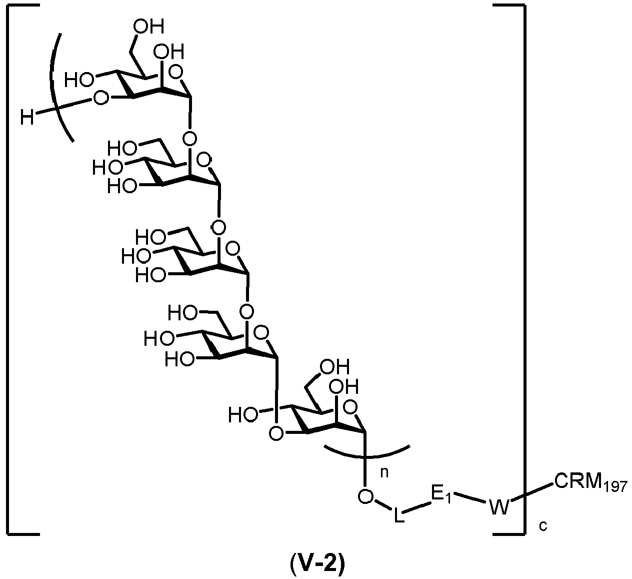 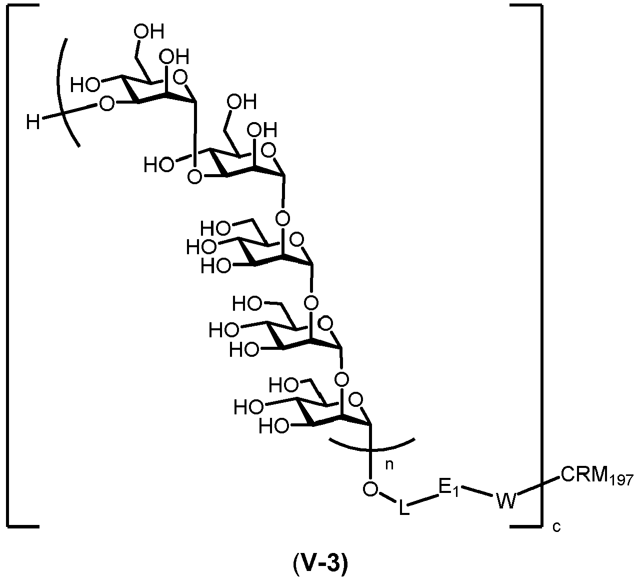 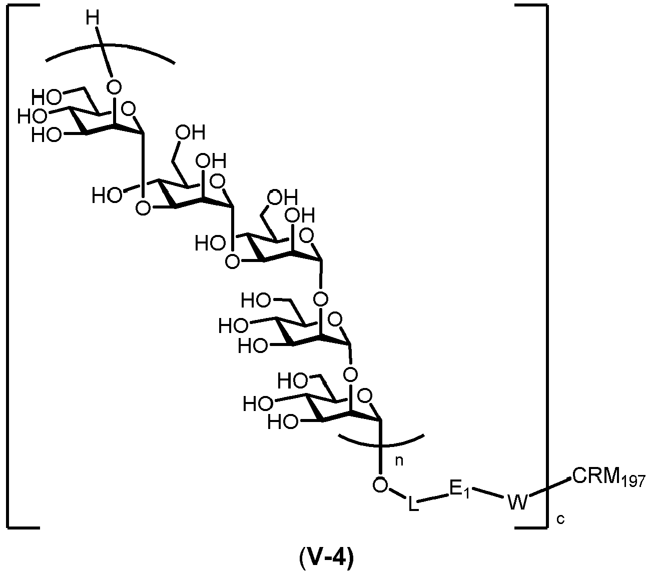 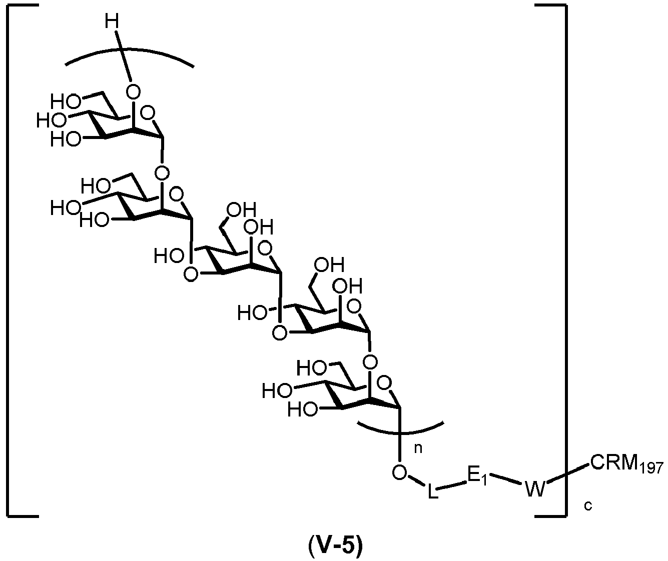 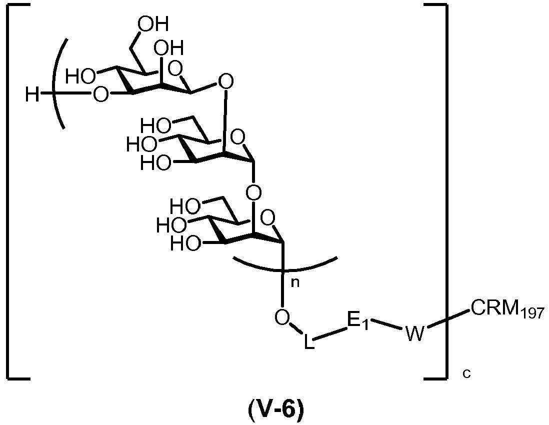 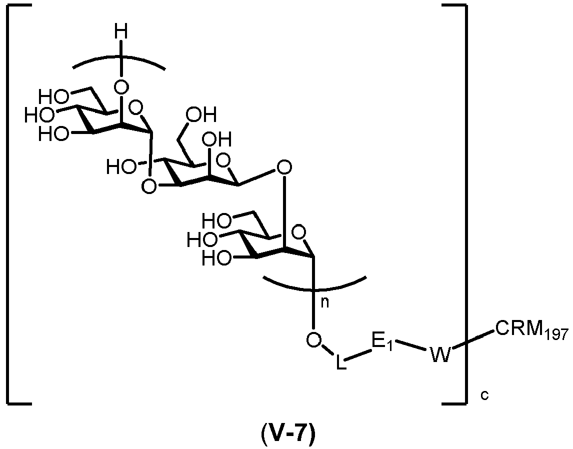 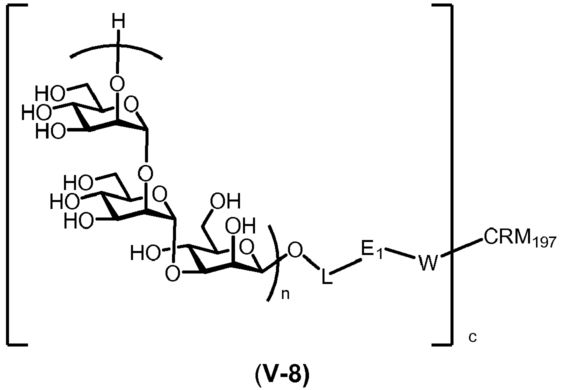 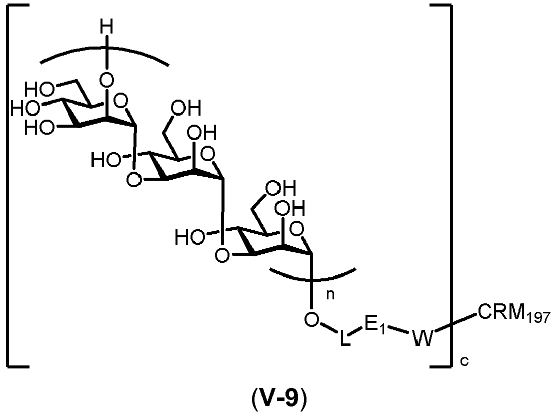 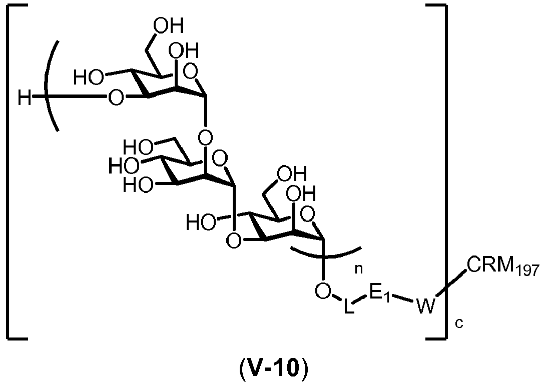 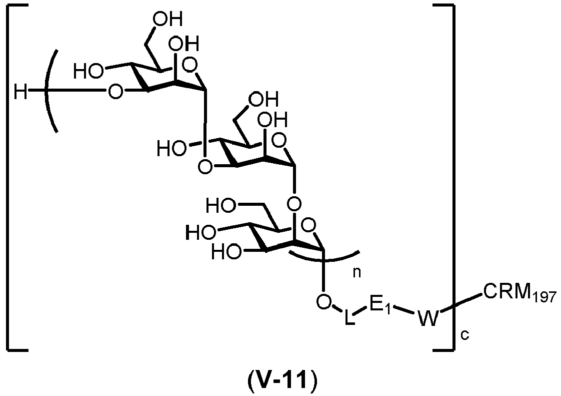 wherein L, E₁, W, c and n have the meanings as defined in claim 19.
